(19) Europäisches Patentamt
European Patent Office
Office européen des brevets

(11) EP 4 778 941 A1

(12) EUROPEAN PATENT APPLICATION
published in accordance with Art. 153(4) EPC

(43) Date of publication:
**22.07.2026 Bulletin 2026/30**

(21) Application number: **24864743.0**

(22) Date of filing: **13.09.2024**

(51) International Patent Classification (IPC):
***C07K 16/46*** (2006.01) ***C07K 16/28*** (2006.01)
***C12N 15/63*** (2006.01) ***A61K 47/65*** (2017.01)
***A61K 47/68*** (2017.01) ***A61K 39/395*** (2006.01)
***A61P 35/00*** (2006.01)

(52) Cooperative Patent Classification (CPC):
**A61K 39/395; A61K 47/65; A61K 47/68; A61P 35/00; C07K 16/28; C07K 16/46; C12N 15/63**

(86) International application number:
**PCT/CN2024/118787**

(87) International publication number:
**WO 2025/056029 (20.03.2025 Gazette 2025/12)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.09.2023 CN 202311193662**
**07.04.2024 CN 202410406619**

(71) Applicants:
- **Jiangsu Hengrui Pharmaceuticals Co., Ltd.**
  **Lianyungang, Jiangsu 222047 (CN)**
- **Shanghai Shengdi Pharmaceutical Co., Ltd**
  **Shanghai 201210 (CN)**

(72) Inventors:
- **LI, Dan**
  **Shanghai 201210 (CN)**
- **HU, Yali**
  **Shanghai 201210 (CN)**
- **ZHANG, Wei**
  **Shanghai 201210 (CN)**
- **CHEN, Simeng**
  **Shanghai 201210 (CN)**
- **YUAN, Jimin**
  **Shanghai 201210 (CN)**
- **LIAO, Cheng**
  **Lianyungang, Jiangsu 222047 (CN)**

(74) Representative: **Dragotti & Associati S.P.A.**
**Via Nino Bixio, 7**
**20129 Milano (MI) (IT)**

Remarks:
The complete document including Reference Table(s) and the Sequence Listing(s) can be downloaded from the EPO website

# (54) HER3/MET BINDING MOLECULE AND PHARMACEUTICAL USE THEREOF

(57) The present disclosure relates to a HER3/MET binding molecule and the pharmaceutical use thereof. Specifically, the present disclosure relates to an anti-HER3/MET bispecific antibody, an anti-HER3/MET antibody-drug conjugate, and a method therefor and the pharmaceutical use thereof for treating cancer.

EP 4 778 941 A1

## Description

### TECHNICAL FIELD

The present disclosure relates to a HER3/MET-binding molecules, e.g., an anti-HER3/MET bispecific antibody, an anti-HER3/MET antibody-drug conjugate, a method for treating cancer using same, and pharmaceutical use thereof.

### BACKGROUND

Antibody-drug conjugates (ADCs) utilize antibodies to provide targeting, while conjugated small-molecule chemical drugs play a role in killing tumor cells. This perfect combination forms a "magic bullet" platform for precise treatment with low side effects. Since the first ADC drug, Mylotarg, was marketed in 2000, continuous innovation has been made in this field, and more than ten ADC drugs have emerged so far.

MET (mesenchymal-epithelial transition factor) is a membrane receptor that is essential for embryonic development and wound healing. Hepatocyte growth factor (HGF) is the only known ligand for the MET receptor. MET is normally expressed by cells of epithelial origin, whereas the expression of HGF is restricted to cells of mesenchymal origin. Upon HGF stimulation, MET induces several biological responses that collectively result in a process known as invasive growth. Aberrant MET activation in cancer is associated with poor prognosis, where aberrantly active MET triggers tumor growth, formation of new blood vessels (angiogenesis) that supply nutrients to the tumor (angiogenesis), and cancer spread to other organs (metastasis).

HER3 (epidermal growth factor receptor 3, ErbB-3 or HER3) is a member of the epidermal growth factor receptor (EGFR) family. These receptors each comprise 3 parts: an extracellular region, a transmembrane region, and an intracellular region. The extracellular region comprises 4 domains. The intracellular region comprises 1 intracellular tyrosine kinase domain for signaling and 1 tail in the cytoplasm comprising tyrosine phosphorylation residues. When ligands bind to extracellular domains I and III, cell signaling is initiated. Normally, these receptors mediate cell division, migration, survival, and organ development. When the EGFR family members mutate, the resulting aberrant signaling stimulates cell survival, associated with cancer progression. c-Met and EGFR (or the HER family) interact and are involved in various mechanisms associated with tumor growth. These proteins (targets) are typical receptor tyrosine kinases (RTKs) present on the surface of cells, thereby inducing proliferation of cancer cells, penetration of cancer cells, angiogenesis, and the like.

The present disclosure provides an anti-HER3/MET bispecific antibody and a drug conjugate thereof. The anti-HER3/MET bispecific antibody-drug conjugate has excellent tumor growth inhibition and killing effects, good druggability, and high potential clinical safety.

### SUMMARY

The present disclosure provides a MET-binding molecule, a HER3-binding molecule, and a HER3/MET-binding molecule, and a coding nucleic acid, a vector, a host cell, and a pharmaceutical composition thereof, as well as use thereof in the treatment of cancer and related pharmaceutical use thereof.

#### MET-Binding Molecule

The present disclosure provides a MET-binding molecule comprising a heavy chain variable region (VH1) and a light chain variable region (VL1), wherein the VH1 comprises a HCDR1, a HCDR2, and a HCDR3 from the amino acid sequence set forth in SEQ ID NO: 71; and the VL1 comprises a LCDR1, a LCDR2, and a LCDR3 from the amino acid sequence set forth in SEQ ID NO: 72.

The CDRs are defined according to the Kabat, IMGT, Chothia, AbM, or Contact numbering scheme. In some specific embodiments, the CDRs are defined according to the Kabat numbering scheme.

In some embodiments, the heavy chain variable region comprises a HCDR1, a HCDR2, and a HCDR3 set forth in SEQ ID NOs: 15-17, respectively, and the light chain variable region comprises a LCDR1, a LCDR2, and a LCDR3 of the amino acid sequences set forth in SEQ ID NOs: 74, 19, and 20, respectively.

In some embodiments, the heavy chain variable region comprises a HCDR1, a HCDR2, and a HCDR3 set forth in SEQ ID NOs:15-17, respectively, and the light chain variable region comprises a LCDR1, a LCDR2, and a LCDR3 of the amino acid sequences set forth in SEQ ID NOs:18-20, respectively.

In some embodiments, in any one of the aforementioned MET-binding molecules, the VH1 comprises a K mutation at position 23 and/or a T mutation at position 78, naturally numbered relative to SEQ ID NO: 1; and/or the VL1 comprises a T mutation at position 69 naturally numbered relative to SEQ ID NO: 2; preferably, any one of the aforementioned MET-binding molecules comprises E23K and S78T mutations naturally numbered relative to SEQ ID

NO: 1, and an A69T mutation naturally numbered relative to SEQ ID NO: 2.

In some embodiments, the heavy chain variable region comprises the amino acid sequence set forth in SEQ ID NO: 71 or an amino acid sequence having at least 80% identity thereto, and the light chain variable region comprises the amino acid sequence set forth in SEQ ID NO: 72 or an amino acid sequence having at least 80% identity thereto.

In some embodiments, the heavy chain variable region comprises the amino acid sequence set forth in SEQ ID NO: 1 or an amino acid sequence having at least 80% identity thereto, and the light chain variable region comprises the amino acid sequence set forth in SEQ ID NO: 2 or an amino acid sequence having at least 80% identity thereto.

In some embodiments, the aforementioned MET-binding molecule inhibits tumor growth, or treats or alleviates cancer.

HER3-Binding Molecule

The present disclosure provides a HER3-binding molecule comprising a heavy chain variable region (VH2) and a light chain variable region (VL2), wherein the VH2 comprises a HCDR1, a HCDR2, and a HCDR3 from the amino acid sequence set forth in SEQ ID NO: 13, and the VL2 comprises a LCDR1, a LCDR2, and a LCDR3 from the amino acid sequence set forth in SEQ ID NO: 70.

The CDRs are defined according to the Kabat, IMGT, Chothia, AbM, or Contact numbering scheme. In some specific embodiments, the CDRs are defined according to the Kabat numbering scheme.

In some embodiments, the heavy chain variable region comprises a HCDR1, a HCDR2, and a HCDR3 set forth in SEQ ID NOs: 51-53, respectively, and the light chain variable region comprises a LCDR1, a LCDR2, and a LCDR3 set forth in SEQ ID NOs: 54, 55, and 73, respectively.

In some embodiments, the heavy chain variable region comprises a HCDR1, a HCDR2, and a HCDR3 set forth in SEQ ID NOs: 51-53, respectively, and the light chain variable region comprises a LCDR1, a LCDR2, and a LCDR3 set forth in SEQ ID NOs: 54-56, respectively.

In some embodiments, the heavy chain variable region comprises the amino acid sequence set forth in SEQ ID NO: 13 or an amino acid sequence having at least 80% identity thereto, and the light chain variable region comprises the amino acid sequence set forth in SEQ ID NO: 70 or an amino acid sequence having at least 80% identity thereto.

In some embodiments, the heavy chain variable region comprises the amino acid sequence set forth in SEQ ID NO: 13 or an amino acid sequence having at least 80% identity thereto, and the light chain variable region comprises the amino acid sequence set forth in SEQ ID NO: 14 or an amino acid sequence having at least 80% identity thereto.

In some embodiments, the aforementioned HER3-binding molecule inhibits tumor growth, or treats or alleviates cancer.

HER3/MET-Binding Molecule

The present disclosure provides a HER3/MET-binding molecule comprising a first binding domain that specifically binds to MET and a second binding domain that specifically binds to HER3, which is capable of specifically binding to HER3 and MET simultaneously or separately.

Regarding the First Binding Domain that Specifically Binds to MET:

In some embodiments, the first binding domain that specifically binds to MET of the HER3/MET-binding molecule comprises a heavy chain variable region (VH1) and a light chain variable region (VL1), wherein the VH1 comprises a HCDR1, a HCDR2, and a HCDR3 from the amino acid sequence set forth in any one of SEQ ID NOs: 71, 1, 3, 5, 7, 9, and 11, and/or the VL1 comprises a LCDR1, a LCDR2, and a LCDR3 from the amino acid sequence set forth in any one of SEQ ID NOs: 72, 2, 4, 6, 8, 10, and 12. In some specific embodiments, the VH1 comprises a HCDR1, a HCDR2, and a HCDR3 from the amino acid sequence set forth in SEQ ID NO: 71, and the VL1 comprises a LCDR1, a LCDR2, and a LCDR3 from the amino acid sequence set forth in SEQ ID NO: 72.

In some specific embodiments, the VH1 comprises a HCDR1, a HCDR2, and a HCDR3 from the amino acid sequence set forth in SEQ ID NO: 1, and the VL1 comprises a LCDR1, a LCDR2, and a LCDR3 from the amino acid sequence set forth in SEQ ID NO: 2.

In some specific embodiments, the VH1 comprises a HCDR1, a HCDR2, and a HCDR3 from the amino acid sequence set forth in SEQ ID NO: 3, and the VL1 comprises a LCDR1, a LCDR2, and a LCDR3 from the amino acid sequence set forth in SEQ ID NO: 4.

In some specific embodiments, the VH1 comprises a HCDR1, a HCDR2, and a HCDR3 from the amino acid sequence set forth in SEQ ID NO: 5, and the VL1 comprises a LCDR1, a LCDR2, and a LCDR3 from the amino acid sequence set forth in SEQ ID NO: 6.

In some specific embodiments, the VH1 comprises a HCDR1, a HCDR2, and a HCDR3 from the amino acid

sequence set forth in SEQ ID NO: 7, and the VL1 comprises a LCDR1, a LCDR2, and a LCDR3 from the amino acid sequence set forth in SEQ ID NO: 8.

In some specific embodiments, the VH1 comprises a HCDR1, a HCDR2, and a HCDR3 from the amino acid sequence set forth in SEQ ID NO: 9, and the VL1 comprises a LCDR1, a LCDR2, and a LCDR3 from the amino acid sequence set forth in SEQ ID NO: 10.

In some specific embodiments, the VH1 comprises a HCDR1, a HCDR2, and a HCDR3 from the amino acid sequence set forth in SEQ ID NO: 11, and the VL1 comprises a LCDR1, a LCDR2, and a LCDR3 from the amino acid sequence set forth in SEQ ID NO: 12.

The CDRs are defined according to the Kabat, IMGT, Chothia, AbM, or Contact numbering scheme. In some specific embodiments, the CDRs are defined according to the Kabat numbering scheme.

In some embodiments, the first binding domain that specifically binds to MET of the HER3/MET-binding molecule comprises a heavy chain variable region (VH1) and a light chain variable region (VL1), wherein the heavy chain variable region comprises a HCDR1, a HCDR2, and a HCDR3 set forth in SEQ ID NOs: 15-17, respectively, and the light chain variable region comprises a LCDR1, a LCDR2, and a LCDR3 set forth in SEQ ID NOs: 76, 19, and 20, respectively.

In some specific embodiments, the first binding domain that specifically binds to MET comprises a HCDR1, a HCDR2, a HCDR3, a LCDR1, a LCDR2, and a LCDR3 as shown in any one of the following:

a-1) a HCDR1, a HCDR2, and a HCDR3 set forth in SEQ ID NOs: 15-17, respectively, and a LCDR1, a LCDR2, and a LCDR3 set forth in SEQ ID NOs: 18-20, respectively; or
a-2) a HCDR1, a HCDR2, and a HCDR3 set forth in SEQ ID NOs: 15-17, respectively, and a LCDR1, a LCDR2, and a LCDR3 set forth in SEQ ID NOs: 74, 19, and 20, respectively.

In some other embodiments, the first binding domain that specifically binds to MET comprises a HCDR1, a HCDR2, a HCDR3, a LCDR1, a LCDR2, and a LCDR3 as shown in any one of the following:

i-1) a HCDR1, a HCDR2, and a HCDR3 set forth in SEQ ID NOs: 21-23, respectively, and a LCDR1, a LCDR2, and a LCDR3 set forth in SEQ ID NOs: 24-26, respectively;
i-2) a HCDR1, a HCDR2, and a HCDR3 set forth in SEQ ID NOs: 27-29, respectively, and a LCDR1, a LCDR2, and a LCDR3 set forth in SEQ ID NOs: 30-32, respectively;
i-3) a HCDR1, a HCDR2, and a HCDR3 set forth in SEQ ID NOs: 33-35, respectively, and a LCDR1, a LCDR2, and a LCDR3 set forth in SEQ ID NOs: 36-38, respectively;
i-4) a HCDR1, a HCDR2, and a HCDR3 set forth in SEQ ID NOs: 39-41, respectively, and a LCDR1, a LCDR2, and a LCDR3 set forth in SEQ ID NOs: 42-44, respectively; or
i-5) a HCDR1, a HCDR2, and a HCDR3 set forth in SEQ ID NOs: 45-47, respectively, and a LCDR1, a LCDR2, and a LCDR3 set forth in SEQ ID NOs: 48-50, respectively.

In some embodiments, the first binding domain that specifically binds to MET of the HER3/MET-binding molecule comprises a VH1 and a VL1, wherein any HCDR contained in the VH1 comprises 1, 2, 3, 4, or 5 amino acid mutations compared to any one of the aforementioned HCDRs; and/or any LCDR contained in the VL1 comprises 1, 2, 3, 4, or 5 amino acid mutations compared to any one of the aforementioned LCDRs. In some specific embodiments, the amino acid mutation described above is a replacement, substitution, modification, deletion, and/or addition of an amino acid (e.g., a conservative replacement of an amino acid), and the mutation does not affect or substantially does not affect the function of the first binding domain that specifically binds to MET.

In some embodiments, in the first binding domain that specifically binds to MET of the HER3/MET-binding molecule, the VH1 comprises a K mutation at position 23 and/or a T mutation at position 78, naturally numbered relative to SEQ ID NO: 1; and/or the VL1 comprises a T mutation at position 69 naturally numbered relative to SEQ ID NO: 2; preferably, the first binding domain that specifically binds to MET comprises an E23K mutation and an S78T mutation naturally numbered relative to SEQ ID NO: 1, and an A69T mutation naturally numbered relative to SEQ ID NO: 2.

In some embodiments, in the first binding domain that specifically binds to MET of the HER3/MET-binding molecule:

the VH1 comprises the amino acid sequence set forth in any one of SEQ ID NOs: 71, 1, 3, 5, 7, 9, and 11 or an amino acid sequence having at least 80% sequence identity thereto; and/or
the VL1 comprises the amino acid sequence set forth in any one of SEQ ID NOs: 72, 2, 4, 6, 8, 10, and 12 or an amino acid sequence having at least 80% sequence identity thereto.

Regarding the Second Binding Domain that Specifically Binds to HER3:

In some embodiments, the second binding domain that specifically binds to HER3 of the HER3/MET-binding molecule comprises a heavy chain variable region (VH2) and a light chain variable region (VL2), wherein the VH2 comprises a HCDR1, a HCDR2, and a HCDR3 from the amino acid sequence set forth in SEQ ID NO: 13, and the VL2 comprises a LCDR1, a LCDR2, and a LCDR3 from the amino acid sequence set forth in SEQ ID NO: 70 or 14.

The CDRs are defined according to the Kabat, IMGT, Chothia, AbM, or Contact numbering scheme. In some specific embodiments, the CDRs are defined according to the Kabat numbering scheme.

In some embodiments, the second binding domain that specifically binds to HER3 of the HER3/MET-binding molecule comprises a heavy chain variable region (VH2) and a light chain variable region (VL2), wherein the VH2 comprises a HCDR1, a HCDR2, and a HCDR3 of the amino acid sequences set forth in SEQ ID NOs: 51-53, respectively, and the VL2 comprises a LCDR1, a LCDR2, and a LCDR3 of the amino acid sequences set forth in SEQ ID NOs: 54, 55, and 75, respectively.

In some specific embodiments, the second binding domain that specifically binds to HER3 comprises a HCDR1, a HCDR2, a HCDR3, a LCDR1, a LCDR2, and a LCDR3 as shown in any one of the following:

b-1) a HCDR1, a HCDR2, a HCDR3, a LCDR1, a LCDR2, and a LCDR3 set forth in SEQ ID NOs: 51-56, respectively; or

b-2) a HCDR1, a HCDR2, a HCDR3, a LCDR1, a LCDR2, and a LCDR3 set forth in SEQ ID NOs: 51-55 and 73, respectively.

In some embodiments, the second binding domain that specifically binds to HER3 of the HER3/MET-binding molecule comprises a VH2 and a VL2, wherein any HCDR contained in the VH2 comprises 1, 2, 3, 4, or 5 amino acid mutations compared to any one of the aforementioned HCDRs; and/or any LCDR contained in the VL2 comprises 1, 2, 3, 4, or 5 amino acid mutations compared to any one of the aforementioned LCDRs.

In some specific embodiments, the amino acid mutation described above is a replacement, substitution, modification, deletion, and/or addition of an amino acid (e.g., a conservative replacement of an amino acid), and the mutation does not affect or substantially does not affect the function of the second binding domain that specifically binds to HER3.

In some embodiments, in the second binding domain that specifically binds to HER3 of the HER3/MET-binding molecule:

the VH2 comprises the amino acid sequence set forth in SEQ ID NO: 13 or an amino acid sequence having at least 80% sequence identity thereto; and/or

the VL2 comprises the amino acid sequence set forth in SEQ ID NO: 70 or 14 or an amino acid sequence having at least 80% sequence identity thereto.

In some embodiments, the HER3/MET-binding molecule further comprises a human immunoglobulin Fc region. In some specific embodiments, the Fc region is a human IgG1, IgG2, IgG3, or IgG4 Fc region, e.g., a human IgG1 Fc region.

In some specific embodiments, the Fc region is half-life extended, e.g., comprising M252Y/S254T/T256E mutations.

In some specific embodiments, the Fc region is ADCC-enhanced, e.g., comprising S239D/A330L/I332E mutations, or fucose removal.

In some embodiments, the Fc region enables the binding molecule to form a dimeric molecule while extending the *in vivo* half-life of the binding molecule.

In some embodiments, the Fc region comprises a first subunit (Fc1) and a second subunit (Fc2). In some embodiments, a mutation that pairs two subunits (Fc1 and Fc2) of the Fc region to form a dimer, or a mutation that reduces homodimerization, is introduced. In some embodiments, the first subunit and the second subunit contain a knob-into-hole mutation. For example, within the CH3/CH3 interface, one, two, or more amino acid residues in the CH3 domain of Fc1 are mutated with one or more amino acid residues having a larger side-chain volume, thereby forming protuberances (or knobs) in the surface of the CH3 domain of Fc1; one, two, or more amino acid residues in the CH3 domain of Fc2 that interact with the CH3 domain of Fc1 are mutated with amino acid residues having a smaller side-chain volume, thereby forming cavities (or holes) in the surface of the CH3 domain of Fc2 that interacts with the CH3 domain of Fc1.

In some embodiments, the Fc1 comprises one or more amino acid substitutions at positions selected from the group consisting of 354, 356, 358, and 366, and the Fc2 comprises one or more amino acid substitutions at positions selected from the group consisting of 349, 356, 358, 366, 368, and 407. In some specific embodiments, the Fc1 comprises a mutation at position 366, and the Fc2 comprises a mutation at a position selected from the group consisting of 366, 368,

and 407, or any combination thereof; in some specific embodiments, the Fc1 comprises a mutation at position 354 or 356, and the Fc2 comprises a mutation at position 349; in some specific embodiments, the Fc1 comprises a mutation at position 354 or 356, and the Fc2 comprises mutations at positions 349, 366, 368, and 407.

In some embodiments, the Fc1 comprises one or more amino acid substitutions selected from the group consisting of 354C, 356E, 358M, and 366W, and the Fc2 comprises one or more amino acid substitutions selected from the group consisting of 349C, 356E, 358M, 366S, 368A, and 407V. In some specific embodiments, the Fc1 comprises a 366W mutation, and the Fc2 comprises a mutation selected from the group consisting of 366S, 368A, and 407V, or any combination thereof; in some specific embodiments, the Fc1 comprises a 354C or 356C mutation, and the Fc2 comprises a 349C mutation; or in some specific embodiments, the Fc1 comprises 354C/366W mutations, and the Fc2 comprises 349C/366S/368A/407V mutations.

In some specific embodiments, the Fc1 comprises a T366W mutation, and the Fc2 comprises a mutation selected from the group consisting of T366S, L368A, and Y407V, or any combination thereof; the first subunit of the Fc region comprises an S354C or E356C mutation, and the second subunit comprises a Y349C mutation; or the first subunit of the Fc region comprises S354C/T366W mutations, and the second subunit comprises Y349C/T366S/L368A/Y407V mutations, wherein the mutations are defined according to the Eu numbering.

In some embodiments, the sequence of the Fc1 is set forth in SEQ ID NO: 67, and the sequence of the Fc2 is set forth in SEQ ID NO: 68.

In some embodiments, the HER3/MET-binding molecule comprises reduced mismatch between light and heavy chains by mutating amino acids at the interface between the heavy chain CH1 and the light chain CL in terms of amino acid size and charge. Illustratively, Roche swapped the domains of CH1 and CL and created the CrossMab platform (Schaefer et al., Proceedings of the National Academy of Sciences of the United States of America, 108(27), pp. 11187-11192 (2011)), and MedImmune mutated the heavy chain F126C and light chain S121C to introduce disulfide bonds (Mazor et al., mAbs, 7(2), pp. 377-389 (2015)). Amgen made further electrostatics in the CH1-CL region (Liu et al., Journal of Biological Chemistry, 290(12), pp. 7535-7562 (2015)), and Lilly (Lewis et al., Nature Biotechnology, 32(2), pp. 191-198 (2014)) and Genentech (Dillon et al., mAbs, 9(2), pp. 213-230 (2017)) introduced mutations in both variable and constant domains." WuXi Biologics replaced the constant region of the antibody with the constant region of TCR, as described in CN109535257A (which is incorporated by reference in its entirety).

In some embodiments, the HER3/MET-binding molecule comprises a linker.

In some embodiments, the linker is an amino acid sequence represented by $(G_mS_n)_h$, $(GGNGT(SEQ\ ID\ NO:\ 88))_h$, $(YGNGT(SEQ\ ID\ NO:\ 89))_h$, or $(EPKSS(SEQ\ ID\ NO:\ 90))_h$, wherein m and n are each independently selected from the group consisting of integers from 1 to 8 (e.g., 1, 2, 3, 4, 5, 6, 7, or 8), and h is independently selected from the group consisting of integers from 1 to 20 (e.g., 1, 2, 3, 4, 5, 6, 7, 8, 9, 10, 11, 12, 13, 14, 15, 16, 17, 18, 19, or 20).

In some embodiments, the linker is a $(G_xS)_y$ linker, wherein x is selected from the group consisting of integers from 1 to 5 (e.g., 1, 2, 3, 4, or 5), and y is selected from the group consisting of integers from 1 to 6 (e.g., 1, 2, 3, 4, 5, or 6). In some embodiments, the linker is selected from the group consisting of $G_4S$ (SEQ ID NO: 91), GS, GAP, $(G_4S)_2$ (SEQ ID NO: 92), $(G_4S)_3$ (SEQ ID NO: 93), $(G_4S)_4$ (SEQ ID NO: 94), $(G_4S)_5$ (SEQ ID NO: 95), and ASGS (SEQ ID NO: 98).

In some embodiments, the HER3/MET-binding molecule comprises a first heavy chain, a first light chain, a second heavy chain, and a second light chain, wherein

the first heavy chain comprises, from the N-terminus to the C-terminus, [VH1]-[linker 1]-[Obscurin-O chain]-[linker 3]-[first subunit of Fc region],
the first light chain comprises, from the N-terminus to the C-terminus, [VL1]-[linker 2]-[Titin-T chain],
the second heavy chain comprises, from the N-terminus to the C-terminus, [VH2]-[CH1]-[second subunit of Fc region], and
the second light chain comprises, from the N-terminus to the C-terminus, [VL2]-[CL]; or
the first heavy chain comprises, from the N-terminus to the C-terminus, [VH2]-[linker 1]-[Obscurin-O chain]-[linker 3]-[first subunit of Fc region],
the first light chain comprises, from the N-terminus to the C-terminus, [VL2]-[linker 2]-[Titin-T chain],
the second heavy chain comprises, from the N-terminus to the C-terminus, [VH1]-[CH1]-[second subunit of Fc region], and
the second light chain comprises, from the N-terminus to the C-terminus, [VL1]-[CL]; wherein - represents a peptide bond, and the linker 1, the linker 2, and the linker 3 may be identical or different, and may be independently present or absent.

In some specific embodiments, the amino acid sequences of the linker 1 and the linker 2 are GGGGS, and the linker 3 is absent.

In some embodiments, the Titin-T chain is set forth in SEQ ID NO: 96.

GIPPKIEALPSDISIDEGKVLTVACAFTGEPTPEVTWSCGGRKIHSQEQGRFHIEN TDDLTTLIIMDVQKQDGGLYTLSLGNEFGSDSATVNIHIRSI

(SEQ ID NO: 96).

In some embodiments, the Titin-T chain is a variant of SEQ ID NO: 96, and the variant comprises amino acid residue substitution(s) at one or more positions selected from the group consisting of positions 3, 8, 11, 13, 20, 22, 25, 26, 39, 40, 42, 45, 47, 49, 56, 58, 60, 64, 66, 70, 75, 77, 79, 81, 82, 83, and 84. Illustratively, the variant comprises one or more amino acid residue substitutions selected from the group consisting of 3W, 8C, 111, 13L, 20C, 22M/22C, 25S, 26C, 39T, 40S, 42K, 45S, 47E, 49G, 56S, 58E, 60S, 64T, 66S/66K, 70R, 75V, 77S, 79T, 81R, 82M, 83D, and 84L. In some specific embodiments, the Titin-T chain is set forth in SEQ ID NO: 64 or is an amino acid sequence having at least 80% sequence identity thereto.

In some embodiments, the Obscurin-O chain is set forth in SEQ ID NO: 97.

SGAPRFLTRPKAFVVSVGKDATLSCQIVGNPTPQVSWEKDQQPVAAGARFRLA QDGDLYRLTILDLALGDSGQYVCRARNAIGEAFAAVGLQVDAEA

(SEQ ID NO: 97).

In some embodiments, the Obscurin-O chain is a variant of SEQ ID NO: 97, and the variant comprises amino acid residue substitution(s) at one or more positions selected from the group consisting of positions 2, 3, 7, 9, 11, 12, 13, 14, 17, 20, 22, 25, 30, 32, 34, 36, 41, 42, 44, 45, 48, 53, 58, 62, 66, 67, 69, 76, 82, 88, 89, 92, 93, 94, and 97. Illustratively, the variant comprises one or more amino acid residue substitutions selected from the group consisting of 2E, 3C, 7K/7R, 9C, 11L, 12S, 13Y/13S, 14T, 17E, 20L, 22M/22S, 25S, 30D, 32P/32F, 34E, 36T, 41K, 42L, 44I, 45T, 48V, 53L, 58V, 62E/62K/62H, 66C, 67Q/67T, 69S, 76S, 82H, 88C, 89L, 92E, 93C, 94G, and 97G. In some specific embodiments, the Obscurin-O chain is set forth in SEQ ID NO: 63 or is an amino acid sequence having at least 80% sequence identity thereto.

In some embodiments, the HER3/MET-binding molecule comprises the following polypeptide chain combinations:

a first heavy chain comprising the amino acid sequence set forth in any one of SEQ ID NOs:77, 57, and 61 or an amino acid sequence having at least 90% sequence identity thereto,
a first light chain comprising the amino acid sequence set forth in any one of SEQ ID NOs:78, 58, and 62 or an amino acid sequence having at least 90% sequence identity thereto,
a second heavy chain comprising the amino acid sequence set forth in SEQ ID NO: 59 or an amino acid sequence having at least 90% sequence identity thereto, and/or a second light chain comprising the amino acid sequence set forth in any one of SEQ ID NO: 79 or 60 or an amino acid sequence having at least 90% sequence identity thereto.

In some embodiments, provided is a HER3/MET-binding molecule comprising:

a first heavy chain set forth in SEQ ID NO: 57,
a first light chain set forth in SEQ ID NO: 58,
a second heavy chain set forth in SEQ ID NO: 59, and
a second light chain set forth in SEQ ID NO: 60.

In some embodiments, provided is a HER3/MET-binding molecule comprising:

a first heavy chain set forth in SEQ ID NO: 61,
a first light chain set forth in SEQ ID NO: 62,
a second heavy chain set forth in SEQ ID NO: 59, and
a second light chain set forth in SEQ ID NO: 60.

In some embodiments, provided is a HER3/MET-binding molecule comprising:

a first heavy chain set forth in SEQ ID NO: 77,
a first light chain set forth in SEQ ID NO: 78,
a second heavy chain set forth in SEQ ID NO: 59, and
a second light chain set forth in SEQ ID NO: 79.

Antibody-Drug Conjugate

In some embodiments, the HER3/MET-binding molecule of the present disclosure is an anti-HER3/MET antibody-drug conjugate.

In some embodiments, the antibody-drug conjugate comprises an effector, wherein the effector is selected from the group consisting of a radioisotope, an anti-tumor agent, an immunomodulator, a biological response modifier, a lectin, a cytotoxic drug, a chromophore, a fluorophore, a chemiluminescent compound, an enzyme, and a metal ion, and any combination thereof.

In some specific embodiments, the effector is a cytotoxic drug, illustratively, a tubulin polymerization inhibitor, a Topo I inhibitor, and MMAE or a derivative thereof. In some specific embodiments, the cytotoxin is selected from the group consisting of MMAE or a derivative thereof, exatecan or a derivative thereof, and eribulin or a derivative thereof.

Antibody-Drug (Exatecan or Derivative thereof) Conjugate

The present disclosure provides an antibody-drug conjugate having a structure represented by formula (I):

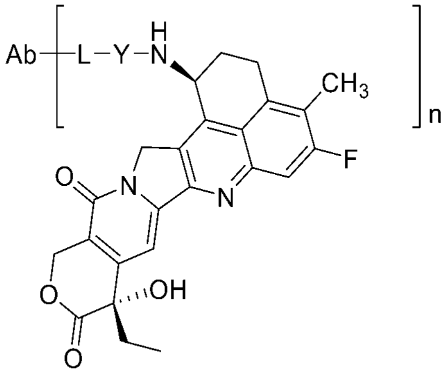

(I),

wherein -L- is a linker unit that is $-L^1-L^2-L^3-L^4-$;

$L^1$ is -(succinimid-3-yl-*N*)-W-C(O)-, $-CH_2-C(O)-NR^3-W-C(O)-$, or -C(O)-W-C(O)-, wherein W is selected from the group consisting of $C_{1-8}$ alkyl, $C_{1-8}$ alkyl-cycloalkyl, and linear heteroalkyl of 1 to 8 atoms, and the heteroalkyl comprises 1 to 3 heteroatoms selected from the group consisting of N, O, and S, wherein the $C_{1-8}$ alkyl, cycloalkyl, and linear heteroalkyl are each independently and optionally further substituted with one or more substituents selected from the group consisting of halogen, hydroxy, cyano, amino, alkyl, chloroalkyl, deuterated alkyl, alkoxy, and cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$, and a chemical bond, wherein $p^1$ is an integer from 1 to 20;

$L^3$ is a peptide residue consisting of 2 to 7 amino acids, wherein the amino acids are optionally further substituted with one or more substituents selected from the group consisting of halogen, hydroxy, cyano, amino, alkyl, chloroalkyl, deuterated alkyl, alkoxy, and cycloalkyl;

$L^4$ is selected from the group consisting of $-NR^5(CR^6R^7)_t-$, $-C(O)NR^5$, $-C(O)NR^5(CH_2)_t-$, and a chemical bond, wherein t is an integer from 1 to 6;

$R^3$, $R^4$, and $R^5$ are identical or different and are each independently selected from the group consisting of hydrogen, alkyl, haloalkyl, deuterated alkyl, and hydroxyalkyl;

$R^6$ and $R^7$ are identical or different and are each independently selected from the group consisting of hydrogen, halogen, alkyl, haloalkyl, deuterated alkyl, and hydroxyalkyl;

Y is selected from the group consisting of $-O-(CR^aR^b)_m-CR^1R^2-C(O)-$, $-O-CR^1R^2-(CR^aR^b)_m-$, $-O-CR^1R^2-$, $-NH-(CR^aR^b)_m-CR^1R^2-C(O)-$, and $-S-(CR^aR^b)_m-CR^1R^2-C(O)-$;

$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl, and heterocyclyl; or $R^a$ and $R^b$, together with the carbon atom to which they are attached, form cycloalkyl or heterocyclyl;

$R^1$ is selected from the group consisting of hydrogen, halogen, haloalkyl, deuterated alkyl, cycloalkyl, cycloalkylalkyl, alkoxyalkyl, heterocyclyl, aryl, and heteroaryl;

$R^2$ is selected from the group consisting of hydrogen, halogen, haloalkyl, deuterated alkyl, cycloalkyl, cycloalkylalkyl, alkoxyalkyl, heterocyclyl, aryl, and heteroaryl;

or $R^1$ and $R^2$, together with the carbon atom to which they are attached, form cycloalkyl or heterocyclyl;
or $R^a$ and $R^2$, together with the carbon atom to which they are attached, form cycloalkyl or heterocyclyl;
m is an integer from 0 to 4;
n is 1 to 10, and n is an integer or a decimal.

In some embodiments, Ab is an antibody comprising a binding domain that specifically binds to MET, and/or a binding domain that specifically binds to HER3.

In some embodiments, the antibody-drug conjugate represented by formula (I) according to any one of the foregoing is provided,
wherein:

Y is $-O-(CR^aR^b)_m-CR^1R^2-C(O)-$;
$R^a$ and $R^b$ are identical or different and are each independently selected from the group consisting of hydrogen, deuterium, halogen, and alkyl;
$R^1$ is selected from the group consisting of hydrogen, haloalkyl, and $C_{3-6}$ cycloalkyl;
$R^2$ is selected from the group consisting of hydrogen, haloalkyl, and $C_{3-6}$ cycloalkyl;
or $R^1$ and $R^2$, together with the carbon atom to which they are attached, form $C_{3-6}$ cycloalkyl;
m is 0 or 1.

In some embodiments, the antibody-drug conjugate represented by formula (I) according to any one of the foregoing is provided,

wherein Y is selected from the group consisting of:

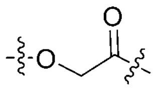, 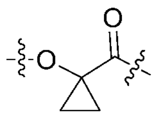, 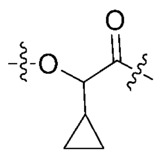, 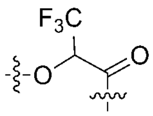, 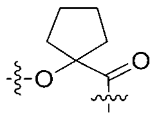, 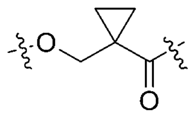,

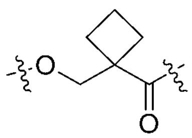, and 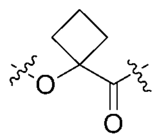,

wherein the O-end of Y is linked to the linker unit L.

In some embodiments, the antibody-drug conjugate represented by formula (I) according to any one of the foregoing is provided,

wherein the linker unit -L- is $-L^1-L^2-L^3-L^4-$;
$L^1$ is selected from the group consisting of -(succinimid-3-yl-*N*)-W-C(O)-, $-CH_2-C(O)-NR^3-W-C(O)-$, and -C(O)-W-C(O)-, wherein W is selected from the group consisting of $C_{1-8}$ alkyl, $C_{1-8}$ alkyl-cycloalkyl, and linear heteroalkyl of 1 to 8 atoms, and the heteroalkyl comprises 1 to 3 heteroatoms selected from the group consisting of N, O, and S, wherein the $C_{1-8}$ alkyl, cycloalkyl, and linear heteroalkyl are each independently and optionally further substituted with one or more substituents selected from the group consisting of halogen, hydroxy, cyano, amino, alkyl, chloroalkyl, deuterated alkyl, alkoxy, and cycloalkyl;
$L^2$ is selected from the group consisting of $-NR^4(CH_2CH_2O)pCH_2CH_2C(O)-$, $-NR^4(CH_2CH_2O)pCH_2C(O)-$, $-S(CH_2)pC(O)-$, and a chemical bond, wherein p is an integer from 1 to 20;
$L^3$ is a peptide residue consisting of 2 to 7 amino acid residues, wherein the amino acid residues are selected from the group consisting of amino acid residues formed from amino acids from phenylalanine, glycine, valine, lysine, citrulline, serine, glutamic acid, and aspartic acid, and are optionally further substituted with one or more substituents selected from the group consisting of halogen, hydroxy, cyano, amino, alkyl, chloroalkyl, deuterated alkyl, alkoxy, and cycloalkyl;
$L^4$ is selected from the group consisting of $-NR^5(CR^6R^7)_t-$, $-C(O)NR^5$, $-C(O)NR^5(CH_2)_t-$, and a chemical bond, wherein t is an integer from 1 to 6;
$R^3$, $R^4$, and $R^5$ are identical or different and are each independently selected from the group consisting of hydrogen,

alkyl, haloalkyl, deuterated alkyl, and hydroxyalkyl;
$R^6$ and $R^7$ are identical or different and are each independently selected from the group consisting of hydrogen, halogen, alkyl, haloalkyl, deuterated alkyl, and hydroxyalkyl.

In some embodiments, the antibody-drug conjugate represented by formula (I) according to any one of the foregoing is provided,

wherein the linker unit -L- is $-L^1-L^2-L^3-L^4-$;
$L^1$ is selected from the group consisting of -(succinimid-3-yl-*N*)-W-C(O)-, $-CH_2-C(O)-NR^3-W-C(O)-$, and -C(O)-W-C(O)-, wherein W is selected from the group consisting of $C_{1-8}$ alkyl, $C_{1-8}$ alkyl-cycloalkyl, and linear heteroalkyl of 1 to 8 chain atoms, and the heteroalkyl comprises 1 to 3 heteroatoms selected from the group consisting of N, O, and S, wherein the $C_{1-8}$ alkyl, cycloalkyl, and linear heteroalkyl are each independently and optionally further substituted with one or more substituents selected from the group consisting of halogen, hydroxy, cyano, amino, alkyl, chloroalkyl, deuterated alkyl, alkoxy, and cycloalkyl;
$L^2$ is selected from the group consisting of $-NR^4(CH_2CH_2O)pCH_2CH_2C(O)-$, $-NR^4(CH_2CH_2O)pCH_2C(O)-$, $-S(CH_2)pC(O)-$, and a chemical bond, wherein p is an integer from 1 to 20;
$L^3$ is a peptide residue consisting of 2 to 7 amino acid residues, wherein the amino acid residues are selected from the group consisting of amino acid residues formed from amino acids from phenylalanine (F), glycine (G), valine (V), lysine (K), citrulline, serine (S), glutamic acid (Q), and aspartic acid (D), and are optionally further substituted with one or more substituents selected from the group consisting of halogen, hydroxy, cyano, amino, alkyl, chloroalkyl, deuterated alkyl, alkoxy, and cycloalkyl;
$L^4$ is selected from the group consisting of $-NR^5(CR^6R^7)_t-$, $-C(O)NR^5-$, $-C(O)NR'(CH_2)t-$, and a chemical bond, wherein t is an integer from 1 to 6, and non-limiting examples are 1, 2, 3, 4, 5, and 6;
$R^3$, $R^4$, and $R^5$ are identical or different and are each independently selected from the group consisting of hydrogen, alkyl, haloalkyl, deuterated alkyl, and hydroxyalkyl;
$R^6$ and $R^7$ are identical or different and are each independently selected from the group consisting of hydrogen, halogen, alkyl, haloalkyl, deuterated alkyl, and hydroxyalkyl. In some embodiments, the antibody-drug conjugate represented by formula (I) according to any one of the foregoing is provided,
wherein the linker unit -L- is $-L^1-L^2-L^3-L^4-$;
$L^1$ is

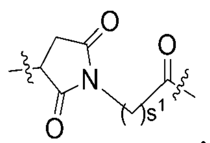
,

wherein $s^1$ is an integer from 2 to 8, and non-limiting examples are 2, 3, 4, 5, 6, 7, and 8;
$L^2$ is a chemical bond;
$L^3$ is a tetrapeptide residue; preferably, $L^3$ is a tetrapeptide residue of GGFG;
$L^4$ is $-NR^5(CR^6R^7)t-$, wherein $R^5$, $R^6$, and $R^7$ are identical or different and are each independently hydrogen or alkyl, and t is 1 or 2;
wherein the $L^1$ end is linked to Ab, and the $L^4$ end is linked to Y.

In some embodiments, the antibody-drug conjugate represented by formula (I) according to any one of the foregoing is an antibody-drug conjugate represented by general formula (II):

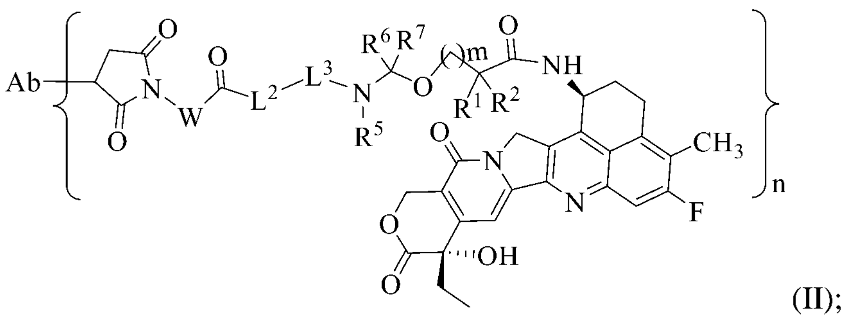
(II);

wherein:

W is selected from the group consisting of $C_{1-8}$ alkyl, $C_{1-8}$ alkyl-cycloalkyl, and linear heteroalkyl of 1 to 8 atoms, and the heteroalkyl comprises 1 to 3 heteroatoms selected from the group consisting of N, O,and S, wherein the $C_{1-8}$ alkyl, cycloalkyl, and linear heteroalkyl are each independently and optionally further substituted with one or more substituents selected from the group consisting of halogen, hydroxy, cyano, amino, alkyl, chloroalkyl, deuterated alkyl, alkoxy, and cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$, and a chemical bond, and $p^1$ is an integer from 1 to 20;

$L^3$ is a peptide residue consisting of 2 to 7 amino acids, wherein the amino acids are optionally further substituted with one or more substituents selected from the group consisting of halogen, hydroxy, cyano, amino, alkyl, chloroalkyl, deuterated alkyl, alkoxy, and cycloalkyl;

$R^1$ is selected from the group consisting of hydrogen, halogen, cycloalkylalkyl, deuterated alkyl, cycloalkyl, heterocyclyl, aryl, and heteroaryl;

$R^2$ is selected from the group consisting of hydrogen, halogen, haloalkyl, deuterated alkyl, cycloalkyl, heterocyclyl, aryl, and heteroaryl;

or $R^1$ and $R^2$, together with the carbon atom to which they are attached, form cycloalkyl or heterocyclyl;

$R^4$ and $R^5$ are identical or different and are each independently selected from the group consisting of hydrogen, alkyl, haloalkyl, deuterated alkyl, and hydroxyalkyl;

$R^6$ and $R^7$ are identical or different and are each independently selected from the group consisting of hydrogen, halogen, alkyl, haloalkyl, deuterated alkyl, and hydroxyalkyl;

m is an integer from 0 to 4;

n is 1 to 10, and n is an integer or a decimal;

Ab is as defined in general formula (I).

In some embodiments, the antibody-drug conjugate represented by formula (II) according to any one of the foregoing is provided, wherein n is 1 to 10, e.g., 1 to 8, 2 to 8, 2 to 7, 2 to 4, 3 to 8, 3 to 7, 3 to 6, 4 to 7, or 4 to 6, and n is a decimal or an integer. In some embodiments, n is 1 to 8, and n is a decimal or an integer. In some embodiments, n is 3 to 7, and n is a decimal or an integer. In some embodiments, n is 4 to 6, and n is a decimal or an integer. In some embodiments, n is an average value of about 1, about 2, about 3, about 4, about 5, about 6, about 7, about 8, about 9, or about 10. In some embodiments, n is an average value of about 4.1, about 4.2, about 4.3, about 4.4, about 4.5, about 4.6, 4.7, about 4.8, about 4.9, about 5, about 5.1, about 5.2, about 5.3, about 5.4, about 5.5, about 5.6, about 5.7, about 5.8, about 5.9, or about 6.

In some embodiments, the antibody-drug conjugate represented by formula (II) according to any one of the foregoing is provided,

wherein:

-L-Y- is represented by the following structure:

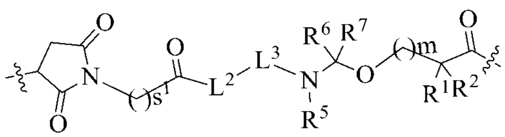

(-L-Y-)

$s^1$ is an integer from 2 to 8;

$L^2$, $L^3$, $R^1$, $R^2$, $R^5$, $R^6$, $R^7$, and m are as defined in the aforementioned general formula (II).

In some embodiments, the antibody-drug conjugate represented by general formula (I) according to any one of the foregoing is an antibody-drug conjugate represented by general formula (III):

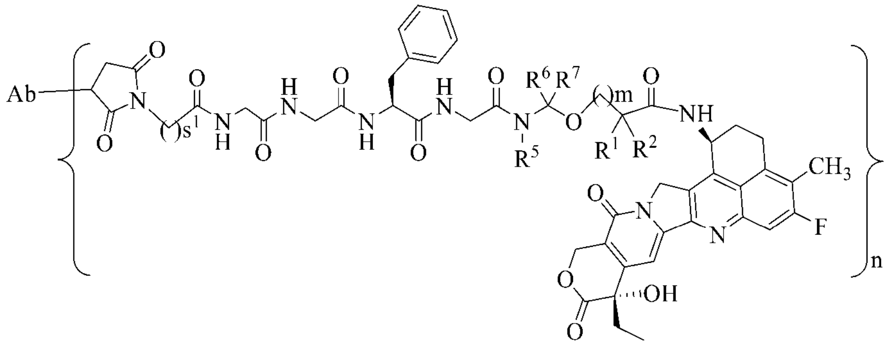

(III).

In some embodiments, the antibody-drug conjugate represented by general formula (III) according to any one of the foregoing is provided,
wherein:

$s^1$ is an integer from 2 to 8;
Ab, $R^1$, $R^2$, $R^5$, $R^6$, $R^7$, m, and n are as defined in general formula (II).

In some embodiments, the antibody-drug conjugate represented by general formula (I) according to any one of the foregoing is provided, wherein -L- is:

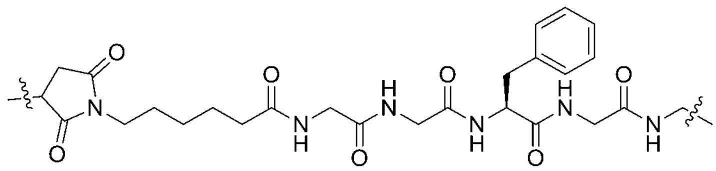

.

In some embodiments, the antibody-drug conjugate represented by general formula (I) according to any one of the foregoing is provided, wherein -L-Y- is optionally selected from the group consisting of:

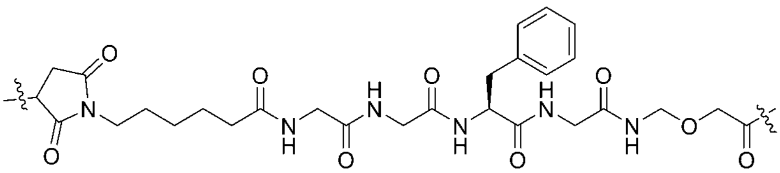

and

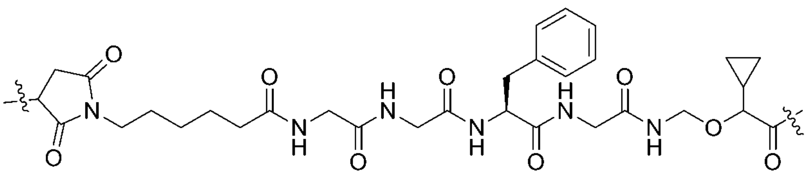

.

In some embodiments, -L-Y- is optionally selected from the group consisting of:

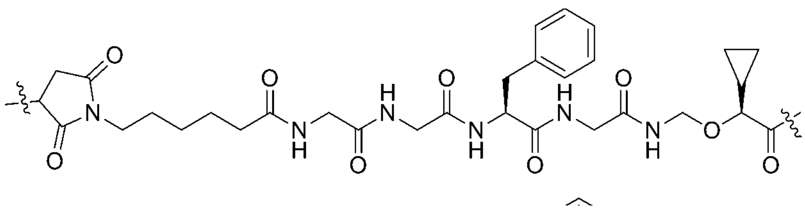

and

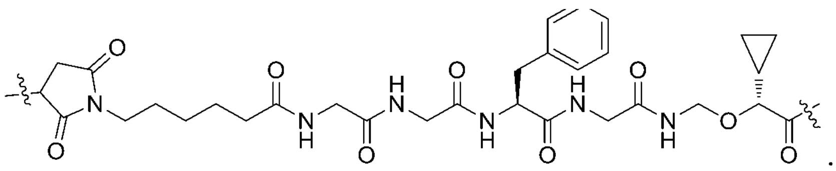

In some embodiments, -L-Y- is

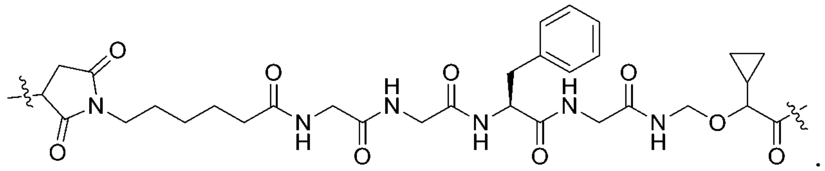

In some embodiments, -L-Y- is

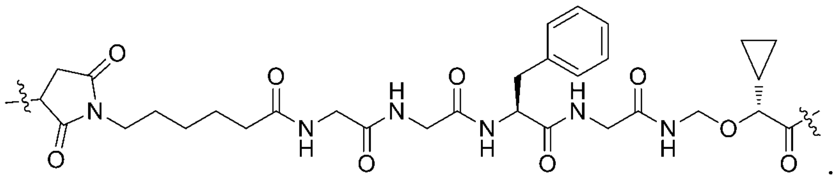

In some embodiments, the antibody-drug conjugate represented by general formula (I) according to any one of the foregoing is provided, wherein the antibody-drug conjugate is selected from the group consisting of:

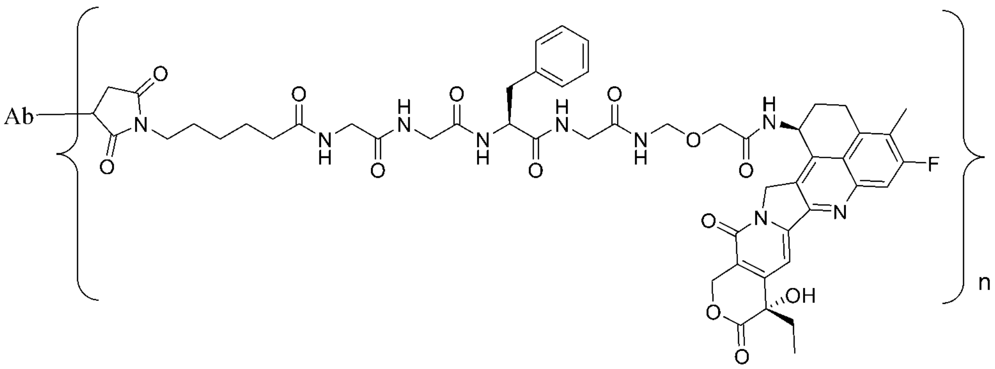

and

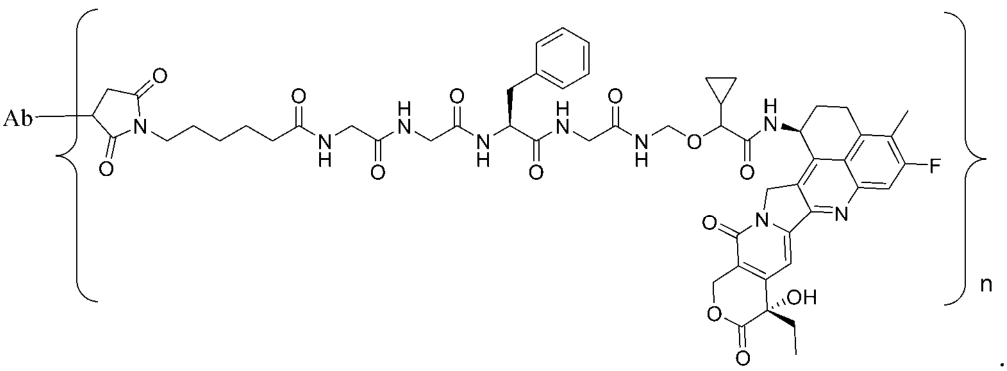

In some embodiments, the antibody-drug conjugate represented by general formula (I) according to any one of the foregoing is provided, wherein the antibody-drug conjugate is selected from the group consisting of:

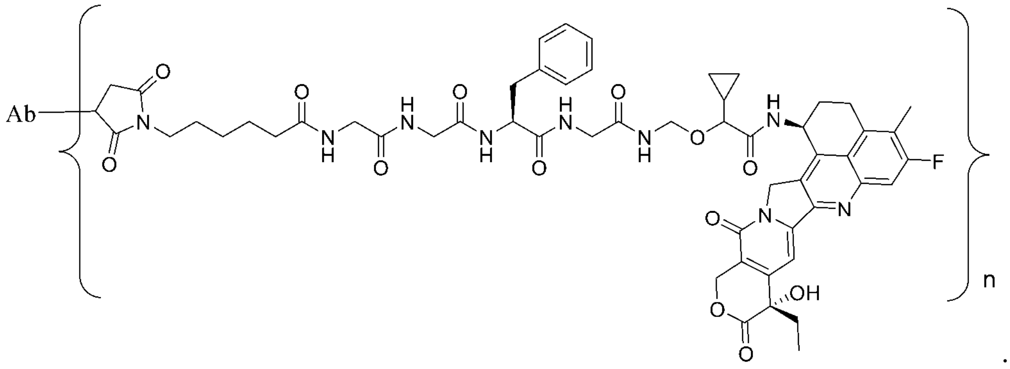

.

In some embodiments, the antibody-drug conjugate represented by general formula (I) according to any one of the foregoing is provided, wherein the antibody-drug conjugate is selected from the group consisting of:

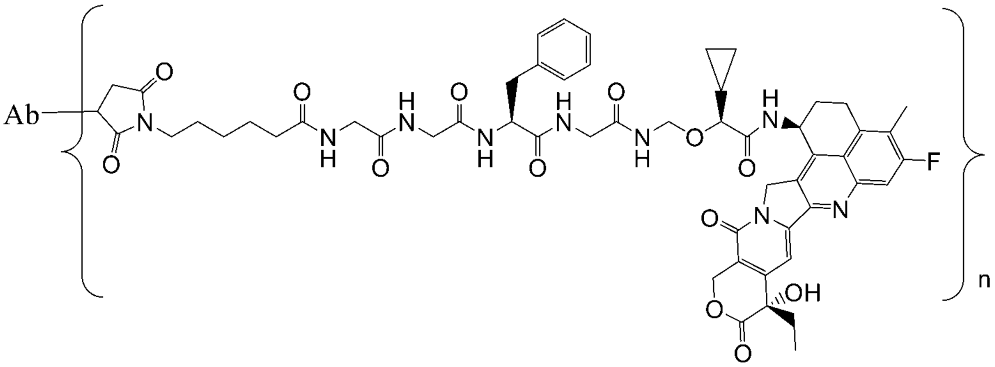

and

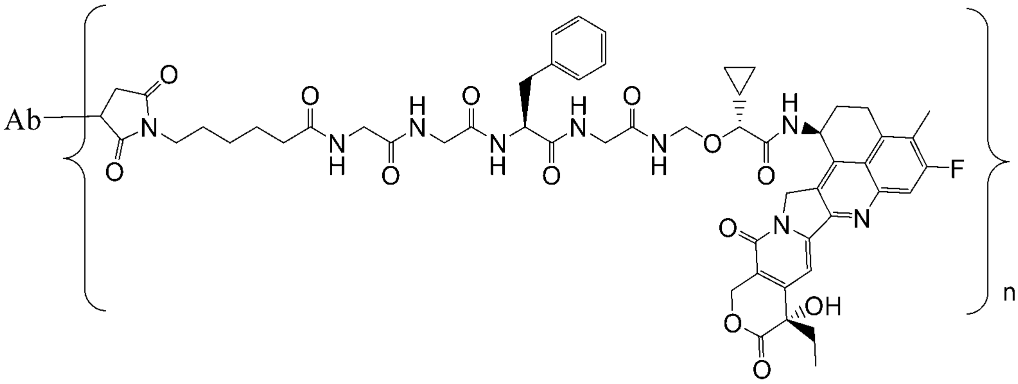

,

wherein Ab and n are as defined in general formula (I).

Antibody-Drug <u>(Eribulin or Derivative thereof) Conjugate</u>

The present disclosure provides an antibody-drug conjugate having a structure represented by formula (IV):

Ab-(L-De)k (IV)

wherein L is a linker covalently attaching Ab to De,
k is 1 to 20 (including 1, 2, 3, 4, 5, 6, 7, 8, 9, 10, 11, 12, 13, 14, 15, 16, 17, 18, 19, 20, or any value between any two of these values),
De is represented by the following formula:

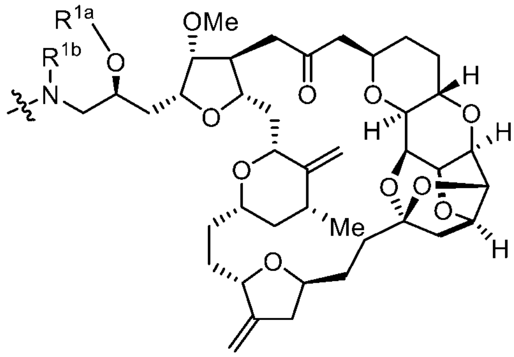

,

wherein $R^{1a}$ is selected from the group consisting of hydrogen, alkyl (e.g., $C_{1-6}$ alkyl including but not limited to methyl, ethyl, and isopropyl), cycloalkyl (e.g., $C_{3-8}$ cycloalkyl including but not limited to cyclopropyl, cyclopentyl, or cyclohexyl), aryl, and heteroaryl, wherein the alkyl, cycloalkyl, aryl, and heteroaryl are each independently and optionally substituted with one or more substituents selected from the group consisting of alkyl (e.g., $C_{1-6}$ alkyl including but not limited to methyl, ethyl, and isopropyl), alkoxy (e.g., $C_{1-6}$ alkoxy including but not limited to methoxy, ethoxy, propoxy, and isopropoxy), halogen (e.g., fluoro, chloro, and bromo), deuterium, amino, cyano, nitro, hydroxy, hydroxyalkyl, cycloalkyl, heterocyclyl, aryl, and heteroaryl; preferably, $R^{1a}$ is methyl;

$R^{1b}$ is selected from the group consisting of hydrogen, alkyl (e.g., $C_{1-6}$ alkyl including but not limited to methyl, ethyl, and isopropyl), alkoxy, cycloalkyl (e.g., $C_{3-8}$ cycloalkyl including but not limited to cyclopropyl, cyclopentyl, or cyclohexyl), aryl, and heteroaryl, wherein the alkyl, cycloalkyl, aryl, and heteroaryl are each independently and optionally substituted with one or more substituents selected from the group consisting of alkyl (e.g., $C_{1-6}$ alkyl including but not limited to methyl, ethyl, and isopropyl), alkoxy (e.g., $C_{1-6}$ alkoxy including but not limited to methoxy, ethoxy, propoxy, and isopropoxy), halogen (e.g., fluoro, chloro, and bromo), deuterium, amino, cyano, nitro, hydroxy, hydroxyalkyl, cycloalkyl, heterocyclyl, aryl, and heteroaryl; preferably, $R^{1b}$ is hydrogen; or

$R^{1a}$ and $R^{1b}$, together with the atom to which they are attached, form $C_{5-8}$ heterocycloalkyl, wherein the heteroalkyl is optionally substituted with one or more substituents of alkyl (e.g., $C_{1-6}$ alkyl including but not limited to methyl, ethyl and isopropyl), alkoxy (e.g., $C_{1-6}$ alkoxy including but not limited to methoxy, ethoxy, propoxy and isopropoxy), halogen (e.g., fluoro, chloro and bromo), deuterium, amino, cyano, nitro, hydroxy, hydroxyalkyl, cycloalkyl (e.g., $C_{3-8}$ cycloalkyl including but not limited to cyclopropyl, cyclopentyl or cyclohexyl), heterocyclyl, aryl and heteroaryl; and $R^{1a}$ and $R^{1b}$ are not both hydrogen.

In some embodiments, Ab is an antibody comprising a binding domain that specifically binds to MET, and/or a binding domain that specifically binds to HER3.

In some embodiments, R1a in De of the antibody-drug conjugate represented by formula (IV) according to any one of the foregoing is methyl.

In some embodiments, De in the antibody-drug conjugate represented by formula (IV) according to any one of the foregoing is represented by the following formula:

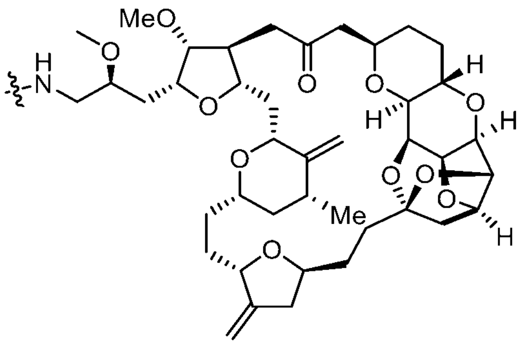

.

In some embodiments, in the antibody-drug conjugate represented by formula (IV) according to any one of the foregoing, k is selected from the group consisting of 1 to 10, and may be an integer or a decimal.

In some embodiments, the linker is stable extracellularly, such that the antibody-drug conjugate represented by formula (IV) according to any one of the foregoing remains intact in the extracellular environment, but is cleavable when, e.g., upon internalization into a cancer cell.

In some embodiments, the linker in the antibody-drug conjugate represented by formula (IV) according to any one of the foregoing comprises a cleavable moiety, wherein the cleavable moiety is located at a position such that no linker and Ab remain in the drug (e.g., an eribulin derivative) after cleavage.

In some specific embodiments, the cleavable moiety in the linker is a cleavable peptide moiety.

In some embodiments, the addition of a cleavable moiety increases cytotoxicity and/or potency relative to a non-cleavable linker. In some embodiments, the cleavable peptide moiety is cleavable by an enzyme, and the linker is one that is cleavable by an enzyme. In some embodiments, the linker is one that is cleavable by a cathepsin. In certain embodiments, the linker that is cleavable by an enzyme (e.g., the linker that is cleavable by a cathepsin) shows one or more of the improved properties described above.

In some embodiments, the linker comprises an amino acid unit (i.e., a peptide residue consisting of 2 to 7 amino acids), wherein preferably, the amino acids are selected from the group consisting of phenylalanine, glycine, valine, lysine, citrulline, serine, glutamic acid, and aspartic acid, and more preferably valine-citrulline (Val-Cit), alanine-alanine-asparagine (Ala-Ala-Asn), glycine-glycine-lysine (Gly-Gly-lys), valine-lysine (Val-lys), valine-alanine (Val-Ala), valine-phenylalanine (Val-Phe), and glycine-glycine-phenylalanine-glycine (Gly-Gly-Phe-Gly).

In some embodiments, the linker in the antibody-drug conjugate represented by formula (IV) of the present disclosure is selected from the group consisting of:

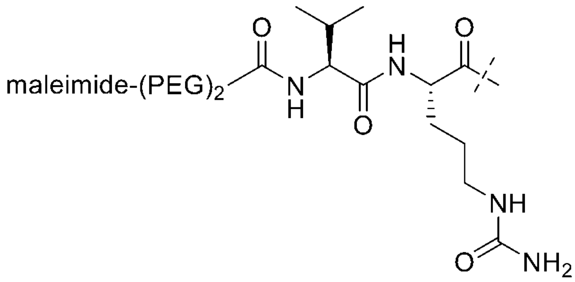
,

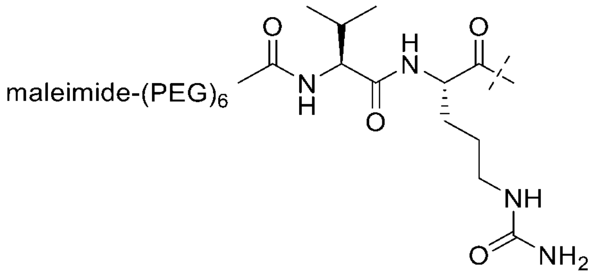
,

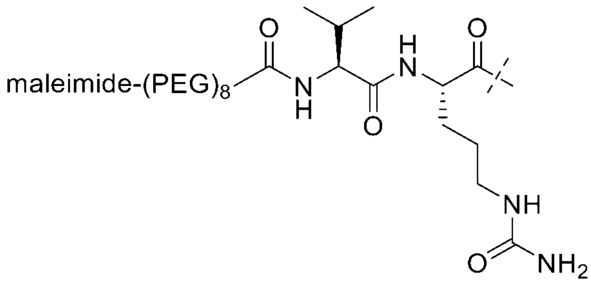
,

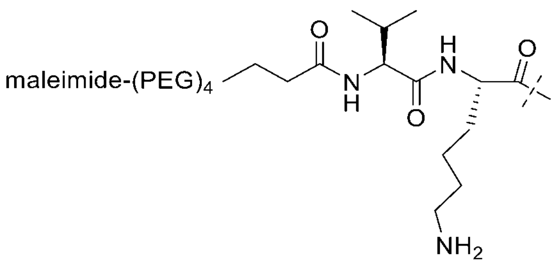
,

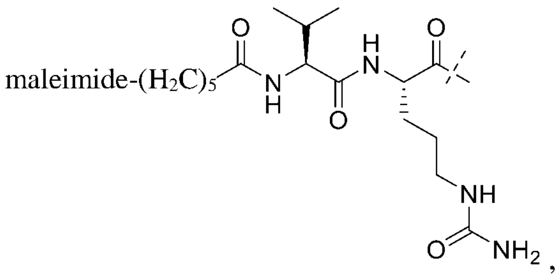

and

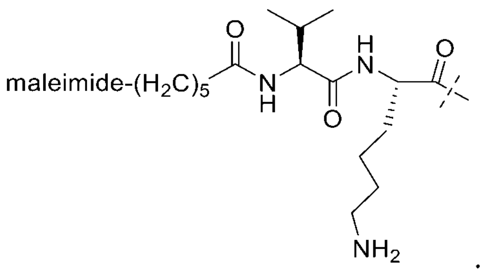

In some embodiments, the amino acid unit comprises valine-citrulline (Val-Cit).

In another aspect, a linker provided by some embodiments comprises a cleavable sulfonamide moiety, and the linker is cleavable under reducing conditions.

In some embodiments, the linker comprises a cleavable disulfide moiety, and the linker is cleavable under reducing conditions.

In another aspect, the linker of the present disclosure comprises at least one spacer unit that attaches De (e.g., an eribulin derivative) to a cleavable moiety.

In some embodiments, the spacer unit comprises p-aminobenzyloxycarbonyl (PAB),

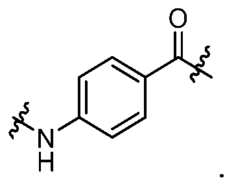

In another aspect, the antibody-drug conjugate represented by formula (IV) provided by some embodiments is represented by the following formula:

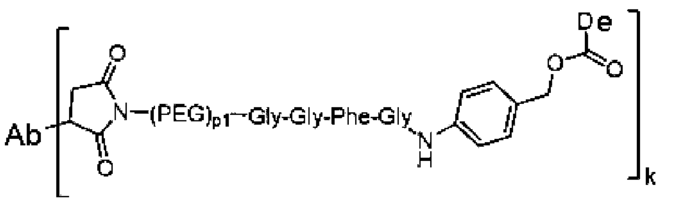

wherein k is selected from the group consisting of 1 to 10, and may be an integer or a decimal; and p1 is selected from the group consisting of 2, 4, 6, and 8;

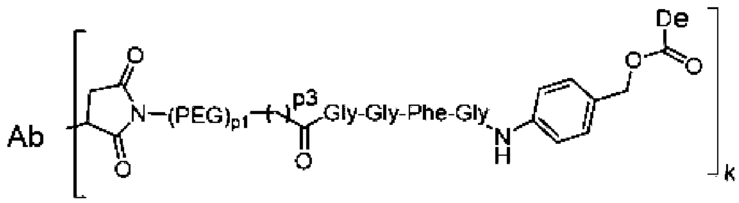

,

wherein k is selected from the group consisting of 1 to 10, and may be an integer or a decimal; p1 is selected from the group consisting of 2, 4, 6, and 8; and P3 is selected from the group consisting of 0, 1, and 2;

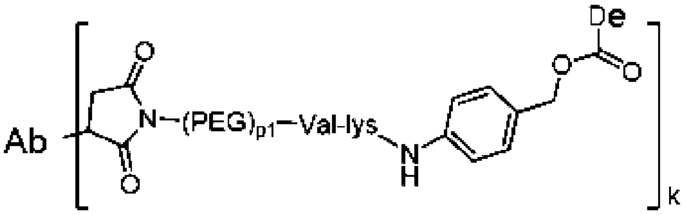

,

wherein k is selected from the group consisting of 1 to 10, and may be an integer or a decimal; and p1 is selected from the group consisting of 2, 4, 6, and 8;

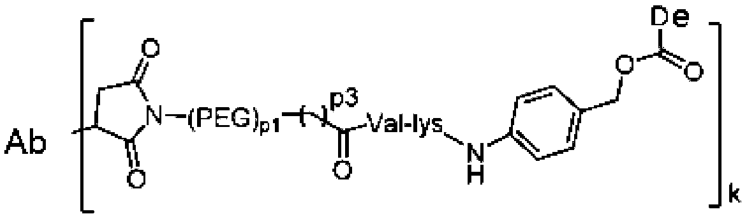

,

wherein k is selected from the group consisting of 1 to 10, and may be an integer or a decimal; p1 is selected from the group consisting of 2, 4, 6, and 8; and P3 is selected from the group consisting of 0, 1, and 2;

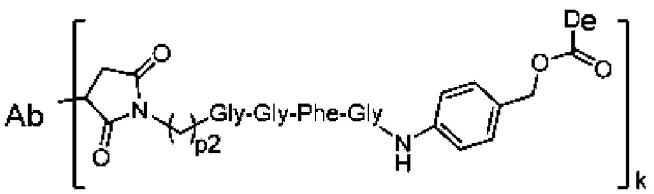

,

wherein k is selected from the group consisting of 1 to 10, and may be an integer or a decimal; and p2 is selected from the group consisting of 2, 4, 6, and 8;

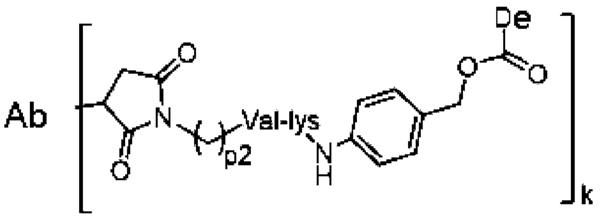

,

wherein k is selected from the group consisting of 1 to 10, and may be an integer or a decimal; and p2 is selected from the group consisting of 2, 4, 6, and 8;

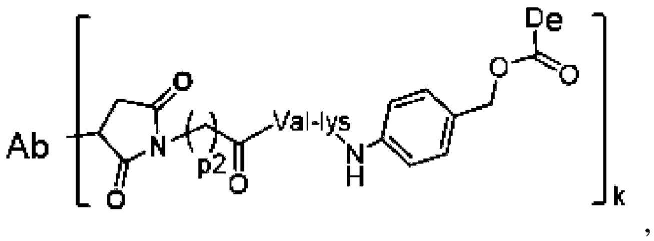

wherein k is selected from the group consisting of 1 to 10, and may be an integer or a decimal; and p2 is selected from the group consisting of 2, 4, 6, and 8;

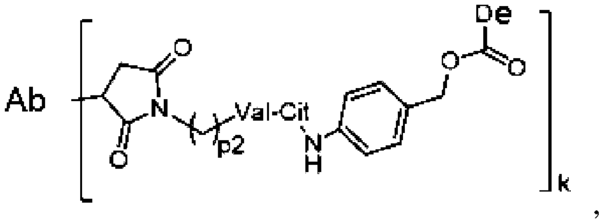

wherein k is selected from the group consisting of 1 to 10, and may be an integer or a decimal; and p2 is selected from the group consisting of 2, 4, 6, and 8;

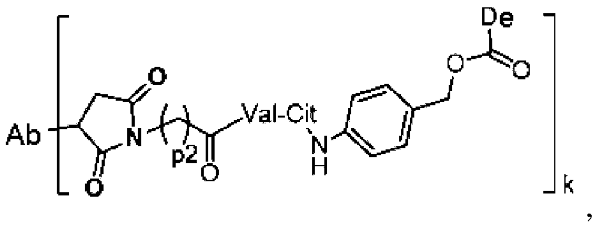

wherein k is selected from the group consisting of 1 to 10, and may be an integer or a decimal; and p2 is selected from the group consisting of 2, 4, 6, and 8;

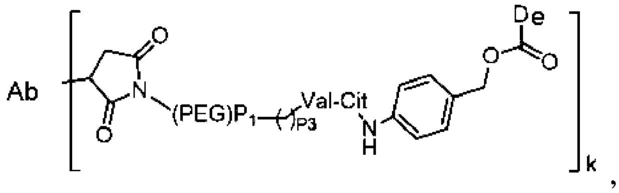

wherein k is selected from the group consisting of 1 to 10, and may be an integer or a decimal; P1 is selected from the group consisting of 2, 4, 6, and 8; and P3 is selected from the group consisting of 0, 1, and 2;

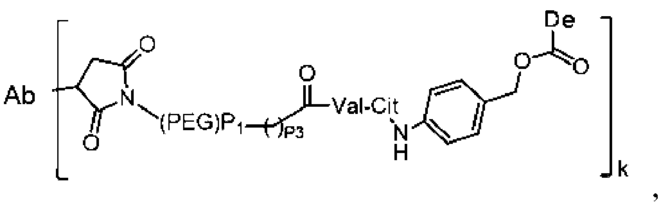

wherein k is selected from the group consisting of 1 to 10, and may be an integer or a decimal; P1 is selected from the group consisting of 2, 4, 6, and 8; and P3 is selected from the group consisting of 0, 1, and 2.

In some embodiments, the antibody-drug conjugate represented by formula (IV) according to any one of the foregoing is represented by the following formula:

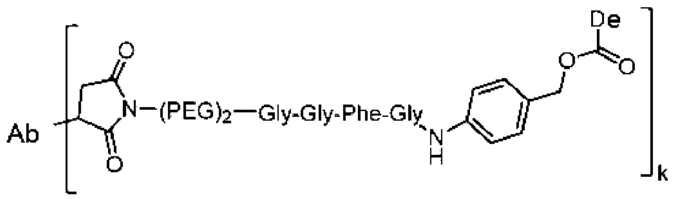

,

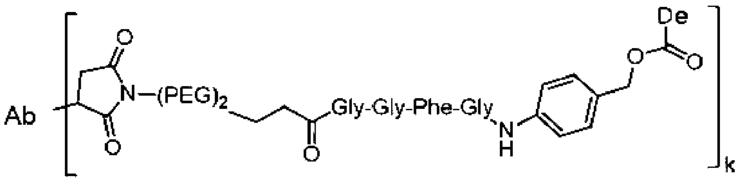

,

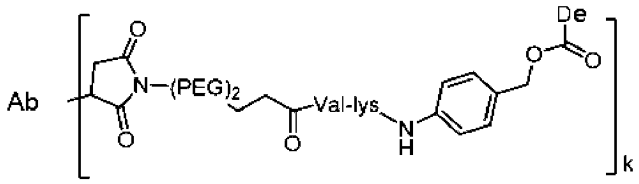

,

or

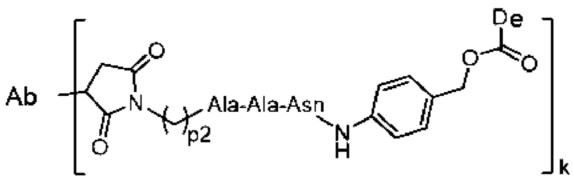

,

wherein k is selected from the group consisting of 1 to 10, and may be an integer or a decimal; further, $R^{1a}$ in De is preferably selected from the group consisting of methyl, and $R^{1b}$ is preferably selected from the group consisting of hydrogen.

In some specific embodiments, the antibody-drug conjugate of the present disclosure includes a tautomer, mesomer, racemate, enantiomer, diastereomer, or deuteride thereof, or a mixture thereof.

In some embodiments, the antibody-drug conjugate represented by general formula (I) or (IV) according to any one of the foregoing is an anti-HER3/MET antibody-drug conjugate,

wherein Ab is an antibody comprising a first binding domain that specifically binds to MET and a second binding domain that specifically binds to HER3; the first binding domain that specifically binds to MET comprises a heavy chain variable region (VH1) and a light chain variable region (VL1), and the second binding domain that specifically binds to HER3 comprises a heavy chain variable region (VH2) and a light chain variable region (VL2).

In some embodiments, the anti-HER3/MET antibody-drug conjugate represented by formula (I) or (IV) is provided, wherein Ab comprises:

a first heavy chain that is, from the N-terminus to the C-terminus, [VH1]-[linker 1]-[Obscurin-O chain]-[linker 3]-[first subunit of Fc region],
a first light chain that is, from the N-terminus to the C-terminus, [VL1]-[linker 2]-[Titin-T chain],
a second heavy chain that is, from the N-terminus to the C-terminus, [VH2]-[CH1]-[second subunit of Fc region], and
a second light chain that is, from the N-terminus to the C-terminus, [VL2]-[CL];
or
a first heavy chain that is, from the N-terminus to the C-terminus, [VH2]-[linker 1]-[Obscurin-O chain]-[linker 3]-[first subunit of Fc region],
a first light chain that is, from the N-terminus to the C-terminus, [VL2]-[linker 2]-[Titin-T chain],
a second heavy chain that is, from the N-terminus to the C-terminus, [VH1]-[CH1]-[second subunit of Fc region], and
a second light chain that is, from the N-terminus to the C-terminus, [VL1]-[CL];
wherein - represents a peptide bond, and the linker 1, the linker 2, and the linker 3 may be identical or different, and may

be independently present or absent.

In some embodiments, the Titin-T chain is set forth in SEQ ID NO: 96.

GIPPKIEALPSDISIDEGKVLTVACAFTGEPTPEVTWSCGGRKIHSQEQGRFHIEN
TDDLTTLIIMDVQKQDGGLYTLSLGNEFGSDSATVNIHIRSI

(SEQ ID NO: 96).

In some embodiments, the Titin-T chain is a variant of SEQ ID NO: 96, and the variant comprises amino acid residue substitution(s) at one or more positions selected from the group consisting of positions 3, 8, 11, 13, 20, 22, 25, 26, 39, 40, 42, 45, 47, 49, 56, 58, 60, 64, 66, 70, 75, 77, 79, 81, 82, 83, and 84. Illustratively, the variant comprises one or more amino acid residue substitutions selected from the group consisting of 3W, 8C, 11I, 13L, 20C, 22M/22C, 25S, 26C, 39T, 40S, 42K, 45S, 47E, 49G, 56S, 58E, 60S, 64T, 66S/66K, 70R, 75V, 77S, 79T, 81R, 82M, 83D, and 84L. In some specific embodiments, the Titin-T chain is set forth in SEQ ID NO: 64 or is an amino acid sequence having at least 80% sequence identity thereto.

In some embodiments, the Obscurin-O chain is set forth in SEQ ID NO: 97,

SGAPRFLTRPKAFVVSVGKDATLSCQIVGNPTPQVSWEKDQQPVAAGARFRLA
QDGDLYRLTILDLALGDSGQYVCRARNAIGEAFAAVGLQVDAEA

(SEQ ID NO: 97).

In some embodiments, the Obscurin-O chain is a variant of SEQ ID NO: 97, and the variant comprises amino acid residue substitution(s) at one or more positions selected from the group consisting of positions 2, 3, 7, 9, 11, 12, 13, 14, 17, 20, 22, 25, 30, 32, 34, 36, 41, 42, 44, 45, 48, 53, 58, 62, 66, 67, 69, 76, 82, 88, 89, 92, 93, 94, and 97. Illustratively, the variant comprises one or more amino acid residue substitutions selected from the group consisting of 2E, 3C, 7K/7R, 9C, 11L, 12S, 13Y/13S, 14T, 17E, 20L, 22M/22S, 25S, 30D, 32P/32F, 34E, 36T, 41K, 42L, 44I, 45T, 48V, 53L, 58V, 62E/62K/62H, 66C, 67Q/67T, 69S, 76S, 82H, 88C, 89L, 92E, 93C, 94G, and 97G. In some specific embodiments, the Obscurin-O chain is set forth in SEQ ID NO: 63 or is an amino acid sequence having at least 80% sequence identity thereto.

In some embodiments, the heavy chain variable region in the first binding domain that specifically binds to MET comprises a HCDR1, a HCDR2, and a HCDR3 of the amino acid sequences set forth in SEQ ID NOs: 15-17, and the light chain variable region comprises a LCDR1, a LCDR2, and a LCDR3 of the amino acid sequences set forth in SEQ ID NOs: 76, 19, and 20; the heavy chain variable region in the second binding domain that specifically binds to HER3 comprises a HCDR1, a HCDR2, and a HCDR3 of the amino acid sequences set forth in SEQ ID NOs: 51-53, and the light chain variable region comprises a LCDR1, a LCDR2, and a LCDR3 of the amino acid sequences set forth in SEQ ID NOs: 54, 55, and 75.

In some embodiments, the heavy chain variable region in the first binding domain that specifically binds to MET comprises a HCDR1, a HCDR2, and a HCDR3 of the amino acid sequences set forth in SEQ ID NOs: 15-17, and the light chain variable region comprises a LCDR1 of the amino acid sequence set forth in SEQ ID NO: 18 or 74, and a LCDR2 and a LCDR3 of the amino acid sequences set forth in SEQ ID NOs: 19 and 20; the heavy chain variable region in the second binding domain that specifically binds to HER3 comprises a HCDR1, a HCDR2, and a HCDR3 of the amino acid sequences set forth in SEQ ID NOs: 51-53, and the light chain variable region comprises a LCDR1 and a LCDR2 of the amino acid sequences set forth in SEQ ID NOs: 54 and 55, and a LCDR3 of the amino acid sequence set forth in SEQ ID NO: 56 or 73.

In some embodiments, the heavy chain variable region in the first binding domain that specifically binds to MET comprises the amino acid sequence set forth in SEQ ID NO: 71 or 1 or an amino acid sequence having at least 90% identity thereto, and the light chain variable region comprises the amino acid sequence set forth in SEQ ID NO: 72 or 2 or an amino acid sequence having at least 90% identity thereto; the heavy chain variable region in the second binding domain that specifically binds to HER3 comprises the amino acid sequence set forth in SEQ ID NO: 13 or an amino acid sequence having at least 90% identity thereto, and the light chain variable region comprises the amino acid sequence set forth in SEQ ID NO: 70 or 14 or an amino acid sequence having at least 90% identity thereto.

In some embodiments, Ab comprises a combination of polypeptide chains set forth in SEQ ID NOs: 77, 78, 59, and 79, or comprises a combination of polypeptide chains set forth in SEQ ID NOs: 57-60.

In some embodiments, the antibody-drug conjugate represented by general formula (I) according to any one of the foregoing is provided, wherein the antibody-drug conjugate is selected from the group consisting of:

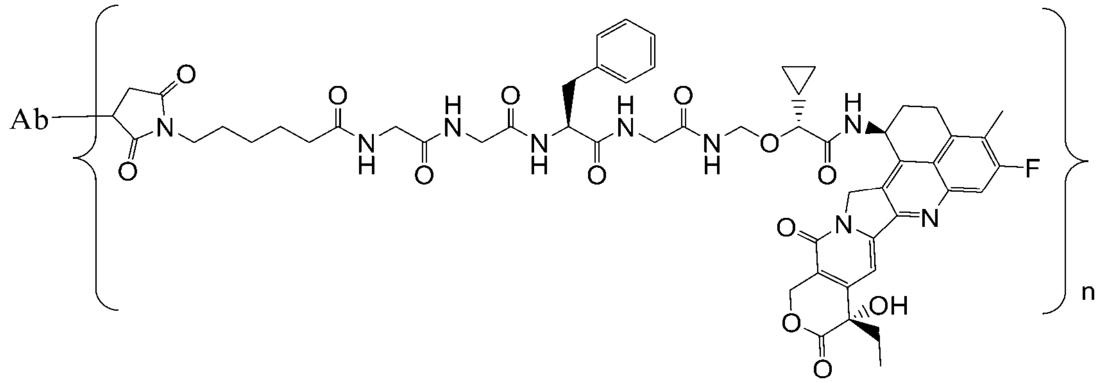

wherein:
n is 1 to 8, n is a decimal or an integer, e.g., an integer or a decimal from 3 to 7, and illustratively, n is about 6; Ab comprises a combination of polypeptide chains set forth in SEQ ID NOs: 77, 78, 59, and 79, or comprises a combination of polypeptide chains set forth in SEQ ID NOs: 57-60.

In some embodiments, the antibody-drug conjugate represented by general formula (IV) according to any one of the foregoing is provided, wherein the antibody-drug conjugate is selected from the group consisting of:

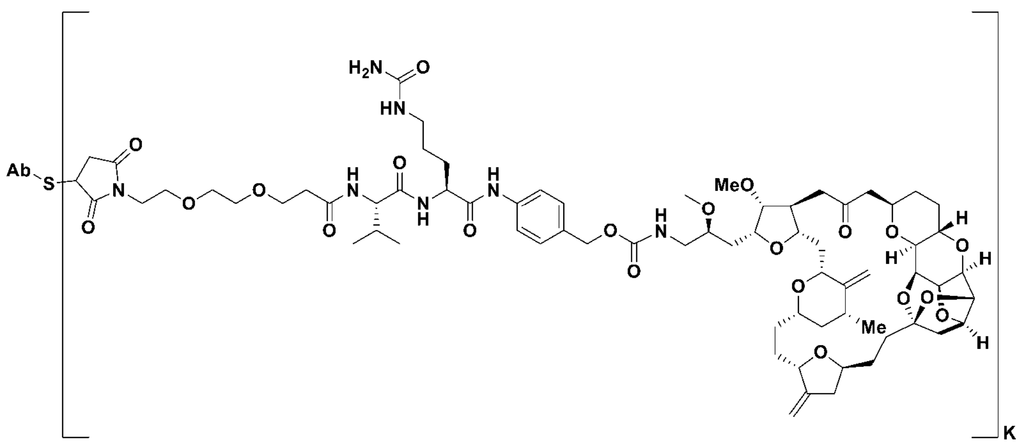

wherein:
k is 1 to 8, k is a decimal or an integer, e.g., an integer or a decimal from 3 to 7, and illustratively, k is about 4 or 6; Ab comprises a combination of polypeptide chains set forth in SEQ ID NOs: 77, 78, 59, and 79, or comprises a combination of polypeptide chains set forth in SEQ ID NOs: 57-60.

In some embodiments, the antibody-drug conjugate represented by general formula (I) or (IV) according to any one of the foregoing is an anti-MET antibody-drug conjugate, wherein Ab is an antibody comprising a binding domain that specifically binds to MET, and the binding domain that specifically binds to MET comprises a heavy chain variable region (VH1) and a light chain variable region (VL1).

In some embodiments, in the anti-MET antibody-drug conjugate represented by formula (I) or (IV), the heavy chain variable region of the binding domain that specifically binds to MET comprises a HCDR1, a HCDR2, and a HCDR3 from the amino acid sequence set forth in SEQ ID NO: 71; and the light chain variable region comprises a LCDR1, a LCDR2, and a LCDR3 from the amino acid sequence set forth in SEQ ID NO: 72.

In some embodiments, in the anti-MET antibody-drug conjugate represented by formula (I) or (IV), the heavy chain variable region of the binding domain that specifically binds to MET comprises a HCDR1, a HCDR2, and a HCDR3 of the amino acid sequences set forth in SEQ ID NOs: 15-17, and the light chain variable region comprises a LCDR1, a LCDR2, and a LCDR3 of the amino acid sequences set forth in SEQ ID NOs: 74, 19, and 20.

In some embodiments, the binding domain that specifically binds to MET comprises one or more amino acid mutations selected from the group consisting of: 23K in the heavy chain variable region, 78T in the heavy chain variable region, and 69T in the light chain variable region, illustratively, VH_E23K, VH_S78T, and VL_A69T mutations. In some embodiments, in the anti-MET antibody-drug conjugate represented by formula (I) or (IV), the heavy chain variable region of the binding domain that specifically binds to MET comprises the amino acid sequence set forth in SEQ ID NO: 71 or an amino acid sequence having at least 80% identity thereto, and the light chain variable region comprises the amino acid

sequence set forth in SEQ ID NO: 72 or an amino acid sequence having at least 80% identity thereto.

In some embodiments, the anti-MET antibody-drug conjugate represented by general formula (I) according to any one of the foregoing is provided, wherein the anti-MET antibody-drug conjugate is selected from the group consisting of:

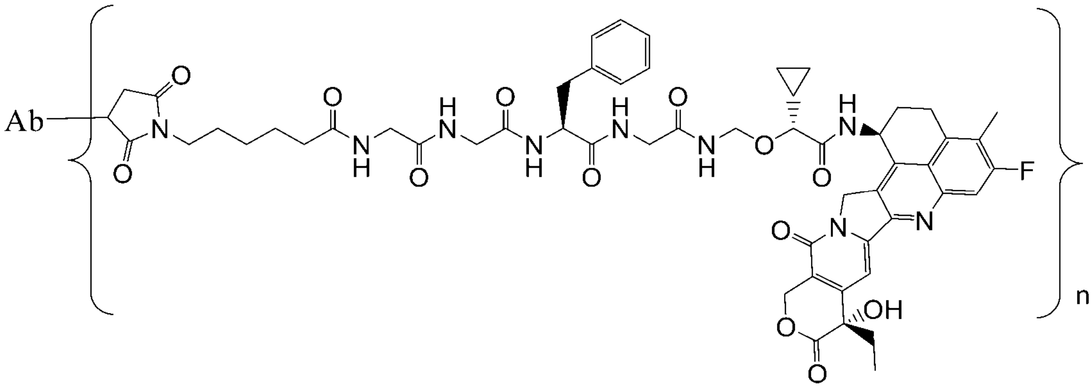

,

wherein:

n is 1 to 8, n is a decimal or an integer, e.g., an integer or a decimal from 3 to 7, and illustratively, n is about 6; Ab comprises a heavy chain variable region set forth in SEQ ID NO: 71 and a light chain variable region set forth in SEQ ID NO: 72.

In some embodiments, the anti-MET antibody-drug conjugate represented by general formula (IV) according to any one of the foregoing is provided, wherein the anti-MET antibody-drug conjugate is selected from the group consisting of:

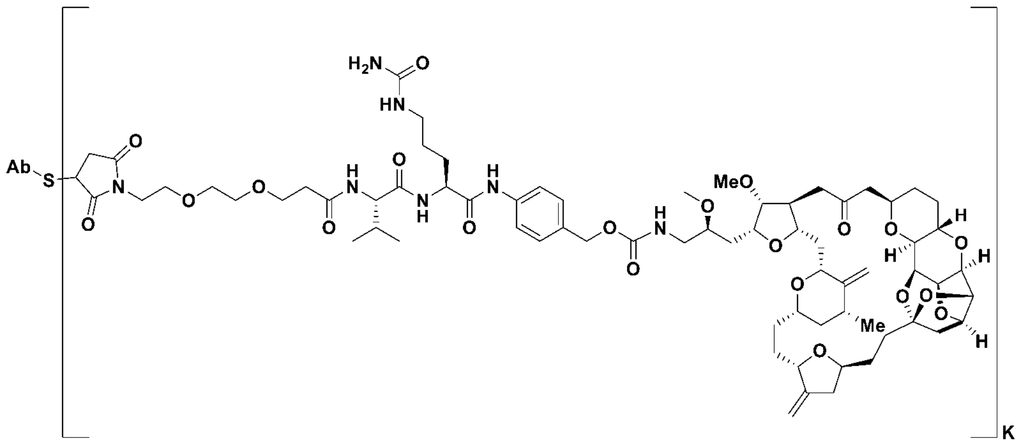

k is 1 to 8, k is a decimal or an integer, e.g., an integer or a decimal from 3 to 7, and illustratively, k is about 4 or 6; Ab comprises a heavy chain variable region set forth in SEQ ID NO: 71 and a light chain variable region set forth in SEQ ID NO: 72.

In some embodiments, the binding molecule represented by general formula (I) according to any one of the foregoing is an anti-HER3 antibody-drug conjugate,

wherein Ab is an antibody comprising a binding domain that specifically binds to HER3, and the binding domain that specifically binds to HER3 comprises a heavy chain variable region (VH2) and a light chain variable region (VL2).

In some embodiments, in the anti-HER3 antibody-drug conjugate represented by formula (I), the heavy chain variable region of the binding domain that specifically binds to HER3 comprises a HCDR1, a HCDR2, and a HCDR3 from the amino acid sequence set forth in SEQ ID NO: 13, and the light chain variable region comprises a LCDR1, a LCDR2, and a LCDR3 from the amino acid sequence set forth in SEQ ID NO: 70.

In some embodiments, in the anti-HER3 antibody-drug conjugate represented by formula (I), the heavy chain

variable region of the binding domain that specifically binds to HER3 comprises a HCDR1, a HCDR2, and a HCDR3 of the amino acid sequences set forth in SEQ ID NOs: 51-53, and the light chain variable region comprises a LCDR1, a LCDR2, and a LCDR3 of the amino acid sequences set forth in SEQ ID NOs: 54, 55, and 73.

In some embodiments, in the anti-HER3 antibody-drug conjugate represented by formula (I), the heavy chain variable region of the binding domain that specifically binds to HER3 comprises the amino acid sequence set forth in SEQ ID NO: 13 or an amino acid sequence having at least 80% identity thereto, and the light chain variable region comprises the amino acid sequence set forth in SEQ ID NO: 70 or an amino acid sequence having at least 80% identity thereto.

In some embodiments, the anti-HER3 antibody-drug conjugate represented by general formula (I) according to any one of the foregoing is provided, wherein the anti-HER3 antibody-drug conjugate is selected from the group consisting of:

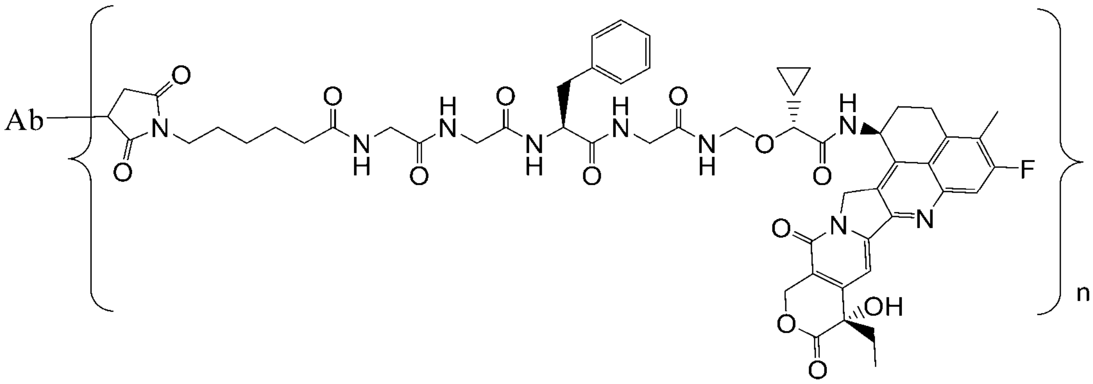

,

wherein:

n is 1 to 8, n is a decimal or an integer, e.g., an integer or a decimal from 3 to 7, and illustratively, n is about 6;
Ab comprises a heavy chain variable region set forth in SEQ ID NO: 13 and a light chain variable region set forth in SEQ ID NO: 70.

In some embodiments, the anti-HER3 antibody-drug conjugate represented by general formula (IV) according to any one of the foregoing is provided, wherein the anti-HER3 antibody-drug conjugate is selected from the group consisting of:

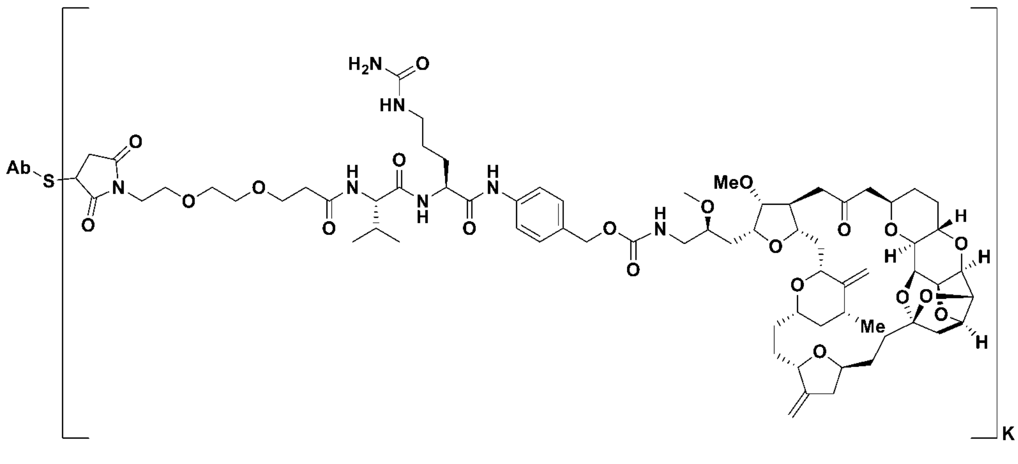

k is 1 to 8, k is a decimal or an integer, e.g., an integer or a decimal from 3 to 7, and illustratively, k is about 4 or 6;
Ab comprises a heavy chain variable region set forth in SEQ ID NO: 13 and a light chain variable region set forth in SEQ ID NO: 70.

In some embodiments, the aforementioned HER3/MET-binding molecule has at least one of the following properties:

a) specifically binding to MET or an epitope thereof; in some embodiments, binding to human MET at a $K_D$ value ≤ $10^7$,

wherein methods for determining the $K_D$ value are commonly used in the art, such as the one in Example 2 of the present disclosure;

b) specifically binding to HER3 or an epitope thereof; in some embodiments, binding to human HER3 at a $K_D$ value ≤ $10^7$, wherein methods for determining the $K_D$ value are commonly used in the art, such as the one in Example 2 of the present disclosure;

c) having good binding activity to cells co-expressing MET and HER3, e.g., with $EC_{50}$ ≤ 5 nM, illustratively, with $EC_{50}$ ≤ 4 nM, $EC_{50}$ ≤ 3 nM, $EC_{50}$ ≤ 2 nM, $EC_{50}$ ≤ 1 nM, $EC_{50}$ ≤ 0.9 nM, $EC_{50}$ ≤ 0.8 nM, $EC_{50}$ ≤ 0.7 nM, $EC_{50}$ ≤ 0.6 nM, $EC_{50}$ ≤ 0.5 nM, $EC_{50}$ ≤ 0.4 nM, $EC_{50}$ ≤ 0.3 nM, or lower, wherein methods for determining the $EC_{50}$ value are commonly used in the art, such as the one in Example 3 of the present disclosure;

d) having good endocytic activity in cells co-expressing MET and HER3, e.g., with $EC_{50}$ ≤ 1 nM, illustratively, with $EC_{50}$ ≤ 0.9 nM, $EC_{50}$ ≤ 0.8 nM, $EC_{50}$ ≤ 0.7 nM, $EC_{50}$ ≤ 0.6 nM, $EC_{50}$ ≤ 0.5 nM, $EC_{50}$ ≤ 0.4 nM, $EC_{50}$ ≤ 0.3 nM, $EC_{50}$ ≤ 0.2 nM, or lower, wherein methods for determining the $EC_{50}$ value are commonly used in the art, such as the one in Example 4 of the present disclosure;

e) having an endocytosis rate in cells co-expressing MET and HER3 superior to that of the anti-HER3 antibody or the anti-MET antibody;

f) not causing HER3/MET target dimerization to activate downstream signaling pathways;

g) inhibiting tumor growth, or treating or alleviating cancer.

In some embodiments, the aforementioned HER3/MET-binding molecule of the present disclosure is capable of inhibiting tumor growth by at least about 10%, e.g., at least about 20%, about 30%, about 40%, about 50%, about 60%, about 70%, about 80%, or about 90%.

In some embodiments, the HER3/MET-binding molecule of the present disclosure is an anti-HER3/MET antibody, e.g., an anti-HER3/MET bispecific antibody or an anti-HER3/MET multispecific antibody.

The antibody comprises an antigen-binding fragment, and the antigen-binding fragment includes, but is not limited to: a Fab, an Fv, an sFv, a Fab', a F(ab')2, a linear antibody, a single-chain antibody, an scFv, an sdAb, an sdFv, a nanobody, a peptibody, a domain antibody, a diabody, a triabody, a tetrabody, a tandem di-scFv, and a tandem tri-scFv. In some specific embodiments, the antigen-binding fragment includes a Fab, an Fv, an sFv, a Fab', and a F(ab')2.

In some embodiments, provided is an anti-HER3/MET antibody that binds to or competes for binding to MET and/or HER3, or binds to or competes for binding to the same epitope of MET and/or HER3, with the aforementioned HER3/MET-binding molecule of the present disclosure.

In some embodiments, provided is an anti-HER3/MET antibody that blocks the binding of the aforementioned HER3/MET-binding molecule of the present disclosure to MET and/or HER3.

## Polynucleotide and Vector

The present disclosure provides a polynucleotide encoding the HER3/MET-binding molecule, the MET-binding molecule, and the HER3-binding molecule provided by the present disclosure.

In some embodiments, the polynucleotide may be RNA, DNA, or cDNA. According to some embodiments of the present disclosure, the polynucleotide of the present disclosure is a substantially isolated nucleic acid.

The nucleic acid of the present disclosure may also be in the form of a vector and may be present in the vector and/or may be part of the vector. The vector is, for example, a plasmid, cosmid, YAC, or viral vector. The vector may especially be an expression vector, i.e., a vector that provides for *in vitro* and/or *in vivo* (i.e., in suitable host cells, host organisms, and/or expression systems) expression of the HER3/MET-binding molecule, the MET-binding molecule, and the HER3-binding molecule. The expression vector generally comprises at least one of the nucleic acids of the present disclosure, which is operably linked to one or more suitable expression regulatory elements (e.g., promoters, enhancers, and terminators). The selection of the elements and sequences thereof for expression in a particular host is common knowledge of those skilled in the art. Regulatory elements and other elements useful or necessary for expression of the HER3/MET-binding molecule of the present disclosure include, for example, promoters, enhancers, terminators, integration factors, selection labels, leader sequences, and reporter genes.

The nucleic acid of the present disclosure may be prepared or obtained by known means (e.g., by automatic DNA synthesis and/or recombinant DNA techniques) based on information about the amino acid sequences of the polypeptides of the present disclosure, and/or may be isolated from a suitable natural source.

## Host Cell

The present disclosure provides a recombinant host cell that expresses or is capable of expressing one or more of the HER3/MET-binding molecule, the MET-binding molecule, and the HER3-binding molecule of the present disclosure, and/or comprises the nucleic acid or vector of the present disclosure.

In some embodiments, the host cell is a bacterial cell, a fungal cell, or a mammalian cell.

Illustratively, the bacterial cell includes, for example, cells of gram-negative bacterial strains (e.g., *Escherichia coli* strains, *Proteus* strains, and *Pseudomonas* strains) and gram-positive bacterial strains (e.g., *Bacillus* strains, *Streptomyces* strains, *Staphylococcus* strains, and *Lactococcus* strains).

Illustratively, the fungal cell includes, for example, cells of the species *Trichoderma, Neurospora,* and *Aspergillus,* or cells of the species *Saccharomyces* (e.g., *Saccharomyces cerevisiae*), *Schizosaccharomyces* (e.g., *Schizosaccharomyces pombe*), *Pichia* (e.g., *Pichia pastoris* and *Pichia methanolica*), and *Hansenula.*

Illustratively, the mammalian cell includes, for example, HEK293 cells, CHO cells, BHK cells, HeLa cells, COS cells, and the like.

Amphibian cells, insect cells, plant cells, and any other cells used in the art for expressing heterologous proteins may also be used in the present disclosure.

The cell of the present disclosure is unable to develop into a complete plant or animal individual.

<u>Production or Preparation Method</u>

The present disclosure provides a method for preparing the HER3/MET-binding molecule, the MET-binding molecule, and the HER3-binding molecule of the present disclosure.

In some embodiments, the method comprises the following steps:

- culturing the host cell of the present disclosure under conditions suitable for the expression of the antibody; and
- isolating from the culture the protein of interest expressed by the host cell; and
- optionally, further purifying and/or modifying the protein of interest of the present disclosure.

The HER3/MET-binding molecule, the MET-binding molecule, and the HER3-binding molecule of the present disclosure may be produced intracellularly (e.g., in the cytoplasm, in the periplasm, or in inclusion bodies) in a cell as described above, followed by isolation from the host cell and optionally further purification; or it may be produced extracellularly (e.g., in the medium in which the host cell is cultured), followed by isolation from the medium and optionally further purification.

Methods and reagents for recombinant production of polypeptides, e.g., specific suitable expression vectors, transformation or transfection methods, selection labels, methods for inducing protein expression, culture conditions, and the like, are known in the art. Similarly, isolation and purification techniques suitable for use in the manufacture of proteins of interest, such as the binding molecules or antibodies of the present disclosure, are well known to those skilled in the art. Methods for producing and purifying antibodies are well known in the prior art and can be found in, for example, "Antibodies: A Laboratory Manual", Cold Spring Harbor Press (chapters 5-8 and 15). The engineered antibodies of the present disclosure may also be prepared and purified by conventional methods. For example, cDNA sequences encoding heavy and light chains can be cloned and recombined into an expression vector. Recombinant immunoglobulin expression vectors can be used to stably transfect cells. Mammalian expression systems may result in glycosylation of antibodies, particularly at the highly conserved N-terminus of the Fc region. Stable clones are obtained by expression of antibodies specifically binding to human antigens. Positive clones are expanded in a serum-free culture medium in a bioreactor to produce antibodies. The culture solution with the secreted antibodies can be purified and collected by conventional techniques. The antibodies can be filtered and concentrated by conventional methods. Soluble mixtures and multimers can also be removed by conventional methods, such as molecular sieves and ion exchange. The resulting products need to be immediately frozen, such as at -70 °C, or lyophilized.

However, the HER3/MET-binding molecule, MET-binding molecule, and the HER3-binding molecule of the present disclosure may also be obtained by other methods for producing proteins known in the art, such as chemical synthesis, including solid-phase or liquid-phase synthesis.

The present disclosure further provides a method for preparing the aforementioned antibody-drug conjugate represented by formula (I) or (IV), comprising the following steps:

subjecting the aforementioned Ab to a coupling reaction with a drug to give the antibody-drug conjugate represented by formula (I) or (IV); and

optionally, purifying the antibody-drug conjugate represented by formula (I) or (IV).

In some embodiments, a method for preparing the antibody-drug conjugate represented by formula (II) is provided, comprising the following step:

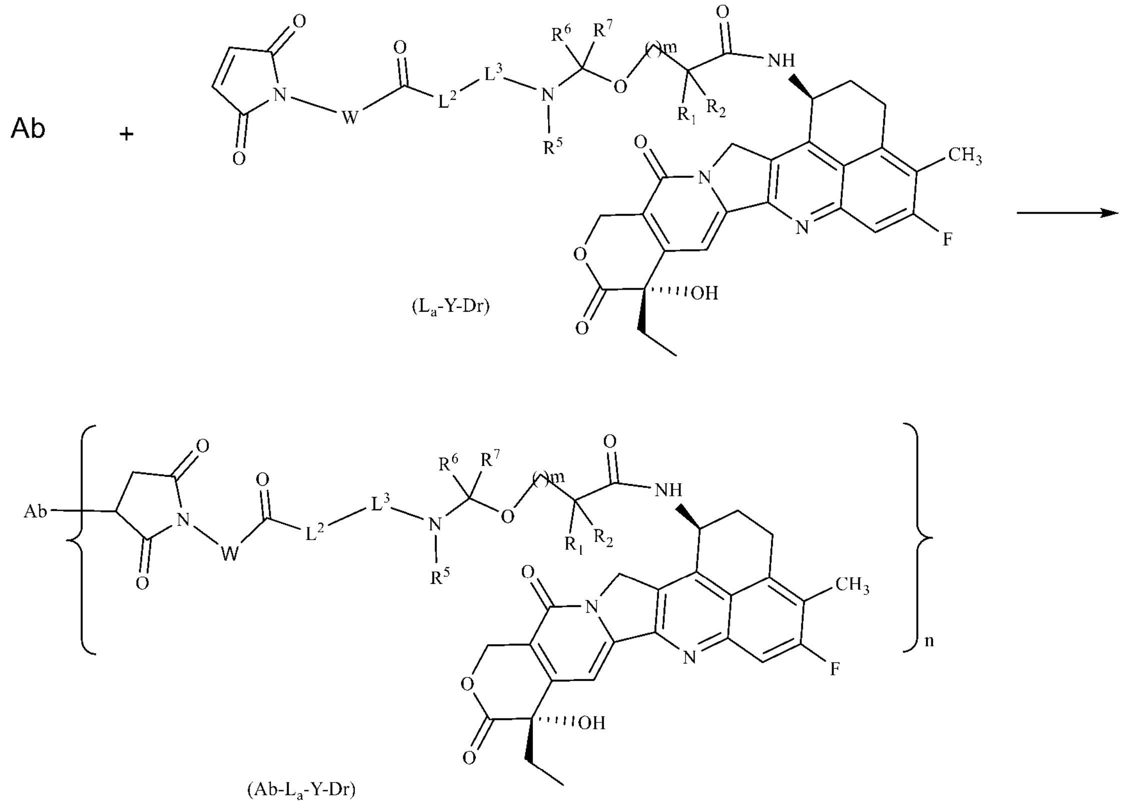

($L_a$-Y-Dr)

(Ab-$L_a$-Y-Dr)

subjecting reduced Ab to a coupling reaction with general formula (La-Y-Dr) to give a compound represented by general formula (Ab-La-Y-Dr), wherein the reductant is preferably TCEP; in particular, the disulfide bonds in the antibody are preferably reduced,

wherein Ab, W, L2, L3, R1, R2, R5 to R7, m, and n are as defined in formula (I).

Composition

The present disclosure provides a composition comprising any one or any combination of the following: any HER3/MET-binding molecule, MET-binding molecule, HER3-binding molecule, polynucleotide encoding a HER3/MET-binding molecule, a MET-binding molecule, or a HER3-binding molecule, and vector provided by the present disclosure.

In some embodiments, the pharmaceutical composition comprises an amount of the HER3/MET-binding molecule, the MET-binding molecule, the HER3-binding molecule, the coding polynucleotide, or the vector described above that is effective in treating, alleviating, or preventing cancer.

In some embodiments, the pharmaceutical composition comprises or consists of a mixture of HER3/MET-binding molecules represented by formula (I) with different n values, wherein at least 65%, illustratively, at least 70%, at least 75%, at least 80%, or at least 90%, of the HER3/MET-binding molecules represented by formula (I) have n of 6.

In some embodiments, the pharmaceutical composition further comprises at least one pharmaceutically acceptable excipient, diluent, or carrier.

In some specific embodiments, a unit dose of the pharmaceutical composition may comprise 0.01 to 99 wt% of the HER3/MET-binding molecule, the MET-binding molecule, or the HER3-binding molecule, or a unit dose of the pharmaceutical composition comprises 0.1-2000 mg of the HER3/MET-binding molecule, the MET-binding molecule, or the HER3-binding molecule. In some specific embodiments, a unit dose of the pharmaceutical composition comprises 1-1000 mg of the HER3/MET-binding molecule, the MET-binding molecule, or the HER3-binding molecule.

In some embodiments, provided is an article of manufacture or product comprising the aforementioned HER3/MET-binding molecule, MET-binding molecule, HER3-binding molecule, polynucleotide, and/or vector. Optionally, the article of manufacture comprises a container and a label. The container is, for example, a bottle, a syringe, and a test tube. The container accommodates a composition effective in treating a disorder. The label on or associated with the container indicates that the composition is used for treating the disorder of choice. The composition comprises the aforementioned HER3/MET-binding molecule, MET-binding molecule, HER3-binding molecule, polynucleotide, and/or vector.

Kit and Detection

The present disclosure provides a kit comprising the aforementioned HER3/MET-binding molecule, MET-binding molecule, HER3-binding molecule, polynucleotide, vector, or composition. The present disclosure further provides a method, system, or device for detecting MET or HER3 *in vivo* or *in vitro,* comprising treating a sample with the aforementioned binding molecule, polynucleotide, vector, or composition of the present disclosure.

In some embodiments, the method, system, or device for *in vitro* detection may comprise, for example,

(1) contacting the sample with the HER3/MET-binding molecule, the MET-binding molecule, the HER3-binding molecule, the polynucleotide, the vector, or the composition;
(2) detecting a complex formed between the aforementioned binding molecule, polynucleotide, or vector and the sample; and/or
(3) contacting a reference sample (e.g., a control sample) with the binding molecule or the polynucleotide; and
(4) determining the extent of formation of the complex by comparison with the reference sample. A change (e.g., a statistically significant change) in the formation of the complex in the sample or subject as compared to a control sample or subject indicates the presence of MET or HER3 in the sample.

In some other embodiments, the method, system, or device for *in vivo* detection may comprise:

(1) administering to a subject the aforementioned binding molecule, polynucleotide, or vector; and
(2) detecting the formation of a complex between the aforementioned binding molecule, polynucleotide, or vector and the subject.

The detection may comprise determining the location or time at which the complex is formed. The aforementioned binding molecule or nucleic acid may be labeled with a detectable substance, and the detection of a substance capable of binding to the protein or nucleic acid (e.g., MET or HER3) is achieved by detecting the label. Suitable detectable substances include various enzymes, prosthetic groups, fluorescent substances, luminescent substances, and radioactive substances. The formation of a complex from the binding molecule or nucleic acid and MET or HER3 can be detected by measuring or visualizing a substance that binds or does not bind to MET or HER3. Conventional detection methods may be used, such as enzyme-linked immunosorbent assay (ELISA), radioimmunoassay (RIA), or tissue immunohistochemistry. For detection purposes, the binding molecule or nucleic acid of the present disclosure may be labeled with a fluorophore chromophore. In some embodiments, further provided are a diagnostic reagent comprising the nucleic acid or binding molecule described above, and related diagnostic use.

In some embodiments, further provided is a kit comprising the aforementioned binding molecule or polynucleotide, and the kit may further comprise instructions for diagnostic use. The kit may further comprise at least one additional reagent, such as a label or an additional diagnostic agent. For *in vivo* use, the binding molecule may be formulated into a pharmaceutical composition.

Method for Treating Disease and Pharmaceutical Use

Provided are use and a method of the HER3/MET-binding molecule, the MET-binding molecule, the HER3-binding molecule, the coding polynucleotide, the vector, or the pharmaceutical composition provided by the present disclosure in the prevention, treatment, or alleviation of a disease or disorder.

In some embodiments, the present disclosure provides the HER3/MET-binding molecule, the MET-binding molecule, the HER3-binding molecule, or the coding polynucleotide, the vector, or the pharmaceutical composition for use in treating or alleviating cancer, or for use in manufacturing a medicament for treating or alleviating cancer.

In some embodiments, the present disclosure provides a method for preventing, treating, or alleviating a cancer or tumor, comprising administering to a patient or subject an amount of the HER3/MET-binding molecule, the MET-binding molecule, the HER3-binding molecule, or the coding polynucleotide, the vector, or the pharmaceutical composition of the present disclosure that is effective in preventing, treating, or alleviating the disease or disorder.

In some specific embodiments, the tumor or cancer is HER3-positive. In some specific embodiments, the tumor or cancer is HER3/MET double-positive.

In some specific embodiments, the tumor or cancer is resistant to EGFR-TKI (epidermal growth factor receptor tyrosinase inhibitor).

In some embodiments, the HER3/MET-binding molecule, the MET-binding molecule, the HER3-binding molecule, or the coding polynucleotide, the vector, or the pharmaceutical composition of the present disclosure can be administered by any suitable method known in the art, and the administration may be systemic or local.

In some embodiments, the administration regimen may be adjusted to provide the optimum desired response

(e.g., a therapeutic or prophylactic response). For example, a single dose may be administered, several doses may be administered over a period of time, or the dose may be proportionally reduced or increased depending on the exigencies of the therapeutic situation.

## BRIEF DESCRIPTION OF THE DRAWINGS

FIGs. 1A-1C show HER3/MET dual-target IHC staining of tumor samples in patients with EGFR-TKI-resistant non-small cell lung cancer.
FIG. 2 shows tandem mass spectrometry results of antibody A and antibody A T94V.
FIG. 3 shows the binding of HER3/MET bispecific antibodies to MET single-positive cells as assayed by FACS.
FIG. 4A shows the binding of antibodies to HER3 single-positive cells as assayed by FACS. FIG. 4B shows the binding of antibodies to MET single-positive cells as assayed by FACS. FIG. 4C shows the binding of antibodies to HER3/MET double-positive cells as assayed by FACS.
FIG. 5A shows the killing of HER3/MET double-positive cells by antibodies as assayed by the aHFc-CL-MMAE toxin antibody system. FIG. 5B shows the killing of HER3 single-positive cells by antibodies as assayed by the aHFc-CL-MMAE toxin antibody system. FIG. 5C shows the killing of MET single-positive cells by antibodies as assayed by the αHFc-CL-MMAE toxin antibody system. FIG. 5D shows the killing of HER3/MET double-positive cells by antibodies as assayed by the αHFc-CL-MMAE toxin antibody system.
FIG. 6 shows the endocytosis of antibodies in HER3/MET double-positive cells as assayed by pHrodo.
FIG. 7 shows the endocytosis of antibodies in HER3/MET double-positive cells as assayed by FACS.
FIG. 8 shows ERK phosphorylation levels of antibodies in HER3/MET double-positive cells.
FIG. 9A shows a schematic diagram of a HER3/MET antibody-drug conjugate; FIG. 9B shows a denaturing mass spectrum of the HER3/MET antibody-drug conjugate; and FIG. 9C shows a native mass spectrum of the HER3/MET antibody-drug conjugate.
FIG. 10 shows the binding of HER3/MET bispecific antibodies and ADCs thereof to HER3/MET double-positive cells as assayed by FACS.
FIG. 11A shows the HER3/MET expression results of HER3/MET double-positive cells as assayed by FACS. FIG. 11B shows the killing results of ADC-4 in HER3/MET double-positive cells. FIG. 11C shows the killing results of ADC-1 in HER3/MET double-positive cells.
FIGs. 12A and 12B show the anti-tumor activity results of the bispecific antibody ADCs in the HCC827 Osimertinib-resistant cell xenograft model, where FIG. 12A shows the change in body weight of mice, and FIG. 12B shows the change in tumor volume of mice.
FIGs. 13A and 13B show the anti-tumor activity results of the bispecific antibody ADCs in the HER3 single-positive cell xenograft model, where FIG. 13A shows the change in body weight of mice, and FIG. 13B shows the change in tumor volume of mice.
FIG. 14 shows the killing activity of the bispecific antibody ADCs in HER3/MET double-positive cells.

## DETAILED DESCRIPTION

Definitions of Terms

In order to facilitate the understanding of the present disclosure, certain technical and scientific terms are specifically defined below. Unless otherwise specifically defined in the present disclosure, all other technical and scientific terms used in the present disclosure have the meanings generally understood by those of ordinary skill in the art to which the present disclosure pertains.

The three-letter and single-letter codes for amino acids used in the present disclosure are as described in J. biol. chem., 243, p3558 (1968).

"MET", "cMET", "MET protein", or "MET polypeptide" may optionally include any such protein or a variant, a conjugate, or a fragment thereof, including (but not limited to) known or wild-type MET described herein, as well as any naturally occurring splice variant, amino acid variant, or isoform. The complete MET sequence can be under the UniProt accession number: P08581.

"HER3", "HER3 protein", or "HER3 polypeptide" may optionally include any such protein or a variant, a conjugate, or a fragment thereof, including (but not limited to) known or wild-type HER3 described herein, as well as any naturally occurring splice variant, amino acid variant, or isoform. The complete HER3 sequence can be under the UniProt accession number: P21860.

The term "functional variant" includes, but is not limited to, homologs, fragments, truncations, mutants,

modifications, etc. of wild-type proteins. A functional variant of a protein has increased, decreased, or maintained protein activity compared to the wild-type protein.

"Binding protein" or "binding molecule" of the present disclosure encompasses any protein or polypeptide capable of specifically binding to an antigen (e.g., MET or HER3) or a fragment or epitope thereof, or any molecule comprising the protein or polypeptide, including but not limited to antibodies as defined in the present disclosure.

In the present disclosure, "polypeptide", "peptide", or "protein" is used interchangeably and refers to a polymer of amino acid residues, or a collection of polymers of multiple amino acid residues. These terms are used to describe amino acid polymers in which one or more amino acid residues are artificial chemical mimics of corresponding naturally occurring amino acids, as well as naturally occurring amino acid polymers and non-naturally occurring amino acid polymers. A polypeptide sequence is generally described as having an amino terminus (N-terminus) at the left end and having a carboxyl terminus (C-terminus) at the right end.

"Titin-T chain" or "T chain" refers to a peptide fragment of 78-118 amino acids in length comprising a Titin Ig-like 152 domain in a Titin protein or a functional variant thereof, wherein the Titin-T chain is capable of binding to the Obscurin Ig-like 1 domain to form a dimerized complex. The functional variant of the T chain is obtained through the mutation on a portion of amino acids of a wild-type T chain, but still has a polypeptide that binds to the Obscurin Ig-like 1 domain to form a dimerized complex. For example, amino acids of suitable length are added or truncated at the C-terminus and/or the N-terminus of the Titin Ig-like 152 domain; 1, 2, 3, 4, 5, 6, 7, 8, 9, or 10 amino acid residues may be added or truncated; for example, 5 amino acids "KAGIR" in wild-type Titin proteins which are immediately adjacent to the N-terminus of the Titin Ig-like 152 domain are added to the N-terminus of the Titin Ig-like 152 domain, which still has the function of binding to the Obscurin Ig-like 1 domain to form a complex. Other mutations can also be made on amino acids of the Titin Ig-like 152 domain, for example, certain amino acids may be mutated to increase inter-chain disulfide bonds, improve complex stability, and the like.

"Obscurin-O chain" or "O chain" refers to a peptide fragment of 87-117 amino acids in length comprising an Obscurin Ig-like 1 domain in an Obscurin protein or a functional variant thereof, which is capable of binding to the Titin Ig-like 152 domain to form a dimerized complex. The functional variant of the Obscurin-O chain is obtained through the mutation on a portion of amino acids of a wild-type O chain, but still has a polypeptide that binds to the Titin Ig-like 152 domain to form a dimerized complex. For example, amino acids of suitable length are added or truncated at the C-terminus and/or the N-terminus of the Obscurin-O domain, for example, 1, 2, 3, 4, 5, 6, 7, 8, 9, or 10 amino acids are added or truncated; for example, 5 amino acids "DQPQF" in 5 wild-type Obscurin proteins which are immediately adjacent to the N-terminus of the Obscurin Ig-like 1 domain are added to the N-terminus of the Obscurin-O domain, which still has the function of binding to the Titin Ig-like 152 domain to form a dimerized complex. Other mutations can also be made on a portion of amino acids of the Obscurin Ig-like 1 domain, for example, certain amino acids may be mutated to increase inter-chain disulfide bonds, improve antibody stability, and the like.

"Nucleic acid" or "polynucleotide" are used interchangeably herein to refer to any DNA or RNA molecule, either single- or double-stranded, and, if single-stranded, the molecule of its complementary sequence, preferably a double-stranded DNA.

"Antibody" encompasses a variety of antibody structures, including but not limited to monoclonal antibodies, polyclonal antibodies, monospecific antibodies, bispecific or multispecific antibodies (e.g., trispecific antibodies), full-length antibodies, and antibody fragments (or antigen-binding fragments, or antigen-binding domains), as long as they exhibit the desired antigen-binding activity. The antibodies of the present disclosure include recombinant antibody forms.

An antibody may refer to an immunoglobulin that is a four-peptide-chain structure formed by linking two identical heavy chains and two identical light chains by interchain disulfide bonds. The heavy chain constant regions of immunoglobulins differ in amino acid composition and arrangement; therefore, they also differ in antigenicity. Accordingly, immunoglobulins can be divided into five classes, or isotypes of immunoglobulins, namely IgM, IgD, IgG, IgA, and IgE, and their corresponding heavy chains are μ chains, δ chains, γ chains, α chains, and ε chains, respectively. Igs of the same class can be divided into different subclasses based on the amino acid composition of their hinge regions and the number and positions of heavy chain disulfide bonds; for example, IgGs can be divided into IgG1, IgG2, IgG3, and IgG4. Light chains are divided into κ or λ chains based on their constant regions. Each of the five Ig classes may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (HVRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus as follows: FR1, CDR1, FR2, CDR2, FR3, CDR3, and FR4. The 3 CDRs of the light chain refer to LCDR1, LCDR2, and LCDR3, and the 3 CDRs of the heavy chain refer to HCDR1, HCDR2, and HCDR3.

Bispecific antibodies of various structures have been disclosed in the prior art; the bispecific antibodies can be

classified into IgG-like bispecific antibodies and antibody-fragment-type bispecific antibodies according to the integrity of IgG molecules; the bispecific antibodies can be classified into bivalent, trivalent, tetravalent, or higher-valent bispecific antibodies according to the number of the antigen-binding regions; the bispecific antibodies can be classified into symmetric bispecific antibodies and asymmetric bispecific antibodies according to the presence of symmetry in their structures. Among them, the bispecific antibodies based on antibody fragments, such as Fab fragments lacking Fc fragments, formed by binding 2 or more Fab fragments in one molecule, have low immunogenicity, small molecular weight and high tumor tissue permeability, and typical antibody structures of this type comprise bispecific antibodies, such as F(ab)2, scFv-Fab and (scFv)2-Fab; IgG-like bispecific antibodies (e.g., having Fc fragments) have relatively large molecular weight, and the Fc fragments facilitate later purification of the antibody and increase their solubility and stability, and the Fc fragments may further bind to the receptor FcRn and increase the serum half-life of the antibody, and typical structural models of bispecific antibodies comprise bispecific antibodies such as KiH, CrossMAb, Triomab quadroma, Fc∆Adp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP- DART, CODV-Fab-TL, HLE-BiTE, F(ab)2-CrossMAb, IgG-(scFv)2, Bs4Ab, DVD-Ig, Tetravalent-DART-Fc, (scFv)4-Fc, CODV-Ig, mAb2, and F(ab)4-CrossMAb (see Aran F. Labrijn et al., Nature Reviews Drug Discovery, volume 18, pages 585-608 (2019); Chen S1 et al., J Immunol Res., Feb. 11, 2019; 2019:4516041).

"Antigen-binding fragment" or "antigen-binding domain" encompasses a Fab, a modified Fab, a Fab', a modified Fab', a F(ab')2, an Fv, a Fab-Fv, a Fab-dsFv, a single-domain antibody (e.g., a VH or VL or VHH), an scFv, a bivalent or trivalent or tetravalent antibody, a Bis-scFv, a diabody, a tribody, a triabody, a tetrabody, and an epitope-binding fragment of any one of the above (see, e.g., Holliger and Hudson, 2005, Nature Biotech. 23(9): 1126-1136; Adair and Lawson, 2005, Drug Design Reviews-Online 2(3), 209-217). Methods for producing and preparing such antigen-binding fragments are well known in the art (see, e.g., Verma et al., 1998, Journal of Immunological Methods, 216, 165-181).

For the determination or definition of CDRs, the deterministic depiction of CDRs and identification of residues comprising binding sites of an antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition. The Kabat numbering scheme is a standard for numbering residues in antibodies and is generally used to identify CDRs (see, e.g., Johnson & Wu, 2000, Nucleic Acids Res., 28: 214-8). The Chothia numbering scheme is similar to the Kabat numbering scheme, except that it takes into account the positions of certain structural loop regions (see, e.g., Chothia et al., 1986, J. Mol. Biol., 196: 901-17; Chothia et al., 1989, Nature, 342: 877-83). The AbM numbering scheme adopts a computer program integration suite for modeling antibody structures manufactured by Oxford Molecular Group (see, e.g., Martin et al., 1989, Proc Natl Acad Sci (USA), 86: 9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3: 194-198). The contact definition is based on the analysis of the available complex crystal structures (see, e.g., MacCallum et al., 1996, J. Mol. Biol., 5: 732-45). In the conformational definition, the positions of the CDRs can be identified as residues that contribute enthalpy to the antigen binding (see, e.g., Makabe et al., 2008, Journal of Biological Chemistry, 283: 1156-1166). In addition, other CDR boundary definitions may not strictly follow one of the methods described above, but still overlap with at least a portion of the Kabat CDRs, although they may be shortened or lengthened based on predictions or experimental results that a particular residue or a particular group of residues does not significantly affect the antigen binding. As used in the present disclosure, a CDR may refer to a CDR defined by any method known in the art, including combinations of methods. The correspondence between the various numbering schemes is well known to those skilled in the art, and examples are shown in Table 1 below.

Table 1. The relationships between CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |

(continued)

| CDR | IMGT | Kabat | AbM | Chothia | Contact |
|---|---|---|---|---|---|
| LCDR3 | 105-117 | 89-97 | 89-97 | 89-97 | 89-96 |

The CDR amino acid residues of the VL and VH regions of the antibody of the present disclosure accord with the known Kabat numbering scheme in terms of number and positions.

"Binding affinity" or "affinity" is used in the present disclosure as a measure of the strength of a non-covalent interaction between two molecules (e.g., an antibody or a portion thereof and an antigen). The binding affinity between two molecules can be quantified by determining the dissociation equilibrium constant ($K_D$). $K_D$ can be determined by measuring the kinetics of complex formation and dissociation by using, e.g., the surface plasmon resonance (SPR) method (Biacore). The rate constants corresponding to the association and dissociation of a complex are referred to as the association rate constant ka (or kon) and the dissociation rate constant kd (or koff), respectively. $K_D$ is related to ka and kd by the equation $K_D$ = kd/ka. The value of the dissociation constant can be determined directly by well-known methods, and calculations can even be performed for complex mixtures by, for example, those methods described in Caceci et al (1984, Byte 9:340-362). For example, $K_D$ can be determined by using a dual filtration nitrocellulose filter binding assay such as that disclosed by Wong & Lohman (1993, Proc. Natl. Acad. Sci. USA 90: 5428-5432). Other standard assays for evaluating the binding ability of an antibody to a target antigen are known in the art and include, for example, ELISA, western blot, RIA, and FACS, as well as other assays exemplified elsewhere in the present disclosure. The binding kinetics and binding affinity of the antibody can also be evaluated by standard assays known in the art, such as surface plasmon resonance (SPR), e.g., by using the Biacore™ system or KinExA. The binding affinities associated with different molecular interactions, e.g., the binding affinities of different antibodies for a given antigen, can be compared by comparing the $K_D$ values of antibody/antigen complexes. Similarly, the specificity of an interaction can be evaluated by determining and comparing the $K_D$ value for the interaction of interest (e.g., a specific interaction between an antibody and an antigen) with the $K_D$ value for an interaction not of interest.

Generally, "specifically binding" refers to the binding of a binding molecule (binding protein) to an epitope on an antigen. The HER3/MET-binding molecule of the present disclosure will bind to an antigen (i.e., HER3 or MET) or an epitope thereof to be bound with a dissociation equilibrium constant ($K_D$) of $\leq 10^{-7}$ M, preferably $\leq 10^{-8}$ M, as measured in a Biacore or KinExA or Fortibio assay. Any $K_D$ value greater than $10^{-4}$ M is generally considered to indicate non-specific binding. The specific binding of a binding molecule to an antigen or epitope can be determined in any known suitable manner, including, for example, the surface plasmon resonance (SPR), enzyme-linked immunosorbent assay (ELISA), and/or flow cytometry sorting (FACS) described in the present disclosure.

"Conservative replacement" refers to replacement by another amino acid residue having similar properties to the original amino acid residue. For example, lysine, arginine, and histidine have similar properties in that they have basic side chains, and aspartic acid and glutamic acid have similar properties in that they have acidic side chains. In addition, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is replaced, it will not exhibit a particular change in properties.

"Homology", "identity", or "sequence identity" refers to sequence similarity between two nucleic acid sequences or between two polypeptides. When positions in two compared sequences are occupied by identical nucleotides or amino acid monomers, e.g., if the position of each of two DNA molecules is occupied by an identical nucleotide, the molecules are homologous at that position. The percent homology between two sequences is a function of the number of matching or homologous positions shared by the two sequences divided by the number of compared positions $\times$ 100%. For example, if 6 out of 10 positions are matched or homologous when two sequences are optimally aligned, the two sequences are 60% homologous. In general, when two sequences are aligned, a comparison is performed to obtain the maximum percent homology.

The term "internalization" refers to the transport of a moiety from the outside of a cell to the inside. The internalized moiety can be located in an intracellular compartment. An "internalized" antigen or antibody refers to an antigen or antibody that is capable of being transported from the outside of a target cell to the inside. Those skilled in the art will readily appreciate that the process of cellular internalization generally refers to the movement of a cell-surface molecule across the plasma membrane from the cell surface to the inside of the cell. After internalization, endosomes can be trafficked to lysosomes for degradation or recycled to the cell surface. The cellular internalization rate of a given cell-surface molecule provides a measurement of the kinetics of the movement of the molecule from the cell surface to the inside of the cell through the plasma membrane. The internalization activity or rates of antigens and antibodies can be monitored and/or

measured by a number of techniques known in the art, including acid dissociation (Li N. et al., Methods Mol. Biol., 457:305-17, 2008) and toxin-killing assays (Pahara J. et al., Exp Cell Res., 316:2237-50, 2010; and Mazor et al., J. Immunol. Methods, 321:41-59, 2007). Many antibody labeling techniques, dyes, and kits for antibody labeling that can be used to quantify and monitor internalization are commercially available (e.g., commercially available pHrodo iFL antibody labeling methods, reagents, and kits from Thermo Fisher Scientific).

The term "antibody-drug conjugate" (ADC) refers to the linking of an antibody to a biologically active drug. The antibody may be coupled to the drug directly or via a linker unit.

The term "drug loading" refers to the average number of cytotoxic drugs loaded onto each ligand in an ADC, and it may also be expressed as the ratio of the number of drugs to the number of antibodies. The drug loading range may be 1-20, preferably 1-10, cytotoxic drugs (D) linked to each antibody (Ab). In the embodiments of the present disclosure, the drug loading is represented by n; examples are 1, 2, 3, 4, 5, 6, 7, 8, 9, 10, 11, 12, 13, 14, 15, 16, 17, 18, 19, 20, or an average of any two values. The average number of drugs per ADC molecule after a coupling reaction can be characterized by conventional methods such as UV/visible spectroscopy, mass spectrometry, an ELISA assay, a monoclonal antibody molecule size variant assay (CE-SDS), and HPLC.

Although the drug-to-antibody ratio has an exact value (e.g., n in formula (I)) for a specific conjugate molecule, it will be understood that when used to describe a sample containing many molecules, the value will often be a mean value, which is attributed to a certain degree of non-uniformity typically associated with the conjugation step. The mean loading of an immunoconjugate sample is referred to herein as the drug-to-antibody ratio or "DAR". In some examples, the DAR is between about 1 and about 10, e.g., 1-8, and is typically about 1, 1.5, 2, 2.5, 3, 3.5, 4, 4.5, 5, 5.5, 6, 6.5, 7.0, 7.5, or 8.0. Examples include immunoconjugates in which the DAR is about 1.1, about 1.2, about 1.3, about 1.4, about 1.5, about 1.6, about 1.7, about 1.8, about 1.9, about 2.0, about 2.1, about 2.2, about 2.3, about 2.4, about 2.5, about 2.6, about 2.7, about 2.8, about 2.9, about 3.0, about 3.1, about 3.2, about 3.3, about 3.4, about 3.5, about 3.6, about 3.7, about 3.8, about 3.9, about 4.0, about 4.1, about 4.2, about 4.3, about 4.4, about 4.5, about 4.6, about 4.7, about 4.8, about 4.9, about 5.0, about 5.1, about 5.2, about 5.4, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, or about 7.0. In some examples, a DAR of 'about x' means that the DAR measurement is within 20% of x.

The method of determining DAR is, for example, extrapolating a DAR value from LC-MS data of reduced and deglycosylated samples. LC/MS allows for quantification of the average number of payload (drug moiety) molecules linked to the antibody in the ADC. HPLC separates the antibody into light and heavy chains, and also separates the heavy (HC) and light (LC) chains according to the number of linker-payload groups in each chain. Mass spectrometry data enables identification of the types of components in a mixture, e.g., LC, LC+1, LC+2, HC, HC+1, and HC+2. From the mean loading of the LC and HC chains, the mean DAR of an ADC can be calculated. The DAR of a given immunoconjugate sample represents the average number of drug (payload) molecules linked to a tetrameric antibody containing two light chains and two heavy chains. An example is the DAR determination method described in WO2018142322.

The term "camptothecin drug" refers to camptothecin, which is cytotoxic, and derivatives thereof, which are selected from the group consisting of, without limitation, 10-hydroxycamptothecin, 7-ethyl-10-hydroxycamptothecin, topotecan, exatecan, irinotecan, or 9-nitro-10-hydroxycamptothecin, and derivatives or pharmaceutically acceptable salts thereof.

The term "alkyl" refers to a saturated aliphatic hydrocarbon group that is a linear or branched group containing 1 to 20 carbon atoms, preferably alkyl containing 1 to 12 carbon atoms, more preferably alkyl containing 1 to 10 carbon atoms, and most preferably alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. Lower alkyl groups having 1 to 6 carbon atoms are more preferred, and non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, etc. Alkyl may be substituted or unsubstituted, and when it is substituted, the substituent may be substituted at any accessible point of attachment, and the substituent is preferably one or more of the following groups, independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, and oxo.

The term "heteroalkyl" refers to an alkyl group containing one or more heteroatoms selected from the group

consisting of N, O, and S, wherein the alkyl is as defined above. The term "alkylene" refers to a saturated linear or branched aliphatic hydrocarbon group having 2 residues derived from the parent alkane by removal of two hydrogen atoms from the same carbon atom or two different carbon atoms, which is a linear or branched group containing 1 to 20 carbon atoms, preferably alkylene containing 1 to 12 carbon atoms, and more preferably alkylene containing 1 to 6 carbon atoms. Non-limiting examples of alkylene include, but are not limited to, methylene($-CH_2-$), 1,1-ethylidene($-CH(CH_3)-$), 1,2-ethylidene($-CH_2CH_2)-$, 1,1-propylidene($-CH(CH_2CH_3)-$), 1,2-propylidene($-CH_2CH(CH_3)-$), 1,3-propylidene($-CH_2CH_2CH_2-$), 1,4-butylidene($-CH_2CH_2CH_2CH_2-$), 1,5-butylidene($-CH_2CH_2CH_2CH_2CH_2-$), and the like. Alkylene may be substituted or unsubstituted, and when it is substituted, the substituent may be substituted at any accessible point of attachment, and the substituent is preferably substituted with one or more substituents independently and optionally selected from the group consisting of alkyl, alkenyl, alkynyl, alkoxy, alkylthio, alkylamino, halogen, sulfhydryl, hydroxy, nitro, cyano, cycloalkyl, heterocyclyl, aryl, heteroaryl, cycloalkoxy, heterocycloalkoxy, cycloalkylthio, heterocycloalkylthio, and oxo.

The term "alkoxy" refers to -O-(alkyl) and -O-(unsubstituted cycloalkyl), wherein the alkyl or cycloalkyl is as defined above. Non-limiting examples of alkoxy groups include methoxy, ethoxy, propoxy, butoxy, cyclopropyloxy, cyclobutoxy, cyclopentyloxy, and cyclohexyloxy. Alkoxy may be optionally substituted or unsubstituted, and when it is substituted, the substituent is preferably one or more of the following groups, independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "cycloalkyl" refers to a saturated or partially unsaturated monocyclic or polycyclic hydrocarbon substituent; the cycloalkyl ring contains 3 to 20 carbon atoms, preferably 3 to 12 carbon atoms, more preferably 3 to 10 carbon atoms, and most preferably 3 to 8 carbon atoms. Non-limiting examples of monocyclic cycloalkyl groups include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc. Polycyclic cycloalkyl groups include spiro-ring, fused-ring, and bridged-ring cycloalkyl groups.

The term "heterocyclyl" refers to a saturated or partially unsaturated monocyclic or polycyclic hydrocarbon substituent containing 3 to 20 ring atoms, wherein one or more of the ring atoms are heteroatoms selected from the group consisting of nitrogen, oxygen, and $S(O)_m$ (where m is an integer from 0 to 2), but a ring moiety of -O-O-, -O-S-, or -S-S- is excluded, and the other ring atoms are carbon atoms. It preferably contains 3 to 12 ring atoms, of which 1 to 4 are heteroatoms; more preferably, the cycloalkyl ring contains 3 to 10 ring atoms. Non-limiting examples of monocyclic heterocyclyl groups include pyrrolidinyl, piperidinyl, piperazinyl, morpholinyl, thiomorpholinyl, homopiperazinyl, etc. Polycyclic heterocyclyl groups include spiro-ring, fused-ring, and bridged-ring heterocyclyl groups.

The term "spiroheterocyclyl" refers to a 5- to 20-membered polycyclic heterocyclyl group in which one atom (referred to as a spiro atom) is shared between monocyclic rings, wherein one or more ring atoms are heteroatoms selected from the group consisting of nitrogen, oxygen, and $S(O)_m$ (where m is an integer from 0 to 2), and the other ring atoms are carbon atoms. It may contain one or more double bonds, but no ring has a fully conjugated π-electron system. It is preferably 6- to 14-membered, and is more preferably 7- to 10-membered. According to the number of spiro atoms shared between rings, spiroheterocyclyl may be monospiroheterocyclyl, bispiroheterocyclyl, or polyspiroheterocyclyl, preferably, monospiroheterocyclyl and bispiroheterocyclyl, and more preferably 4-membered/4-membered, 4-membered/5-membered, 4-membered/6-membered, 5-membered/5-membered, or 5-membered/6-membered monospiroheterocyclyl. Non-limiting examples of spiroheterocyclyl groups include:

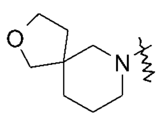 , 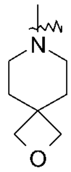 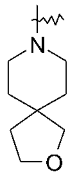 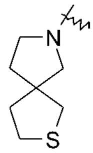 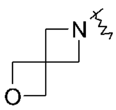 and 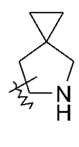 .

The term "fused heterocyclyl" refers to a 5- to 20-membered polycyclic heterocyclyl group in which each ring in the system shares a pair of adjacent atoms with the other rings in the system, wherein one or more rings may contain one or more double bonds, but none of the rings has a fully conjugated π-electron system; one or more ring atoms are heteroatoms selected from the group consisting of nitrogen, oxygen, and $S(O)_m$ (where m is an integer from 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic fused heterocyclyl, preferably bicyclic or tricyclic, and more preferably 5-membered/5-membered or 5-membered/6-membered bicyclic fused heterocyclyl. Non-limiting examples of fused heterocyclyl groups include:

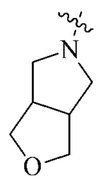, 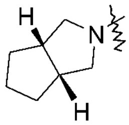, 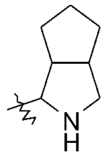, 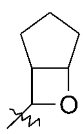, 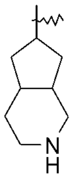, 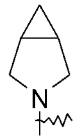, 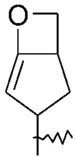, 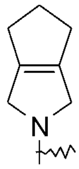,

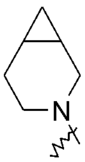, 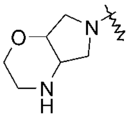, 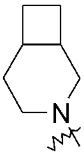, 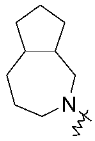, 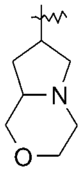, 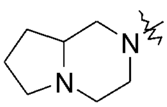, and 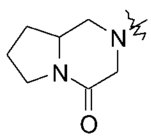.

The term "bridged heterocyclyl" refers to a 5- to 14-membered polycyclic heterocyclyl group in which any two rings share two atoms that are not directly connected; it may contain one or more double bonds, but none of the rings has a fully conjugated π-electron system, wherein one or more ring atoms are heteroatoms selected from the group consisting of nitrogen, oxygen, and $S(O)_m$ (where m is an integer from 0 to 2), and the other ring atoms are carbon atoms. It is preferably 6- to 14-membered, and is more preferably 7- to 10-membered. According to the number of constituent rings, it may be bicyclic, tricyclic, tetracyclic, or polycyclic bridged heterocyclyl, preferably bicyclic, tricyclic, or tetracyclic, and more preferably bicyclic or tricyclic. Non-limiting examples of bridged heterocyclyl groups include:

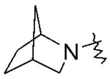 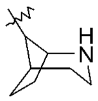 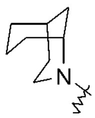 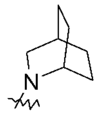

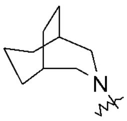 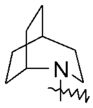 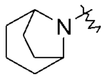, and 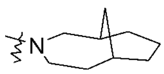.

The heterocyclyl ring may be fused to an aryl, heteroaryl, or cycloalkyl ring, wherein the ring attached to the parent structure is heterocyclyl; its non-limiting examples include:

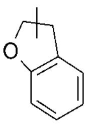, 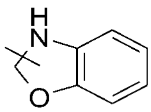, 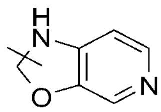, 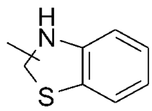,

etc.

Heterocyclyl may be optionally substituted or unsubstituted, and when it is substituted, the substituent is preferably one or more of the following groups, independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, heterocycloalkylthio, and oxo.

The term "aryl" refers to a 6- to 14-membered, preferably 6- to 10-membered, all-carbon monocyclic or fused polycyclic (i.e., rings sharing a pair of adjacent carbon atoms) group having a conjugated π-electron system, such as phenyl and naphthyl, preferably phenyl. The aryl ring may be fused to a heteroaryl, heterocyclyl, or cycloalkyl ring, wherein the ring attached to the parent structure is the aryl ring; its non-limiting examples include:

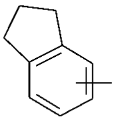, 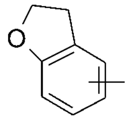, 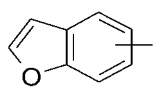, 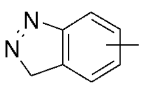, 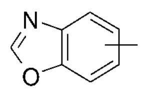, 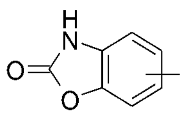,

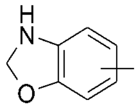, 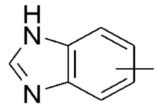, 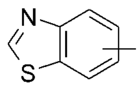, 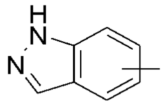, 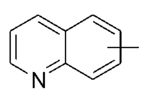, 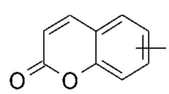,

and

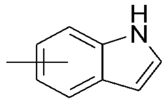.

Aryl may be substituted or unsubstituted, and when it is substituted, the substituent is preferably one or more of the following groups, independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "heteroaryl" refers to a heteroaromatic system containing 1 to 4 heteroatoms and 5 to 14 ring atoms, wherein the heteroatoms are selected from the group consisting of oxygen, sulfur, and nitrogen. Heteroaryl is preferably 5- to 10-membered, and is more preferably 5- or 6-membered, e.g., furanyl, thienyl, pyridyl, pyrrolyl, N-alkylpyrrolyl, pyrimidinyl, pyrazinyl, imidazolyl, and tetrazolyl. The heteroaryl ring may be fused to an aryl, heterocyclyl, or cycloalkyl ring, wherein the ring attached to the parent structure is the heteroaryl ring; its non-limiting examples include:

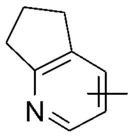, 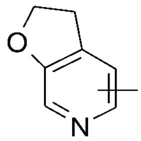, 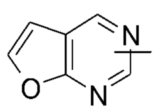, 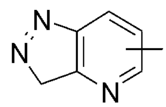, 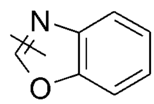, 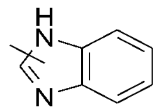,

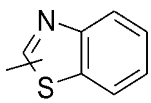, 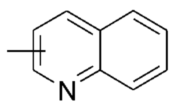, 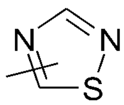, and 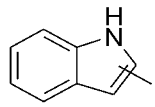.

Heteroaryl may be optionally substituted or unsubstituted, and when it is substituted, the substituent is preferably one or more of the following groups, independently selected from the group consisting of alkyl, alkenyl, alkynyl, alkoxy, alkylthio, alkylamino, halogen, sulfhydryl, hydroxy, nitro, cyano, cycloalkyl, heterocycloalkyl, aryl, heteroaryl, cycloalkoxy, heterocycloalkoxy, cycloalkylthio, and heterocycloalkylthio.

The term "amino protecting group" refers to an easily removable group for protecting an amino group from being changed when a reaction is taking place elsewhere in the molecule. Non-limiting examples include 9-fluorenylmethoxycarbonyl, tert-butoxycarbonyl, acetyl, benzyl, allyl, p-methoxybenzyl, etc. These groups may be optionally substituted with 1-3 substituents selected from the group consisting of halogen, alkoxy, and nitro. The amino protecting group is preferably 9-fluorenylmethoxycarbonyl.

The term "cycloalkylalkyl" refers to alkyl substituted with one or more cycloalkyl groups, preferably one cycloalkyl group, wherein the alkyl is as defined above, and the cycloalkyl is as defined above.

The term "haloalkyl" refers to an alkyl group substituted with one or more halogens, wherein the alkyl is as defined above.

The term "deuterated alkyl" refers to an alkyl group substituted with one or more deuterium atoms, wherein the alkyl is as defined above.

The term "hydroxy" refers to the -OH group.

The term "halogen" refers to fluorine, chlorine, bromine, or iodine.

The term "amino" refers to $-NH_2$.

The term "nitro" refers to $-NO_2$.

The term "acylamino" refers to -C(O)N(alkyl) or -C(O)N(cycloalkyl), wherein the alkyl and cycloalkyl are as defined above.

The term "carboxylate group" refers to -C(O)O(alkyl) or -C(O)O(cycloalkyl), wherein the alkyl and cycloalkyl are as defined above.

The present disclosure also includes various deuterated forms of the compound of formula (I). Each available hydrogen atom linked to a carbon atom may be independently replaced with a deuterium atom. Those skilled in the art are

able to synthesize the deuterated forms of the compound of formula (I) with reference to the relevant literature. Commercially available deuterated starting materials can be used in preparing the deuterated forms of the compound of formula (I), or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, etc.

"Substituted" means that one or more, preferably up to 5, and more preferably 1 to 3, hydrogen atoms in the group are independently substituted with a corresponding number of substituents. It goes without saying that a substituent is only in its possible chemical positions, and those skilled in the art will be able to determine (experimentally or theoretically) possible or impossible substitutions without undue effort. For example, the binding of an amino or hydroxy group having free hydrogen to a carbon atom having an unsaturated (e.g., olefinic) bond may be unstable.

The term "pharmaceutically acceptable auxiliary material" or "pharmaceutically acceptable excipient" includes any material that, when combined with an active ingredient, allows the ingredient to retain biological activity and is non-reactive with the immune system of a subject. Examples include, but are not limited to, any standard pharmaceutical carrier, such as buffered saline solution, water, emulsions such as oil/water emulsions, and various types of wetting agents.

"Inhibition" and "blocking" are used interchangeably and encompass both partial and complete inhibition/blocking. "Inhibition of growth" (e.g., involving cells) is intended to include any measurable reduction in cell growth.

"Proliferative disease" refers to a disorder associated with a certain degree of abnormal cell proliferation. In one embodiment, the proliferative disorder is a cancer. "Tumor" refers to all neoplastic cell growth and proliferation, whether malignant or benign, and all pre-cancerous and cancerous cells and tissues. "Cancer", "cancerous", "proliferative disorder", and "tumor" are not mutually exclusive when referred to in the present disclosure.

"Giving", "administering", and "treating", when applied to animals, humans, experimental subjects, cells, tissues, organs, or biological fluids, refer to contact of an exogenous drug, a therapeutic agent, a diagnostic agent, or a composition with the animals, humans, subjects, cells, tissues, organs, or biological fluids, e.g., therapeutic, pharmacokinetic, diagnostic, research, and experimental methods. The treatment of cells comprises contacting a reagent with cells and contacting the reagent with a fluid, wherein the fluid is in contact with the cells. "Giving", "administering", and "treating" also mean treating, e.g., a cell, by a reagent, diagnosis, a binding composition, or by another cell *in vitro* and *ex vivo.* When applied to humans, veterinary medicine, or research subjects, they refer to therapeutic treatment, preventive or prophylactic measures, and research and diagnostic applications.

"Treating" or "treatment" refers to administering a therapeutic agent, such as a therapeutic agent comprising any one of the binding proteins of the present disclosure or a pharmaceutical composition thereof, either internally or externally, to a subject who has had, is suspected of having, or is predisposed to having one or more proliferative diseases or symptoms thereof on which the therapeutic agent is known to have a therapeutic effect. Typically, the therapeutic agent is administered in an amount effective in alleviating one or more disease symptoms in the subject or population being treated, whether by inducing regression of such symptoms or inhibiting the development of such symptoms into any clinically measurable degree. The amount of therapeutic agent effective in alleviating any specific disease symptom (also referred to as the "therapeutically effective amount") may vary depending on a variety of factors such as the disease state, age, and weight of the subject, and the capability of the drug to produce the desired therapeutic effect in the subject. Whether a disease symptom has been palliated can be evaluated by any clinical testing methods commonly used by doctors or other health care professionals to evaluate the severity or progression of the symptom. Although embodiments of the present disclosure (e.g., treatment methods or products) may be ineffective in alleviating a disease symptom of interest in a certain subject, they shall alleviate the disease symptom of interest in a statistically significant number of subjects as determined by any statistical test method known in the art, such as the Student's t-test, Chi-square test, U-test by Mann and Whitney, Kruskal-Wallis test (H-test), Jonckheere-Terpstra test, and Wilcoxon test.

"Effective amount" comprises an amount sufficient to ameliorate or prevent a symptom or sign of a medical disorder. An effective amount also means an amount sufficient to allow or facilitate diagnosis. The effective amount for a subject may vary depending on factors such as the disorder to be treated, the general health of the subject, the method, route, and dose of administration, and the severity of side effects. An effective amount may be the maximum dose or administration regimen to avoid significant side effects or toxic effects.

"Optional" or "optionally" means that the event or circumstance subsequently described may, but does not necessarily, occur. This description includes the instance where the event or circumstance occurs or does not occur. "And/or" should be taken as specific disclosure of each of the two specified features or components with or without the other. Thus, the term "and/or" as used in a phrase such as "A and/or B" in the present disclosure includes "A and B", "A or B", "A" (alone), and "B" (alone). Unless the context clearly requires otherwise, throughout the description and the claims, the words "comprise", "have", "include", etc., are to be understood in an inclusive sense as opposed to an exclusive or exhaustive sense, that is to say, in the sense of "including but not limited to".

The "subject" or "patient" in the present disclosure means a mammal, particularly a primate, and especially a human.

## Examples

The present disclosure is further described with reference to the following examples; however, these examples are not intended to limit the scope of the present disclosure. Experimental procedures without specific conditions indicated in the examples or test examples of the present disclosure are generally conducted under conventional conditions, or under conditions recommended by the manufacturers of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY. Reagents without specific sources indicated were commercially available conventional reagents.

### Example 1. HER3/MET Dual-Target IHC Staining Results of Tumor Samples in Patients with EGFR-TKI-Resistant Non-Small Cell Lung Cancer

Tumor samples in patients with EGFR-TKI-resistant non-small cell lung cancer were detected by a HER3/MET dual-target IHC staining method. FIG. 1A to FIG. 1C show that HER3/MET were expressed in tumor tissues of patients with EGFR-TKI-resistant non-small cell lung cancer, with a co-expression rate of 100%. This indicates that HER3/MET target expression and high co-expression rate are present in patients with EGFR-TKI-resistant non-small cell lung cancer, and HER3/MET bispecific antibody ADCs can achieve better anti-tumor activity than HER3 monoclonal antibody ADCs or MET monoclonal antibody ADCs in these patients.

### Example 2. Design and Preparation of Anti-HER3/MET Bispecific Antibodies

1. Design of anti-HER3/MET bispecific antibodies

The variable region at the HER3 terminus was selected from the group consisting of antibody A; and the variable region at the MET terminus was selected from the group consisting of antibody A13, antibody S3, antibody A9, antibody P8, antibody P3, and antibody 5D5. The heavy chain variable region (VH) and light chain variable region (VL) sequences of the antibodies are shown below:

> Anti-MET antibody A13 VH

QVQLVQSGAEVKKPGASVKVSCEASGYTFTSYGFSWVRQAPGQGLEWMGWIS
ASNGNTYYAQKLQGRVTMTTDTSTSSAYMELRSLRSDDTAVYYCARVYADYA
DYWGQGTLVTVSS (SEQ ID NO: 1)

> Anti-MET antibody A13 VL

DIQMTQSPSSVSASVGDRVTITCRASQGINTWLAWYQQKPGKAPKLLIYAASSL
KSGVPSRFSGSGSGADFTLTISSLQPEDFATYYCQQANSFPLTFGGGTKVEIK
(SEQ ID NO: 2)

> Anti-MET antibody S3 VH

QVQLVESGGGVVQPGRSLRLSCAASGFSLSNYGVHWVRQAPGKGLEWLAVIW
SGGSTNYAAAFVSRLTISKDNSKNTVYLQMNSLRAEDTAVYYCARNHDNPYN
YAMDYWGQGTTVTVSS (SEQ ID NO: 3)

> Anti-MET antibody S3 VL

DIVLTQSPDSLAVSLGERATINCRADKSVSTSTYNYLHWYQQKPGQPPKLLIYL
ASNLASGVPDRFSGSGSGTDFTLTISSLQAEDVAVYYCQHSRDLPPTFGQGTKL
EIK

(SEQ ID NO: 4)

> Anti-MET antibody A9 VH

QVQLQESGPGLVKPSQTLSLTCTVSGGSISSGGYYWSWIRQHPGKGLEWIGYIY
YSGSTYYNPSLKSRVTISVDTSKNQFSLKLSSVTAADTAVYYCARDGPLGYCSS
TSCPVTGEYYYYGMDVWGQGTTVTVSS (SEQ ID NO: 5)

> Anti-MET antibody A9 VL

EIVLTQSPGTLSLSPGERATLSCRASQSVSNNYLAWYQQKPGQAPRLLIFGASSR
ATGIPDRFSGSGSGTDFTLTISRLEPEDFAVYYCQQYDISPMYSFGQGTKLEMK

(SEQ ID NO: 6)

> Anti-MET antibody P8 VH

EVQLLESGGGLVQPGGSLRLSCAASGFTFSSYAMSWVRQAPGKGLEWVSAISG
SGGSTYYADSVKGRFTISRDNSKNTLYLQMNSLRAEDTAVYYCAKDHYYDSS
GYLDYWGQGTLVTVSS (SEQ ID NO: 7)

> Anti-MET antibody P8 VL

NFMLTQPHSVSESPGKTVTISCTRSSGSIAFDYVQWYQQRPGSAPTTVIYEDNQR
PSGVPDRFSASIDSSSNSASLTISALKTEDEADYYCQSYDNSNSWVFGGGTKLTV
L

(SEQ ID NO: 8)

> Anti-MET antibody P3 VH

QVQLQESGPGLVKPSATLSLTCAVSGGSISSNHWWSWVRQSPGKGLEWIGEIYT
YGGANYNPSLKSRVDISMDKSKNQFSLHLSSVTAADTAVYYCGRHLTGYDCFD
IWGQGTLVTVSS (SEQ ID NO: 9)

> Anti-MET antibody P3 VL

QAVLTQPSSVSGAPGQRVTISCTGSSSNIGAGYDVHWYQQLPGTAPKLLIYGNS
NRPSGVPDRFSGSKSGTSASLAITGLQAEDEADYYCQSYDSSLSGVFGTGTQLT
VL

(SEQ ID NO: 10)

> Anti-MET antibody 5D5 VH

EVQLVESGGGLVQPGGSLRLSCAASGYTFTSYWLHWVRQAPGKGLEWVGMID PSNSDTRFNPNFKDRFTISADTSKNTAYLQMNSLRAEDTAVYYCATYRSYVTPL DYWGQGTLVTVSS (SEQ ID NO: 11)

> Anti-MET antibody 5D5 VL

DIQMTQSPSSLSASVGDRVTITCKSSQSLLYTSSQKNYLAWYQQKPGKAPKLLI YWASTRESGVPSRFSGSGSGTDFTLTISSLQPEDFATYYCQQYYAYPWTFGQGT

KVEIK

(SEQ ID NO: 12)

> Anti-HER3 antibody A VH

QVQLVQSGGGLVQPGRSLRLSCAASGFTFDDYAMHWVRQAPGKGLEWVSGIS WNSGSIGYADSVKGRFTISRDNAKNSLYLQMNSLRAEDTALYYCAKEGLPGLD YWGQGTLVTVSS (SEQ ID NO: 13)

> Anti-HER3 antibody A VL

DIQMTQSPSSLSASIGDRATITCRASQHVGTYLNWYQQKPGKTPKLLISGAANL QSGVPSRFSGSGSGTDFTLTISSLQPEDFATYYCQQSYNTPPFSFGQGTKVEIK (SEQ ID NO: 14)

Table 2. CDR sequences of antibodies (Kabat numbering scheme)

| Antibody name | CDR | | Sequence No. |
|---|---|---|---|
| Anti-MET antibody A13 | HCDR1 | SYGFS | SEQ ID NO:15 |
| | HCDR2 | WISASNGNTYYAQKLQG | SEQ ID NO:16 |
| | HCDR3 | VYADYADY | SEQ ID NO:17 |
| | LCDR1 | RASQGINTWLA | SEQ ID NO:18 |
| | LCDR2 | AASSLKS | SEQ ID NO:19 |
| | LCDR3 | QQANSFPLT | SEQ ID NO:20 |
| Anti-MET antibody S3 | HCDR1 | NYGVH | SEQ ID NO:21 |
| | HCDR2 | VIWSGGSTNYAAAFVS | SEQ ID NO:22 |
| | HCDR3 | NHDNPYNYAMDY | SEQ ID NO:23 |
| | LCDR1 | RADKSVSTSTYNYLH | SEQ ID NO:24 |
| | LCDR2 | LASNLAS | SEQ ID NO:25 |
| | LCDR3 | QHSRDLPPT | SEQ ID NO:26 |

(continued)

| Antibody name | CDR | | Sequence No. |
|---|---|---|---|
| Anti-MET antibody A9 | HCDR1 | SGGYYWS | SEQ ID NO:27 |
| | HCDR2 | YIYYSGSTYYNPSLKS | SEQ ID NO:28 |
| | HCDR3 | DGPLGYCSSTSCPVTGEYYYYGMDV | SEQ ID NO:29 |
| | LCDR1 | RASQSVSNNYLA | SEQ ID NO:30 |
| | LCDR2 | GASSRAT | SEQ ID NO:31 |
| | LCDR3 | QQYDISPMYS | SEQ ID NO:32 |
| Anti-MET antibody P8 | HCDR1 | SYAMS | SEQ ID NO:33 |
| | HCDR2 | AISGSGGSTYYADSVKG | SEQ ID NO:34 |
| | HCDR3 | DHYYDSSGYLDY | SEQ ID NO:35 |
| | LCDR1 | TRSSGSIAFDYVQ | SEQ ID NO:36 |
| | LCDR2 | EDNQRPS | SEQ ID NO:37 |
| | LCDR3 | QSYDNSNSWV | SEQ ID NO:38 |
| Anti-MET antibody P3 | HCDR1 | SNHWWS | SEQ ID NO:39 |
| | HCDR2 | EIYTYGGANYNPSLKS | SEQ ID NO:40 |
| | HCDR3 | HLTGYDCFDI | SEQ ID NO:41 |
| | LCDR1 | TGSSSNIGAGYDVH | SEQ ID NO:42 |
| | LCDR2 | GNSNRPS | SEQ ID NO:43 |
| | LCDR3 | QSYDSSLSGV | SEQ ID NO:44 |
| Anti-MET antibody 5D5 | HCDR1 | SYWLH | SEQ ID NO:45 |
| | HCDR2 | MIDPSNSDTRFNPNFKD | SEQ ID NO:46 |
| | HCDR3 | YRSYVTPLDY | SEQ ID NO:47 |
| | LCDR1 | KSSQSLLYTSSQKNYLA | SEQ ID NO:48 |
| | LCDR2 | WASTRES | SEQ ID NO:49 |
| | LCDR3 | QQYYAYPWT | SEQ ID NO:50 |
| Anti-HER3 antibody A | HCDR1 | DYAMH | SEQ ID NO:51 |
| | HCDR2 | GISWNSGSIGYADSVKG | SEQ ID NO:52 |
| | HCDR3 | EGLPGLDY | SEQ ID NO:53 |
| | LCDR1 | RASQHVGTYLN | SEQ ID NO:54 |
| | LCDR2 | GAANLQS | SEQ ID NO:55 |
| | LCDR3 | QQSYNTPPFS | SEQ ID NO:56 |

A bispecific antibody with the following structures was constructed:

a first heavy chain that is, from the N-terminus to the C-terminus, anti-MET antibody heavy chain variable region-GGGGS-[Obscurin-O chain]-[IgG1 Fc1],
a first light chain that is, from the N-terminus to the C-terminus, anti-MET antibody light chain variable region-GGGGS-[Titin-T chain],
a second heavy chain that is, from the N-terminus to the C-terminus, anti-HER3 antibody heavy chain variable region-[CH1]-[IgG1 Fc2], and
a second light chain that is, from the N-terminus to the C-terminus, anti-HER3 antibody light chain variable region-[CL];
wherein -- represents a peptide bond; the Obscurin-O chain is set forth in SEQ ID NO: 63; the Titin-T chain is set forth in SEQ ID NO: 64; the CH1 is set forth in SEQ ID NO: 65; the CL is set forth in SEQ ID NO: 66; the IgG1 Fc1 is set forth in SEQ ID NO: 67; and the IgG1 Fc2 is set forth in SEQ ID NO: 68.

Anti-HER3/MET bispecific antibodies 2232-01 (A13-A), 2232-02 (S3-A), A9-A, P8-A, P3-A, and 5D5-A were constructed by operably linking the heavy and light chain variable regions of the heavy chain of antibody A with the heavy and light chain variable regions selected from the group consisting of antibody A13, antibody S3, antibody A9, antibody P8, antibody P3, and antibody 5D5 according to the structures described above. Among them, the heavy chain (H) and light chain (L) sequences of 2232-01 and 2232-02 are shown below:

> 2232-01 H1

QVQLVQSGAEVKKPGASVKVSCEASGYTFTSYGFSWVRQAPGQGLEWMGWIS
ASNGNTYYAQKLQGRVTMTTDTSTSSAYMELRSLRSDDTAVYYCARVYADYA
DYWGQGTLVTVSS***GGGGS***SGAPRFLTRPKASVVSVGKDATLSCQIVGNPFPQV
SWEKDKQPVTAGVRFRLAQDGDLYRLKILDLQLSDSGQYVCRARNAHGEAFA
CLGLQVDAEA*DKTHTCPPCPAPELLGGPSVFLFPPKPKDTLMISRTPEVTCVVVDV*

*SHEDPEVKFNWYVDGVEVHNAKTKPREEQYNSTYRVVSVLTVLHQDWLNGKEYKC*
*KVSNKALPAPIEKTISKAKGQPREPQVYTLPPCREEMTKNQVSLWCLVKGFYPSDIAV*
*EWESNGQPENNYKTTPPVLDSDGSFFLYSKLTVDKSRWQQGNVFSCSVMHEALHN*
*HYTQKSLSLSPGK* (SEQ ID NO: 57)

> 2232-01 L1

DIQMTQSPSSVSASVGDRVTITCRASQGINTWLAWYQQKPGKAPKLLIYAASSLK
SGVPSRFSGSGSGADFTLTISSLQPEDFATYYCQQANSFPLTFGGGTKVEIK***GGGGS***
GIPPKIECLPIDISIDEGKVLTVASAFTGEPTPEVTWSTGGRKIHSQEQGRFHIENTD
DSTTLTIKDVQKQDGGLYTLTLRNEFGSDSATVNIHIRSI (SEQ ID NO: 58)

> 2232-01 H2

QVQLVQSGGGLVQPGRSLRLSCAASGFTFDDYAMHWVRQAPGKGLEWVSGIS
WNSGSIGYADSVKGRFTISRDNAKNSLYLQMNSLRAEDTALYYCAKEGLPGLD
YWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVD
KKVEPKSC*DKTHTCPPCPAPELLGGPSVFLFPPKPKDTLMISRTPEVTCVVVDVSH*
*EDPEVKFNWYVDGVEVHNAKTKPREEQYNSTYRVVSVLTVLHQDWLNGKEYKCKVS*
*NKALPAPIEKTISKAKGQPREPQVCTLPPSREEMTKNQVSLSCAVKGFYPSDIAVEWE*
*SNGQPENNYKTTPPVLDSDGSFFLVSKLTVDKSRWQQGNVFSCSVMHEALHNHYTQ*
*KSLSLSPGK* (SEQ ID NO: 59)

> 2232-01 L2

DIQMTQSPSSLSASIGDRATITCRASQHVGTYLNWYQQKPGKTPKLLISGAAN
LQSGVPSRFSGSGSGTDFTLTISSLQPEDFATYYCQQSYNTPPFSFGQGTKVEI
KRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNR
GEC (SEQ ID NO: 60)

> 2232-02 H1

QVQLVESGGGVVQPGRSLRLSCAASGFSLSNYGVHWVRQAPGKGLEWLAVIW
SGGSTNYAAAFVSRLTISKDNSKNTVYLQMNSLRAEDTAVYYCARNHDNPYN
YAMDYWGQGTTVTVSS***GGGGS***SGAPRFLTRPKASVVSVGKDATLSCQIVGNPF
PQVSWEKDKQPVTAGVRFRLAQDGDLYRLKILDLQLSDSGQYVCRARNAHGE
AFACLGLQVDAEA*DKTHTCPPCPAPELLGGPSVFLFPPKPKDTLMISRTPEVTCVV*
*VDVSHEDPEVKFNWYVDGVEVHNAKTKPREEQYNSTYRVVSVLTVLHQDWLNGKE*
*YKCKVSNKALPAPIEKTISKAKGQPREPQVYTLPPCREEMTKNQVSLWCLVKGFYPS*
*DIAVEWESNGQPENNYKTTPPVLDSDGSFFLYSKLTVDKSRWQQGNVFSCSVMHEA*
*LHNHYTQKSLSLSPGK* (SEQ ID NO: 61)

> 2232-02 L1

DIVLTQSPDSLAVSLGERATINCRADKSVSTSTYNYLHWYQQKPGQPPKLLI
YLASNLASGVPDRFSGSGSGTDFTLTISSLQAEDVAVYYCQHSRDLPPTFGQ
GTKLEIK***GGGGS***GIPPKIECLPIDISIDEGKVLTVASAFTGEPTPEVTWSTGGR
KIHSQEQGRFHIENTDDSTTLTIKDVQKQDGGLYTLTLRNEFGSDSATVNIHI
RSI (SEQ ID NO: 62)

2232-02 H2 is identical to SEQ ID NO: 59;
2232-02 L2 is identical to SEQ ID NO: 60.

In the above heavy chain sequences, the Fc constant region of IgG1 is in italics, the Obscurin-O chain is underlined with a wavy line, and the VH1 is underlined with a horizontal line; in the light chain sequences, the Cκ of IgG1 is underlined with a horizontal line, and the Titin-T chain is underlined with a wavy line; and the GGGGS (SEQ ID NO: 91) linker is in italics and bold.

> Obscurin-O chain

SGAPRFLTRPKASVVSVGKDATLSCQIVGNPFPQVSWEKDKQPVTAGVRFRLAQD
GDLYRLKILDLQLSDSGQYVCRARNAHGEAFACLGLQVDAEA (SEQ ID NO: 63)

> Titin-T chain

GIPPKIECLPIDISIDEGKVLTVASAFTGEPTPEVTWSTGGRKIHSQEQGRFHIENT
DDSTTLTIKDVQKQDGGLYTLTLRNEFGSDSATVNIHIRSI (SEQ ID NO: 64)

> CH1

ASTKGPSVFPLAPSSKSTSGGTAALGCLVKDYFPEPVTVSWNSGALTSGVH
TFPAVLQSSGLYSLSSVVTVPSSSLGTQTYICNVNHKPSNTKVDKKVEPKS
C (SEQ ID NO: 65)

> CL

RTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNR
GEC (SEQ ID NO: 66)

> IgG1 Fc1 (S354C/T366W)

DKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQVYTLPP**C**REEMTKNQVSL**W**CLVKGFYPSDIAVE
WESNGQPENNYKTTPPVLDSDGSFFLYSKLTVDKSRWQQGNVFSCSVMHEAL
HNHYTQKSLSLSPGK

(SEQ ID NO: 67)

> IgG1 Fc2 (Y349C/T366S/L368A/Y407V)

DKTHTCPPCPAPELLGGPSVFLFPPKPKDTLMISRTPEVTCVVVDVSHEDPEVKF
NWYVDGVEVHNAKTKPREEQYNSTYRVVSVLTVLHQDWLNGKEYKCKVSNK
ALPAPIEKTISKAKGQPREPQV**C**TLPPSREEMTKNQVSL**S**C**A**VKGFYPSDIAVEW
ESNGQPENNYKTTPPVLDSDGSFFL**V**SKLTVDKSRWQQGNVFSCSVMHEALHN
HYTQKSLSLSPGK

(SEQ ID NO: 68)

2. Antibody engineering

Four sets of mutations N93Q, N93T, N93S, and T94V against the anti-HER3 antibody, i.e., antibody A, (comprising a heavy chain set forth in SEQ ID NO: 86 and a light chain set forth in SEQ ID NO: 87) were designed. The antibody A N93Q comprises a mutation of N to Q only at position 93 of VL relative to the antibody A, the antibody A N93T comprises a mutation of N to T only at position 93 of VL relative to the antibody A, the antibody A N93S comprises a mutation of N to S only at position 93 of VL relative to the antibody A, and the antibody A T94V comprises a mutation of T to V only at position 94 of VL relative to the antibody A.

Experimental protocol: The antibody A, antibody A N93Q, antibody A N93T, antibody A N93S, and antibody A T94V were diluted to 1 μg/mL with the HBS-EP+ buffer at a flow rate of 10 μL/min, and the antibodies were captured to a level of 200 RU. A His-tagged human HER3 antigen was diluted with the HBS-EP+ buffer at a certain ratio, with concentration gradients of 6.25 nM, 12.5 nM, 25 nM, 50 nM, 100 nM, 200 nM, and 400 nM. The flow rate was set to 30 μL/min during sample analysis. The association time was 120 s, and the dissociation time was 900 s. Subsequently, regeneration was performed for 30 s. A Gly-HCl buffer at pH 1.5 was used as the regeneration buffer, and the regeneration flow rate was set to 30 μL/min. Their response signals were plotted with analysis time on the abscissa and response value on the ordinate. The data obtained were fitted by the BIAcore 8K analysis software, and their kinetic constants, such as the association rate constant (Ka), dissociation rate constant (Kd), and dissociation equilibrium constant (KD), were determined using a 1:1 Langmuir binding model.

Experimental results: The results in Table 3-1 and FIG. 2 show that the binding of the antibody to HER3 before and after the T94V mutation exhibited no significant change, but the deamidation level of the antibody was significantly reduced.

Table 3-1. Binding activity of antibody A before and after mutation

| Antibody name | ka (1/Ms) | kd (1/s) | KD (M) |
|---|---|---|---|
| Antibody A | 7.07E+05 | 1.03E-02 | 1.45E-08 |
| Antibody A N93Q | 5.73E+05 | 2.95E-02 | 5.14E-08 |
| Antibody A N93T | 3.36E+05 | 3.29E-02 | 9.79E-08 |

(continued)

| Antibody name | ka (1/Ms) | kd (1/s) | KD (M) |
|---|---|---|---|
| Antibody A N93S | 6.59E+05 | 2.15E-02 | 3.26E-08 |
| Antibody A T94V | 7.17E+05 | 1.08E-02 | 1.50E-08 |

Among them, the variable region sequences of the antibody A T94V are shown below:

> Anti-HER3 antibody A VH

QVQLVQSGGGLVQPGRSLRLSCAASGFTFDDYAMHWVRQAPGKGLEWVSGIS WNSGSIGYADSVKGRFTISRDNAKNSLYLQMNSLRAEDTALYYCAKEGLPGLD YWGQGTLVTVSS (SEQ ID NO: 13)

> Anti-HER3 antibody A VL T94V

DIQMTQSPSSLSASIGDRATITCRASQHVGTYLNWYQQKPGKTPKLLISGAANL QSGVPSRFSGSGSGTDFTLTISSLQPEDFATYYCQQSYN**V**PPFSFGQGTKVEIK (SEQ ID NO: 70)

Four sets of mutations N30Q, N30T, N30S, and T31V against the anti-MET antibody, i.e., antibody A13, (comprising a heavy chain set forth in SEQ ID NO: 84 and a light chain set forth in SEQ ID NO: 85) were designed. The antibody A13 N30Q comprises a mutation of N to Q only at position 30 of VL relative to the antibody A13, the antibody A13 N30T comprises a mutation of N to T only at position 30 of VL relative to the antibody A13, the antibody A13 N30S comprises a mutation of N to S only at position 30 of VL relative to the antibody A13, and the antibody A13 T31V comprises a mutation of T to V only at position 31 of VL relative to the antibody A13.

Experimental protocol: The antibody A13, antibody A13 N30Q, antibody A13 N30T, antibody A13 N30S, and antibody A13 T31V were diluted to 1 μg/mL with the HBS-EP+ buffer at a flow rate of 10 μL/min, and the antibodies were captured to a level of 200 RU. A His-tagged human MET antigen was diluted with the HBS-EP+ buffer at a certain ratio, with concentration gradients of 6.25 nM, 12.5 nM, 25 nM, 50 nM, 100 nM, 200 nM, and 400 nM. The flow rate was set to 30 μL/min during sample analysis. The association time was 120 s, and the dissociation time was 900 s. Subsequently, regeneration was performed for 30 s. A Gly-HCl buffer at pH 1.5 was used as the regeneration buffer, and the regeneration flow rate was set to 30 μL/min. Their response signals were plotted with analysis time on the abscissa and response value on the ordinate. The data obtained were fitted by the BIAcore 8K analysis software, and their kinetic constants, such as the association rate constant (Ka), dissociation rate constant (Kd), and dissociation equilibrium constant (KD), were determined using a 1:1 Langmuir binding model.

Experimental results: The results in Table 3-2 show that the binding of the antibody to HER3 before and after the N30S mutation exhibited no significant change, and the deamidation level of the antibody was significantly reduced.

Table 3-2. Binding activity of antibody A13 before and after mutation

| Antibody name | ka (1/Ms) | kd (1/s) | KD (M) |
|---|---|---|---|
| Antibody A13 | 9.38E+04 | 6.14E-04 | 6.55E-09 |
| Antibody A13 N30Q | 8.30E+04 | 1.46E-03 | 1.76E-08 |
| Antibody A13 N30T | 1.06E+05 | 1.80E-03 | 1.70E-08 |
| Antibody A13 N30S | 1.01E+05 | 7.76E-04 | 7.69E-09 |
| Antibody A13 T31V | 5.60E+04 | 5.21E-03 | 9.31E-08 |

On the basis of N30S, the framework region (FR) of the anti-MET antibody A13 was further mutated. Specifically, E23K and S78T mutations were further introduced into the VH, and an A69T mutation was further introduced into the VL, thereby obtaining antibody A13 E23K/S78T/A69T/N30S. After analysis, the expression level of the antibody A13 E23K/S78T/A69T/N30S was increased from 96 mg/L to 200 mg/L.

The variable region sequences of the antibody A13 E23K/S78T/A69T/N30S are shown below:

> Anti-MET antibody A13 E23K/S78T/A69T/N30 VH

QVQLVQSGAEVKKPGASVKVSCKASGYTFTSYGFSWVRQAPGQGLEWMGWIS ASNGNTYYAQKLQGRVTMTTDTSTSTAYMELRSLRSDDTAVYYCARVYADY ADYWGQGTLVTVSS

(SEQ ID NO: 71)

> Anti-MET antibody A13 E23K/S78T/A69T/N30 VL

DIQMTQSPSSVSASVGDRVTITCRASQGISTWLAWYQQKPGKAPKLLIYAASSL KSGVPSRFSGSGSGTDFTLTISSLQPEDFATYYCQQANSFPLTFGGGTKVEIK

(SEQ ID NO: 72)

Table 3-3. CDRs of antibodies (Kabat numbering scheme)

| Antibody name | CDR sequences | | SEQ ID NO: |
|---|---|---|---|
| Antibody A | HCDR1 | DYAMH | 51 |
| | HCDR2 | GISWNSGSIGYADSVKG | 52 |
| | HCDR3 | EGLPGLDY | 53 |
| | LCDR1 | RASQHVGTYLN | 54 |
| | LCDR2 | GAANLQS | 55 |
| | LCDR3 | QQSYNTPPFS | 56 |
| Antibody A T94V | HCDR1 | DYAMH | 51 |
| | HCDR2 | GISWNSGSIGYADSVKG | 52 |
| | HCDR3 | EGLPGLDY | 53 |
| | LCDR1 | RASQHVGTYLN | 54 |
| | LCDR2 | GAANLQS | 55 |
| | LCDR3 | QQSYNVPPFS | 73 |
| Antibody A13 | HCDR1 | SYGFS | 15 |
| | HCDR2 | WISASNGNTYYAQKLQG | 16 |
| | HCDR3 | VYADYADY | 17 |
| | LCDR1 | RASQGINTWLA | 18 |
| | LCDR2 | AASSLKS | 19 |
| | LCDR3 | QQANSFPLT | 20 |
| Antibody A13 E23K S78T A69T N30S | HCDR1 | SYGFS | 15 |
| | HCDR2 | WISASNGNTYYAQKLQG | 16 |
| | HCDR3 | VYADYADY | 17 |
| | LCDR1 | RASQGISTWLA | 74 |
| | LCDR2 | AASSLKS | 19 |
| | LCDR3 | QQANSFPLT | 20 |

That is, the antibody A of the present disclosure has the following general sequence:

HCDR1: DYAMH (SEQ ID NO: 51)
HCDR2: GISWNSGSIGYADSVKG (SEQ ID NO: 52)
HCDR3: EGLPGLDY (SEQ ID NO: 53)
LCDR1: RASQHVGTYLN (SEQ ID NO: 54)
LCDR2: GAANLQS (SEQ ID NO: 55)
LCDR3: QQSY$X_1X_2$PPFS (SEQ ID NO: 75), wherein $X_1$ is selected from the group consisting of N, Q, T, and S, and $X_2$ is selected from the group consisting of T and V.

The antibody A13 of the present disclosure has the following general sequence:

HCDR1: SYGFS (SEQ ID NO: 15)
HCDR2: WISASNGNTYYAQKLQG (SEQ ID NO: 16)
HCDR3: VYADYADY (SEQ ID NO: 17)
LCDR1: RASQGI$X_3X_4$WLA (SEQ ID NO: 76), wherein $X_3$ is selected from the group consisting of N, Q, T, and S, and $X_4$ is selected from the group consisting of T and V.
LCDR2: AASSLKS (SEQ ID NO: 19)
LCDR3: QQANSFPLT (SEQ ID NO: 20).

The anti-HER3/MET bispecific antibody 2232-06 was constructed using the mutated sequences described above. The full-length amino acid sequence is shown below:

> 2232-06 (A13 E23K/S78T/A69T/N30S, A T94V) H1 heavy chain

QVQLVQSGAEVKKPGASVKVSCKASGYTFTSYGFSWVRQAPGQGLEWMGWIS
ASNGNTYYAQKLQGRVTMTTDTSTSTAYMELRSLRSDDTAVYYCARVYADY
ADYWGQGTLVTVSSGGGGSSGAPRFLTRPKASVVSVGKDATLSCQIVGNPFPQ
VSWEKDKQPVTAGVRFRLAQDGDLYRLKILDLQLSDSGQYVCRARNAHGEAF
ACLGLQVDAEADKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWL
NGKEYKCKVSNKALPAPIEKTISKAKGQPREPQVYTLPPCREEMTKNQVSLWC
LVKGFYPSDIAVEWESNGQPENNYKTTPPVLDSDGSFFLYSKLTVDKSRWQQG
NVFSCSVMHEALHNHYTQKSLSLSPGK (SEQ ID NO: 77)

> 2232-06 (A13 E23K/S78T/A69T/N30S, A T94V) L1 light chain

DIQMTQSPSSVSASVGDRVTITCRASQGISTWLAWYQQKPGKAPKLLIYAASSLK
SGVPSRFSGSGSGTDFTLTISSLQPEDFATYYCQQANSFPLTFGGGTKVEIKGGGG
SGIPPKIECLPIDISIDEGKVLTVASAFTGEPTPEVTWSTGGRKIHSQEQGRFHIENT
DDSTTLTIKDVQKQDGGLYTLTLRNEFGSDSATVNIHIRSI (SEQ ID NO: 78)

> 2232-06 (A13 E23K/S78T/A69T/N30S, A T94V) H2 heavy chain that is identical to SEQ ID NO: 59;
> 2232-06 (A13 E23K/S78T/A69T/N30S, A T94V) L2 light chain

DIQMTQSPSSLSASIGDRATITCRASQHVGTYLNWYQQKPGKTPKLLISGAAN
LQSGVPSRFSGSGSGTDFTLTISSLQPEDFATYYCQQSYNVPPFSFGQGTKVEI
KRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNR
GEC (SEQ ID NO: 79)

3. Control antibody

The full-length sequences of the heavy chains (HC) and the light chains (LC) of the naked antibody (U3-1402 Ab) of the control antibody U3-1402, the antibody S3, the antibody A13, and the antibody A are shown below:

> U3-1402 Ab HC

QVQLQQWGAGLLKPSETLSLTCAVYGGSFSGYYWSWIRQPPGKGLEWIGEINH
SGSTNYNPSLKSRVTISVETSKNQFSLKLSSVTAADTAVYYCARDKWTWYFDL
WGRGTLVTVSSASTKGPSVFPLAPSSKSTSGGTAALGCLVKDYFPEPVTVSWNS

GALTSGVHTFPAVLQSSGLYSLSSVVTVPSSSLGTQTYICNVNHKPSNTKVDKR
VEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPS
DIAVEWESNGQPENNYKTTPPVLDSDGSFFLYSKLTVDKSRWQQGNVFSCSVM
HEALHNHYTQKSLSLSPGK (SEQ ID NO: 80)

> U3-1402 Ab LC

DIEMTQSPDSLAVSLGERATINCRSSQSVLYSSSNRNYLAWYQQNPGQPPKLLI
YWASTRESGVPDRFSGSGSGTDFTLTISSLQAEDVAVYYCQQYYSTPRTFGQGT
KVEIKRTVAAPSVFIFPPSDEQLKSGTASVVCLLNNFYPREAKVQWKVDNALQS
GNSQESVTEQDSKDSTYSLSSTLTLSKADYEKHKVYACEVTHQGLSSPVTKSFN
RGEC (SEQ ID NO: 81)

> Antibody S3 HC

QVQLVESGGGVVQPGRSLRLSCAASGFSLSNYGVHWVRQAPGKGLEWLAVIW
SGGSTNYAAAFVSRLTISKDNSKNTVYLQMNSLRAEDTAVYYCARNHDNPYN
YAMDYWGQGTTVTVSSASTKGPSVFPLAPCSRSTSESTAALGCLVKDYFPEPVT
VSWNSGALTSGVHTFPAVLQSSGLYSLSSVVTVPSSNFGTQTYTCNVDHKPSNT
KVDKTVERKCCVECPPCPAPPVAGPSVFLFPPKPKDTLMISRTPEVTCVVVDVS
HEDPEVQFNWYVDGVEVHNAKTKPREEQFNSTFRVVSVLTVVHQDWLNGKE
YKCKVSNKGLPAPIEKTISKTKGQPREPQVYTLPPSREEMTKNQVSLTCLVKGF
YPSDIAVEWESNGQPENNYKTTPPMLDSDGSFFLYSKLTVDKSRWQQGNVFSC
SVMHEALHNHYTQKSLSLSPGK (SEQ ID NO: 82)

> Antibody S3 LC

DIVLTQSPDSLAVSLGERATINCRADKSVSTSTYNYLHWYQQKPGQPPKLLIYL
ASNLASGVPDRFSGSGSGTDFTLTISSLQAEDVAVYYCQHSRDLPPTFGQGTKL
EIKRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNRG
EC (SEQ ID NO: 83)

> Antibody A13 HC

QVQLVQSGAEVKKPGASVKVSCEASGYTFTSYGFSWVRQAPGQGLEWMGWIS
ASNGNTYYAQKLQGRVTMTTDTSTSSAYMELRSLRSDDTAVYYCARVYADYA
DYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKV
DKKVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGK
EYKCKVSNKALPAPIEKTISKAKGQPREPQVYTLPPSREEMTKNQVSLTCLVKG
FYPSDIAVEWESNGQPENNYKTTPPVLDSDGSFFLYSKLTVDKSRWQQGNVFS
CSVMHEALHNHYTQKSLSLSPGK

(SEQ ID NO: 84)

> Antibody A13 LC

DIQMTQSPSSVSASVGDRVTITCRASQGINTWLAWYQQKPGKAPKLLIYAASSL
KSGVPSRFSGSGSGADFTLTISSLQPEDFATYYCQQANSFPLTFGGGTKVEIKRT
VAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

(SEQ ID NO: 85)

> Antibody A HC

QVQLVQSGGGLVQPGRSLRLSCAASGFTFDDYAMHWVRQAPGKGLEWVSGIS
WNSGSIGYADSVKGRFTISRDNAKNSLYLQMNSLRAEDTALYYCAKEGLPGLD
YWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVD
KKVEPKSCDKTHTCPPCPAPELLGGPSVFLFPPKPKDTLMISRTPEVTCVVVDVS
HEDPEVKFNWYVDGVEVHNAKTKPREEQYNSTYRVVSVLTVLHQDWLNGKE
YKCKVSNKALPAPIEKTISKAKGQPREPQVYTLPPSRDELTKNQVSLTCLVKGF
YPSDIAVEWESNGQPENNYKTTPPVLDSDGSFFLYSKLTVDKSRWQQGNVFSC
SVMHEALHNHYTQKSLSLSPGK (SEQ ID NO: 86)

> Antibody A LC

DIQMTQSPSSLSASIGDRATITCRASQHVGTYLNWYQQKPGKTPKLLISGAAN
LQSGVPSRFSGSGSGTDFTLTISSLQPEDFATYYCQQSYNTPPFSFGQGTKVEI
KRTVAAPSVFIFPPSDEQLKSGTASVVCLLNNFYPREAKVQWKVDNALQSGN
SQESVTEQDSKDSTYSLSSTLTLSKADYEKHKVYACEVTHQGLSSPVTKSFNR
GEC (SEQ ID NO: 87)

4. Preparation of antibodies

The nucleotide sequences encoding the heavy chains and the light chains of the antibodies were separately cloned into a pTT5 vector and then transfected into ExpiCHO cells. After 8 days, the cells were removed by centrifugation,

and the cell culture media were collected and filtered. The harvested cell culture media were purified by using a Protein A affinity column (MabSelect SuRe, GE). The bound antibodies were separately eluted with glycine, and the eluates were neutralized with 1 M Tris and then desalted. Analysis showed that the antibodies of interest were obtained.

**Example 3. Assay for Antigen-Binding Activity of Anti-HER3/MET Bispecific Antibodies**

In this example, the binding activity of anti-HER3/MET bispecific antibodies to human MET protein and HER3/MET protein on the cell surface was assayed by FACS.

1. Assay for binding activity to MET protein on cell surface

Experimental method: NCI-H1975 (lung cancer cell line) was a MET single-positive cell line. The cell culture medium was RPMI 1640 culture medium (ATCC modification) (Gibco, Cat# A1049101) containing 10% fetal bovine serum. The experimental culture medium was sterile PBS (phosphate-buffered saline, pH 7.40) containing 2% fetal bovine serum (the same applies hereinafter). The NCI-H1975 cells were washed twice with the experimental culture medium and seeded into a 96-well U-bottom plate at $1 \times 10^5$ cells/well, and test samples at different concentrations were added. The cells were incubated at 4 °C for 1 h and then washed twice with the experimental culture medium. Subsequently, the Alexa Fluor 647-mouse anti-human (IgG, Fc$\gamma$ fragment specific) antibody (Jackson, Cat# 209-605-098) was added. After two washes, the fluorescence signal values were measured using a flow cytometer.

Experimental results: The FACS results in FIG. 3 show that different anti-HER3/MET bispecific antibodies (with the same anti-HER3 antibody sequence, and different anti-MET antibody sequences) exhibited different binding abilities to MET single-positive cells, wherein A13-A has the strongest binding ability and is an anti-HER3/MET bispecific antibody with high affinity for MET; P8-A has the weakest binding ability and is an anti-HER3/MET bispecific antibody with low affinity for MET; and the binding $EC_{50}$ of S3-A differs by 10-fold from that of A13-A and S3-A is an anti-HER3/MET bispecific antibody with moderate-to-low affinity for MET. 2232-01 (A13-A) with high affinity for MET and 2232-02 (S3-A) with moderate-to-low affinity for MET were selected for the next screening step.

2. Assay for binding activity to human HER3/MET protein on cell surface

Experimental method: MDA-MB-453 (breast cancer cell line) was a HER3 single-positive cell line, the cell culture medium was Leibovitz's L-15 (Gibco, Cat# 11415064) containing 10% fetal bovine serum, and the cells were cultured in air. NCI-H1703 (lung cancer cell line) was a MET single-positive cell line, and the cell culture medium was RPMI 1640 culture medium (ATCC modification) (Gibco, Cat# A1049101) containing 10% fetal bovine serum. HCC827 Osimertinib-resistant cell strain (lung cancer cell line) was a HER3/MET double-positive cell constructed in-house, which was obtained by subjecting HCC827 cells to gradient drug resistance induction with Osimertinib, and the cell culture medium was RPMI 1640 culture medium (ATCC modification) (Gibco, Cat# A1049101) containing 10% fetal bovine serum. The MDA-MB-453, NCI-H1703, or HCC827 Osimertinib-resistant cells were washed twice with the experimental culture medium and seeded into a 96-well U-bottom plate at $1 \times 10^5$ cells/well, and test samples at different concentrations were added. The cells were incubated at 4 °C for 1 h and then washed twice with the experimental culture medium. Subsequently, the Alexa Fluor 647-mouse anti-human (IgG, Fc$\gamma$ fragment specific) antibody (Jackson, Cat# 209-605-098) was added. After two washes, the fluorescence signal values were measured using a flow cytometer. IgG1 is an isotype control antibody. The same applies hereinafter.

Experimental results: The FACS assay results in Tables 4-1 and 4-2 and FIGs. 4A and 4B show that the anti-HER3/MET bispecific antibodies were capable of binding to the HER3/MET antigen on the cell surface. In MET single-positive cells, 2232-01 with high affinity for MET exhibited better binding ability than that of 2232-02 with moderate-to-low affinity for MET. In HER3 single-positive cells, the binding abilities of the two molecules to HER3 were similar and slightly weaker than that of the antibody A.

The results in Table 4-3 and FIG. 4C show that, in HER3/MET double-positive cells, the maximum fluorescence values of the anti-HER3/MET bispecific antibodies were higher than those of the anti-HER3 antibodies (antibody A and U3-1402 Ab) and the anti-MET antibody (antibody S3), indicating that more anti-HER3/MET bispecific antibodies bind to the double-positive cells.

Table 4-1. Results of binding of anti-HER3/MET bispecific antibodies to HER3 single-positive cells

| Antibody name | $EC_{50}$ (nM) | Maximum fluorescence value |
|---|---|---|
| 2232-01 | 2.72 | 1,947 |
| 2232-02 | 2.64 | 1,866 |

(continued)

| Antibody name | $EC_{50}$ (nM) | Maximum fluorescence value |
|---|---|---|
| Antibody S3 | - | 77 |
| Antibody A | 0.48 | 1,857 |
| U3-1402 Ab | 0.25 | 1,993 |
| IgG1 | - | 74 |

Table 4-2. Results of binding of anti-HER3/MET bispecific antibodies to MET single-positive cells

| Antibody name | $EC_{50}$ (nM) | Maximum fluorescence value |
|---|---|---|
| 2232-01 | 1.03 | 2,427 |
| 2232-02 | 10.62 | 2,042 |
| Antibody S3 | 1.27 | 1,930 |
| Antibody A | - | 81 |
| U3-1402 Ab | - | 90 |
| IgG1 | - | 74 |

Table 4-3. Results of binding of anti-HER3/MET bispecific antibodies to HER3/MET double-positive cells

| Antibody name | $EC_{50}$ (nM) | Maximum fluorescence value |
|---|---|---|
| 2232-01 | 0.52 | 2473 |
| 2232-02 | 2.16 | 2297 |
| Antibody S3 | 2.15 | 1697 |
| Antibody A | 2.06 | 1023 |
| U3-1402 Ab | 0.46 | 1235 |
| IgG1 | - | 74 |

**Example 4. Assay for Endocytic Activity of Anti-HER3/MET Bispecific Antibodies**

1. αHFc-CL-MMAE toxin antibody internalization activity evaluation

The internalization and toxin killing activities of the antibodies were evaluated using the αHFc-CL-MMAE toxin antibody internalization activity evaluation system. αHFc-CL-MMAE is an anti-human Fc antibody-conjugated MMAE toxin with a stable cleavable linker, which is capable of specifically binding to the Fc portion of human IgG. MMAE is a toxic small molecule that inhibits cell division by blocking tubulin polymerization. The linker is stable in the extracellular matrix and can be cleaved by lysosomal cathepsin upon entry into the cell by endocytosis, thereby releasing the toxin. Therefore, the endocytic activity of the antibody can be evaluated based on the cell killing activity.

Experimental method: NCI-H441 (lung cancer cell line) was used as a MET/HER3 double-positive cell line, MDA-MB-453 (breast cancer cell line) was used as a HER3 single-positive cell line, NCI-H1703 (lung cancer cell line) was used as a MET single-positive cell line, and HCC827 Osimertinib-resistant cell strain (lung cancer cell line, constructed in-house) was used as a MET/HER3 double-positive cell line, which was obtained by subjecting HCC827 cells to gradient resistance induction with Osimertinib. The culture medium and culture conditions were the same as those in Example 3. Equimolar concentrations of αHFc-CL-MMAE (Moradec, Cat# AH-102AE-50) and the test antibody were mixed in a volume ratio of 1:1, and the mixture was left to stand and incubated at 37 °C for 30 min. Subsequently, the mixture was serially diluted 3-fold with a complete culture medium and added to the cells that had been plated one day in advance (600 cells/well), and the cells were incubated in a 5% $CO_2$ incubator at 37 °C for 6 days. After the incubation was completed, CellTiter-Glo (Promega, Cat# G7570) was added, and the cells were incubated in the dark at room temperature for 10 min. The chemiluminescence was read on the PerkinElmer ENVISION, and the $EC_{50}$ value and Emax value (relative to the reading of the antibody-free group) were calculated.

Experimental results: The results in Table 5-1 and FIG. 5A show that different anti-HER3/MET bispecific

antibodies (with the same anti-HER3 antibody sequence and different anti-MET antibody sequences) exhibited different killing activities on HER3/MET double-positive cells, wherein A13-A has the strongest killing ability and P3-A has the weakest killing ability. 2232-01 (A13-A) and 2232-02 (S3-A) were selected for the next screening step to explore the anti-tumor activity of bispecific antibodies ADCs with different MET affinities.

The cell activity assay results in Tables 5-2 and 5-3 and FIGs. 5B and 5C show that, in HER3 single-positive cells, the αHFc-CL-MMAE toxin antibody killing activities of the anti-HER3/MET bispecific antibodies were similar to that of the antibody A.In MET single-positive cells, the αHFc-CL-MMAE toxin antibody killing activity of 2232-01 was superior to that of 2232-02.

The cell activity assay results in Table 5-4 and FIG. 5D show that, in double-positive cells, the αHFc-CL-MMAE toxin antibody killing activities of the anti-HER3/MET bispecific antibodies were stronger than those of the antibody S3 or antibody A and U3-1402 Ab, indicating that the endocytic activities of the bispecific antibodies were stronger in the double-positive cells.

Table 5-1. Assay results of endocytic activity of anti-HER3/MET bispecific antibodies in HER3/MET double-positive cells (NCI-H441)

| Antibody name | $EC_{50}$ (nM) | Minimum cell viability (%) |
|---|---|---|
| S3-A | 0.15 | 16.1 |
| A13-A | 0.04 | 12.4 |
| A9-A | 0.15 | 15.9 |
| P8-A | 0.07 | 12.1 |
| P3-A | 0.28 | 16.4 |
| 5D5-A | 0.14 | 20.5 |
| IgG1 | - | 90.9 |

Table 5-2. Assay results of endocytic activity of anti-HER3/MET bispecific antibodies in HER3 single-positive cells (MDA-MB-453)

| Antibody name | $EC_{50}$ (nM) | Minimum cell viability (%) |
|---|---|---|
| 2232-01 | 0.18 | 21.7 |
| 2232-02 | 0.18 | 25.8 |
| Antibody S3 | - | 97.3 |
| Antibody A | 0.11 | 28.5 |
| U3-1402 Ab | 0.52 | 63.9 |
| IgG1 | - | 92.9 |

Table 5-3. Assay results of endocytic activity of anti-HER3/MET bispecific antibodies in MET single-positive cells (NCI-H1703)

| Antibody name | $EC_{50}$ (nM) | Minimum cell viability (%) |
|---|---|---|
| 2232-01 | 0.05 | 0.6 |
| 2232-02 | 0.24 | 0.8 |
| Antibody S3 | 0.20 | 0.8 |
| Antibody A | - | 77.8 |
| U3-1402 Ab | - | 72.9 |
| IgG1 | - | 78.6 |

Table 5-4. Assay results of endocytic activity of anti-HER3/MET bispecific antibodies in HER3/MET double-positive cells (HCC827 Osimertinib)

| Antibody name | $EC_{50}$ (nM) | Minimum cell viability (%) |
|---|---|---|
| 2232-01 | 0.14 | 24.7 |
| 2232-02 | 0.36 | 30.2 |
| Antibody S3 | 1.1 | 33.3 |
| Antibody A | 22.9 | 63.8 |
| U3-1402 Ab | 17.2 | 73.6 |
| IgG1 | - | 101.6 |

2. Zenon™ pHrodo™ iFL IgG evaluation

The Zenon™ pHrodo™ iFL IgG indicating reagent (Invitrogen, Cat# Z25612) provides a rapid, real-time, and reliable method to evaluate the internalization of antibodies. The pHrodo iFL Red-labeled Fab fragment is capable of binding to the Fc portion of an intact IgG antibody, thereby forming a labeled complex within 5 minutes. When the complex enters cells through endocytosis, the fluorescence increases sharply as the acidity of the surrounding environment of the complex increases. The fluorescence intensity can be recorded in real time by the Incucyte instrument to determine the degree of internalization of the antibody.

Experimental method: 5000 cells/well of HCC827 Osimertinib-resistant cells were plated in advance and cultured overnight at 50 μL/well. The cell culture medium was RPMI 1640 culture medium containing 10% fetal bovine serum. The next day, the pre-incubated antibody and labeled complex were added at 50 μL/well. The plate was placed in the Incucyte instrument (IncuCyte S3 Live-Cell Analysis System, Sartorius), and the plate reading conditions and time intervals were set according to the instrument operation procedures. After the completion of plate reading, the data were analyzed according to the steps of the analysis software provided by the instrument.

The results in FIG. 6 show that the endocytic activities of the anti-HER3/MET bispecific antibodies were significantly superior to those of the monoclonal antibodies, indicating that more bispecific antibody molecules enter cells through endocytosis.

3. Endocytosis evaluation by FACS

The antibody was endocytosed at 37 °C, and the endocytosis ratio of the antibody at each time point was obtained by comparison with the antibody incubated at 4 °C, such that the endocytic activity of the antibody was evaluated.

Experimental method: HCC827 Osimertinib-resistant cell strain (lung cancer cell line) was a HER3/MET double-positive cell constructed in-house, which was obtained by subjecting HCC827 cells to gradient drug resistance induction with Osimertinib, and the cell culture medium was RPMI 1640 culture medium (ATCC modification) (Gibco, Cat# A1049101) containing 10% fetal bovine serum. The HCC827 Osimertinib-resistant cells were washed twice with the experimental culture medium and seeded into a 96-well U-bottom plate at $1 \times 10^5$ cells/well, and the 96-well U-bottom plate at each time point included a 0 h point (incubation at 4 °C). Test samples at a saturation concentration were added, and the cells were incubated at 4 °C for 1 h and then washed twice with the experimental culture medium. The samples were incubated at 37 °C and 4 °C, and taken out for assay at each time point. Subsequently, the Alexa Fluor 647-mouse anti-human (IgG, Fcγ fragment specific) antibody (Jackson, Cat# 209-605-098) was added. After two washes, the fluorescence signal values were measured using a flow cytometer. Calculation of endocytosis ratio: (MFI of antibody incubated at 4 °C - MFI of antibody incubated at 37 °C) - MFI of antibody incubated at 37 °C.

The results in Table 6 and FIG. 7 show that the endocytosis rate of the anti-HER3 antibody A was superior to that of the anti-MET antibody A13, and the endocytosis rate of the anti-HER3/MET bispecific antibody 2203-01 was superior to those of the anti-HER3 antibody A and the anti-MET antibody A13.

Table 6. Endocytosis ratio at 0-6 h

| Time | 2232-01 | Antibody A13 | Antibody A |
|---|---|---|---|
| 0 h | 0% | 0% | 0% |
| 0.5 h | 67.9% | 25.7% | 55.5% |
| 2 h | 83.2% | 45.7% | 80.0% |

(continued)

| Time | 2232-01 | Antibody A13 | Antibody A |
|---|---|---|---|
| 6 h | 84.1% | 57.6% | 82.2% |

**Example 5. Assay for ERK Phosphorylation Activation Activity**

HER3/MET dimerization causes activation of downstream signaling pathways (Tanizaki et al., British Journal of Cancer, 105(6), pp. 807-813 (2011)), thereby promoting proliferation and survival of tumor cells. In order to detect whether the antibodies of the present disclosure have agonist activity, ERK phosphorylation was used for evaluation.

Experimental method: HCC827 Osimertinib-resistant cells were plated in a 96-well flat-bottom plate at $4 \times 10^4$ cells/well and cultured overnight. The cell culture medium was RPMI 1640 culture medium containing 10% fetal bovine serum. The next day, the culture medium was replaced with a low-serum culture medium (50 μL/well), that is, the cell culture medium was RPMI 1640 containing 0.05% fetal bovine serum. The starvation culture was performed for 5 h. The anti-HER3/MET bispecific antibodies and the control antibody were diluted with a serum-free culture medium, and added to the 96-well plate at 50 μL/well. The cells were incubated at 37 °C for 5 min. After the incubation was completed, the ERK phosphorylation level in the tumor was rapidly measured using Advanced Phospho-ERK1/29 (THR202/TYR204) Kits (PerkinElmer, Cat# 64ERKPEG).

Experimental results: FIG. 8 shows that the anti-HER3 antibody A did not cause ERK phosphorylation, and the anti-HER3/MET bispecific antibody 2232-01 did not significantly cause ERK phosphorylation. This indicates that the anti-HER3/MET bispecific antibody does not cause HER3/MET target dimerization to activate downstream signaling pathways. That is, the bispecific antibody 2232-01 of the present disclosure does not cause proliferation and survival of tumor cells.

**Example 6. Design and Preparation of Anti-HER3/MET Antibody-Drug Conjugates (ADCs)**

1. Preparation of compound

1.1. Preparation of compound 9A and compound 9B

*N*-((2*R*,10*S*)-10-Benzyl-2-cyclopropyl-1-(((1*S*,9*S*)-9-ethyl-5-fluoro-9-hydroxy-4-methyl -10,13-dioxo-2,3,9,10,13,15-hexahydro-1*H*,12*H*-benzo[*de*]pyrano[3',4':6,7]indolizino[1, 2-*b*]quinolin-1-yl)amino)-1,6,9,12,15-pentaoxo-3-oxa-5,8,11,14-tetraazahexadec-16-yl) -6-(2,5-dioxo-2,5-dihydro-1*H*-pyrrol-1-yl)hexanamide **9-A**

*N*-((2*S*,10*S*)-10-Benzyl-2-cyclopropyl-1-(((1*S*,9*S*)-9-ethyl-5-fluoro-9-hydroxy-4-methyl -10,13-dioxo-2,3,9,10,13,15-hexahydro-1*H*,12*H*-benzo[*de*]pyrano[3',4':6,7]indolizino[1, 2-*b*]quinolin-1-yl)amino)-1,6,9,12,15-pentaoxo-3-oxa-5,8,11,14-tetraazahexadec-16-yl) -6-(2,5-dioxo-2,5-dihydro-1*H*-pyrrol-1-yl)hexanamide **9-B**

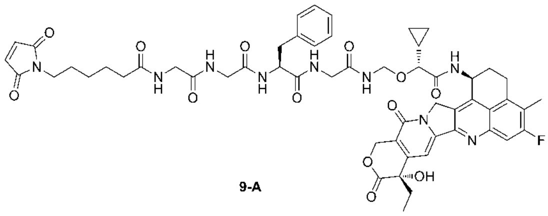

9-A

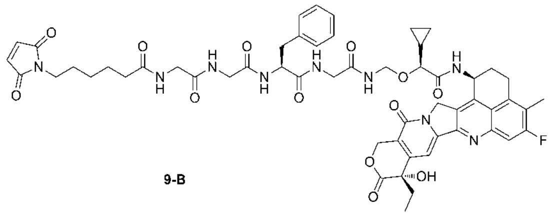

9-B

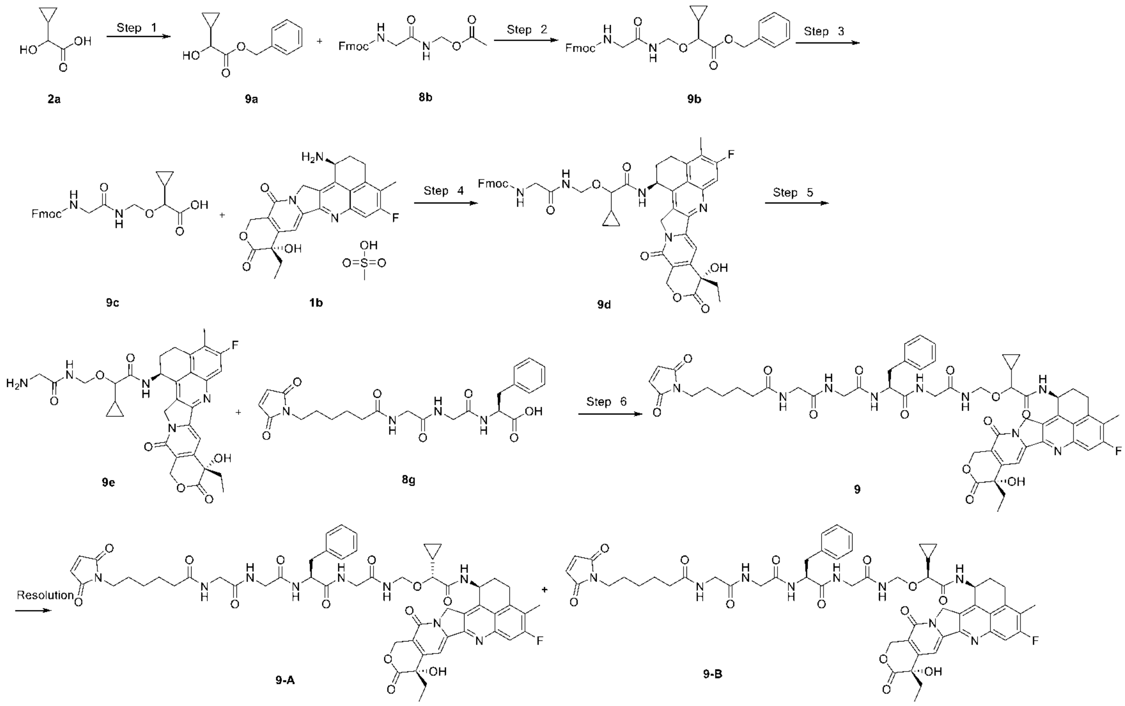


2a 9a 8b 9b 9c 1b 9d 9e 8g 9 9-A 9-B

Step 1

Benzyl 2-cyclopropyl-2-hydroxyacetate **9a**

**2a** (1.3 g, 11.2 mmol; prepared using the method disclosed in the patent application "WO2013/106717") was dissolved in 50 mL of acetonitrile, and potassium carbonate (6.18 g, 44.8 mmol), benzyl bromide (1.33 mL, 11.2 mmol), and tetrabutylammonium iodide (413 mg, 1.1 mmol) were sequentially added. The reaction mixture was stirred at room temperature for 48 h and filtered through celite, and the filter cake was rinsed with ethyl acetate (10 mL). The filtrates were combined and concentrated under reduced pressure, and the resulting residue was purified by silica gel column chromatography with developing solvent system C to give title product **9a** (2 g, yield: 86.9%).

Step 2

Benzyl

10-cyclopropyl-1-(9*H*-fluoren-9-yl)-3,6-dioxo-2,9-dioxa-4,7-diazaundecan-11-oate **9b** **9a** (120.9 mg, 0.586 mmol) and **8b** (180 mg, 0.489 mmol, prepared using the method disclosed in the patent application "CN105829346A") were added to a reaction flask, and 4 mL of tetrahydrofuran was added. The system was purged with argon gas three times. The mixture was cooled to 0-5 °C in an ice-water bath, and potassium tert-butoxide (109 mg, 0.98 mmol) was added. The ice bath was removed, and the mixture was warmed to room temperature and stirred for 40 min. 10 mL of ice water was added, and extraction was performed with ethyl acetate (20 mL × 2) and chloroform (10 mL × 5). The organic phases were combined and concentrated. The resulting residue was dissolved in 4 mL of dioxane. 2 mL of water was added, and sodium bicarbonate (49.2 mg, 0.586 mmol) and 9-fluorenylmethyl chloroformate (126 mg, 0.49 mmol) were added. The mixture was stirred at room temperature for 2 h. 20 mL of water was added, and extraction was performed with ethyl acetate (10 mL × 3). The organic phase was washed with a saturated sodium chloride solution (20 mL), dried over anhydrous sodium sulfate, and filtered. The filtrate was concentrated under reduced pressure. The resulting residue was purified by silica gel column chromatography with developing solvent system C to give title product

**9b** (48 mg, yield: 19%).
MS m/z (ESI): 515.0 [M+1].

Step 3

10-Cyclopropyl-1-(9*H*-fluoren-9-yl)-3,6-dioxo-2,9-dioxa-4,7-diazaundecan-11-oic acid **9c**

**9b** (20 mg, 0.038 mmol) was dissolved in 4.5 mL of a solvent mixture of tetrahydrofuran and ethyl acetate (V:V = 2:1), and palladium on carbon (12 mg, 10% loading, dry) was added. The system was purged with hydrogen gas three times, and the mixture was stirred at room temperature for 1 h. The reaction mixture was filtered through celite, and the filter cake was rinsed with ethyl acetate. The filtrate was concentrated to give crude title product **9c** (13 mg), and the product was directly used in the next step without purification.
MS m/z (ESI): 424.9 [M+1].

Step 4

(9*H*-Fluoren-9-yl)methyl(2-(((1-cyclopropyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indoli zino[1,2-*b*]quinolin-1-yl)amino)-2-oxoethoxy) methyl)amino)-2-oxoethyl)carbamate **9d** **1b** (10 mg, 18.8 μmol) was added to a reaction flask, and 1 mL of N,N-dimethylformamide was added. The system was purged with argon gas three times. The mixture was cooled to 0-5 °C in an ice-water bath, and a drop of triethylamine was added. Crude **9c** (13 mg, 30.6 μmol) was added, and 4-(4,6-dimethoxy-1,3,5-triazin-2-yl)-4-methylmorpholinium chloride (16.9 mg, 61.2 μmol) was added. The mixture was stirred in the ice bath for 40 min. 10 mL of water was added, and extraction was performed with ethyl acetate (10 mL × 3). The organic phases were combined, washed with a saturated sodium chloride solution (10 mL × 2), dried over anhydrous sodium sulfate, and filtered. The filtrate was concentrated under reduced pressure. The resulting residue was purified by thin-layer chromatography with developing solvent system B to give title product **9d** (19 mg, yield: 73.6%).
MS m/z (ESI): 842.1[M+1].

Step 5

2-((2-Aminoacetylamino)methoxy)-2-cyclopropyl-*N*-((1*S*,9*S*)-9-ethyl-5-fluoro-9-hydro xy-4-methyl-10,13-dioxo-2,3,9,10,13,15-hexahydro-1*H*,12*H*-benzo[de]pyrano[3',4':6,7] indolizino[1,2-*b*]quinolin-1-yl)acetamide **9e**

**9d** (19 mg, 22.6 μmol) was dissolved in 2 mL of dichloromethane, and 1 mL of diethylamine was added. The mixture was stirred at room temperature for 2 h. The reaction mixture was concentrated under reduced pressure. 1 mL of toluene was added, and the mixture was concentrated under reduced pressure; the procedures were repeated twice. The residue was triturated with 3 mL of n-hexane, and the triturate was left to stand. The supernatant was then removed, and the solid was kept. The solid residue was concentrated under reduced pressure and dried using an oil pump to give crude title product **9e** (17 mg), and the product was directly used in the next step without purification.
MS m/z (ESI): 638.0[M+18].

Step 6

*N*-((2*R*,10*S*)-10-Benzyl-2-cyclopropyl-1-(((1*S*,9*S*)-9-ethyl-5-fluoro-9-hydroxy-4-methyl -10,13-dioxo-2,3,9,10,13,15-hexahydro-1*H*,12*H*-benzo[de]pyrano[3',4':6,7]indolizino[1, 2-*b*]quinolin-1-yl)amino)-1,6,9,12,15-pentaoxo-3-oxa-5,8,11,14-tetraazahexadec-16-yl) -6-(2,5-dioxo-2,5-dihydro-1*H*-pyrrol-1-yl)hexanamide **9-A**

*N*-((2*S*,10*S*)-10-Benzyl-2-cyclopropyl-1-(((1*S*,9*S*)-9-ethyl-5-fluoro-9-hydroxy-4-methyl -10,13-dioxo-2,3,9,10,13,15-hexahydro-1*H*,12*H*-benzo[*de*]pyrano[3',4':6,7]indolizino[1, 2-*b*]quinolin-1-yl)amino)-1,6,9,12,15-pentaoxo-3-oxa-5,8,11,14-tetraazahexadec-16-yl) -6-(2,5-dioxo-2,5-dihydro-1*H*-pyrrol-1-yl)hexanamide **9-B**

Crude **9e** (13.9 mg, 22.4 μmol) was dissolved in 0.6 mL of *N*,*N*-dimethylformamide. The system was purged with argon gas three times, and the solution was cooled to 0-5 °C in an ice-water bath. A solution of **8g** (21.2 mg, 44.8 μmol, prepared using the method disclosed in the patent application "EP2907824") in 0.3 mL of *N*,*N*-dimethylformamide was added, and 4-(4,6-dimethoxy-1,3,5-triazin-2-yl)-4-methylmorpholinium chloride (18.5 mg, 67.3 μmol) was added. The mixture was stirred in an ice bath for 10 min. The ice bath was removed, and the reaction mixture was warmed to room temperature and stirred for 1 h. The reaction yielded compound **9.** The reaction mixture was purified by high performance liquid chromatography (separation conditions: chromatography column: XBridge Prep C18 OBD 5 μm 19 × 250 mm;

mobile phase: A-water (10 mmol of $NH_4OAc$), B-acetonitrile, gradient elution, flow rate: 18 mL/min). The corresponding fractions were collected and concentrated under reduced pressure to give the title products (9-A: 2.4 mg, 9-B: 1.7 mg).

MS m/z (ESI): 1074.4 [M+1].

Single-configuration compound 9-A (shorter retention time):

UPLC analysis: retention time: 1.14 min, purity: 85% (chromatography column: ACQUITY UPLC BEHC18 1.7 μm 2.1 × 50 mm, mobile phase: A-water (5 mmol $NH_4OAc$), B-acetonitrile).

$^1H$ NMR (400 MHz, DMSO-$d_6$): δ 8.60 (t, 1H), 8.51-8.49 (d, 1H), 8.32-8.24 (m, 1H), 8.13-8.02 (m, 2H), 8.02-7.96 (m, 1H), 7.82-7.75 (m, 1H), 7.31 (s, 1H), 7.26-7.15 (m, 4H), 6.99 (s, 1H), 6.55-6.48 (m, 1H), 5.65-5.54 (m, 1H), 5.41 (s, 2H), 5.35-5.15 (m, 3H), 4.74-4.62 (m, 1H), 4.54-4.40 (m, 2H), 3.76-3.64 (m, 4H), 3.62-3.48 (m, 2H), 3.20-3.07 (m, 2H), 3.04-2.94 (m, 1H), 2.80-2.62 (m, 1H), 2.45-2.30 (m, 3H), 2.25-2.15 (m, 2H), 2.15-2.04 (m, 2H), 1.93-1.78 (m, 2H), 1.52-1.39 (m, 3H), 1.34-1.12 (m, 5H), 0.87 (t, 3H), 0.64-0.38 (m, 4H).

Single-configuration compound 9-B (longer retention time):

UPLC analysis: retention time: 1.16 min, purity: 89% (chromatography column: ACQUITY UPLC BEHC18 1.7 μm 2.1 × 50 mm, mobile phase: A-water (5 mmol $NH_4OAc$), B-acetonitrile).

$^1H$ NMR (400 MHz, DMSO-$d_6$): δ 8.68-8.60 (m, 1H), 8.58-8.50 (m, 1H), 8.32-8.24 (m, 1H), 8.13-8.02 (m, 2H), 8.02-7.94 (m, 1H), 7.82-7.75 (m, 1H), 7.31 (s, 1H), 7.26-7.13 (m, 3H), 6.99 (s, 1H), 6.55-6.48 (m, 1H), 5.60-5.50 (m, 1H), 5.41 (s, 2H), 5.35-5.15 (m, 2H), 4.78-4.68 (m, 1H), 4.60-4.40 (m, 2H), 3.76-3.58 (m, 4H), 3.58-3.48 (m, 1H), 3.20-3.10 (m, 2H), 3.08-2.97 (m, 2H), 2.80-2.72 (m, 2H), 2.45-2.30 (m, 3H), 2.25-2.13 (m, 2H), 2.13-2.04 (m, 2H), 2.03-1.94 (m, 2H), 1.91-1.78 (m, 2H), 1.52-1.39 (m, 3H), 1.34-1.12 (m, 4H), 0.91-0.79 (m, 3H), 0.53-0.34 (m, 4H).

1.2. Preparation of compound L-1

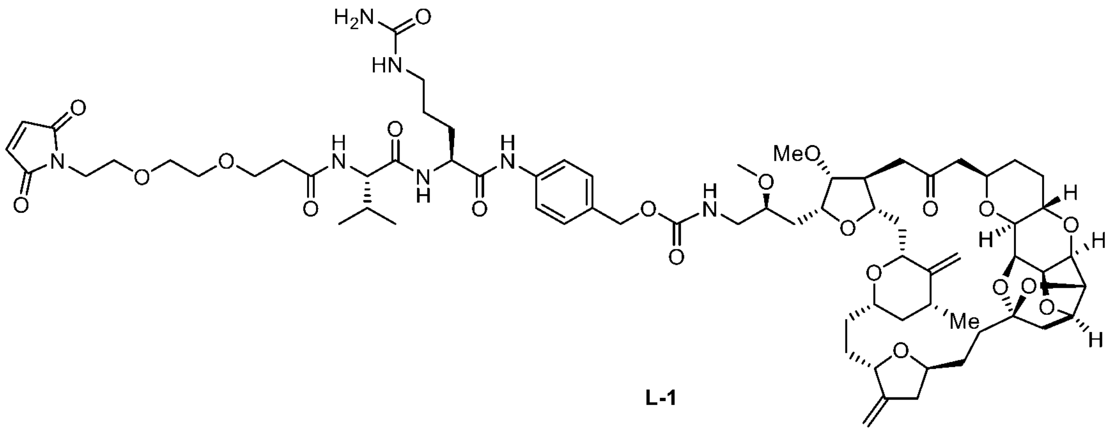

**L-1**

Compound L-1 was synthesized with reference to the method provided in "Example 1 on Page 28 of the specification of Patent No. CN117460540A".

1.3. Preparation of compound 1

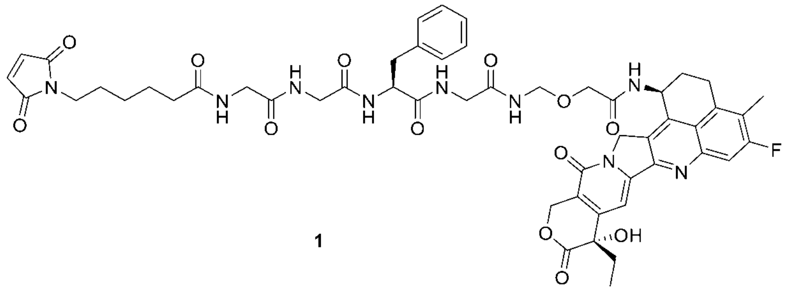

1

Compound 1 was synthesized with reference to the method provided in "Example 58 on page 163 of the specification of Patent No. CN104755494A".

2. Anti-HER3/MET antibody-drug conjugates

2.1. An antibody-drug conjugate (ADC) of the following structure was prepared, wherein the Ab in ADC-1 was 2232-01, the Ab in ADC-2 was 2232-02, and the Ab in ADC-5 was 2232-06. The target DAR was 6.

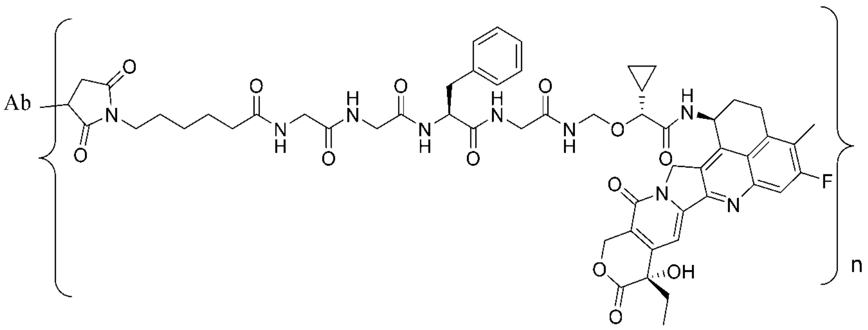

Preparation method:

ADC-1: An aqueous solution of tris(2-carboxyethyl)phosphine hydrochloride (TCEP·HCl) (10 mM, 35 μL, 350 nmol, 5.0 eq) was added to the antibody 2232-01 (5.0 mg/mL, 2.0 mL, 70 nmol) in an aqueous PBS solution (pH = 7.2). The mixture was shaken on a temperature-controlled shaker at 37 °C for 3 h. The reaction system was then cooled to 25 °C. A prepared solution of compound 9-A (1.13 mg, 1050 nmol, 15.0 eq) in dimethyl sulfoxide was added to the above reaction system. The mixture was shaken on a temperature-controlled shaker at 25 °C for 3 h. Finally, the reaction solution was subjected to desalting and buffer exchange (buffer: PBS pH 7.2, flow rate: 10 mL/min) using a desalting column (HiPrep 26/10, GE) to obtain ADC-1 in a PBS buffer (2.0 mg/mL, 4.7 mL) at a yield of 94%, which was refrigerated at 4 °C. The target DAR was 6, the drug loading was calculated by RP-HPLC, and the found DAR was 5.90. Based on the assay by denaturing mass spectrometry, as shown in FIG. 9B, the main peaks were LC1, LC2+1drug, HC1+2drug, and HC2+3drug.

ADC-2: 2232-02 was conjugated to the aforementioned compound 9-A with reference to the preparation method for ADC-1 to obtain ADC-2.

ADC-5: 2232-06 was conjugated to the aforementioned compound 9-A with reference to the preparation method for ADC-1 to obtain ADC-5. Based on the assay by native mass spectrometry, as shown in FIG. 9C, the average DAR value was 5.87, wherein DAR6 accounted for 94.9%, DAR2 and DAR4 accounted for 1.3% and 3.8%, respectively, and DAR8 was not detected. This indicates that the anti-HER3/MET antibody-drug conjugate (ADC) of this structure has a high proportion of DAR6, while other DAR values are low, exhibiting high homogeneity.

2.2. An antibody-drug conjugate (ADC) of the following structure was prepared, wherein the Ab moieties in ADC-6 and ADC-7 were 2232-06. The target DAR value of ADC-6 was 4, and the target DAR value of ADC-7 was 6.

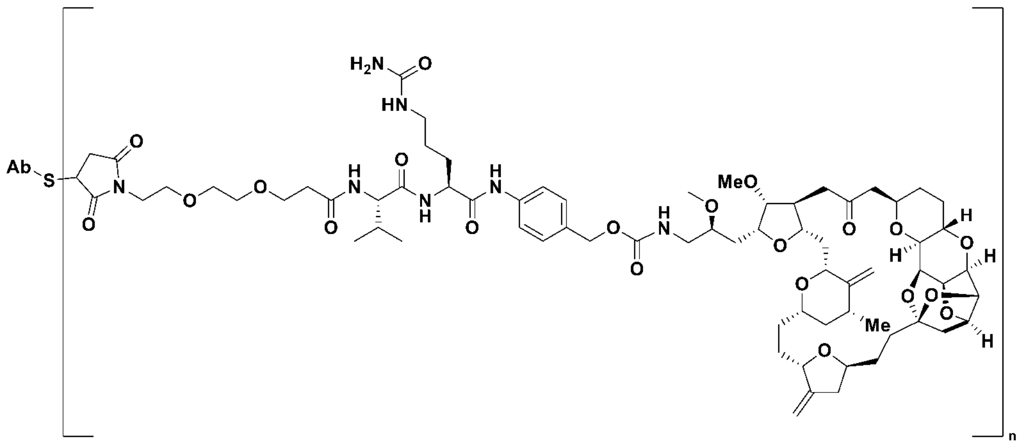

Preparation method:

ADC-6: An aqueous solution of tris(2-carboxyethyl)phosphine hydrochloride (TCEP·HCl) (10 mM, 17 μL, 173 nmol, 3.6 eq) was added to the antibody 2232-06 (7.0 mg/mL, 1.0 mL, 48 nmol) in an aqueous PBS solution (pH = 7.2). The mixture was shaken on a temperature-controlled shaker at 37 °C for 2 h. The reaction system was then cooled to 25 °C. A prepared solution of compound L-1 (0.53 mg, 384 nmol, 8.0 eq) in dimethyl sulfoxide was added to the above reaction system. The mixture was shaken on a temperature-controlled shaker at 25 °C for 2 h. Finally, the reaction solution was subjected to desalting and buffer exchange (buffer: PBS pH 7.2, flow rate: 10 mL/min) using a desalting column (HiPrep 26/10, GE) to obtain ADC-6 in a PBS buffer (3.44 mg/mL, 1.71 mL) at a yield of 84.1%, which was refrigerated at 4 °C. The target DAR was 4, the drug loading was calculated by mass spectrometry, and the found DAR was 3.90.

ADC-7: An aqueous solution of tris(2-carboxyethyl)phosphine hydrochloride (TCEP·HCl) (10 mM, 48 μL, 480 nmol, 10 eq) was added to the antibody 2232-06 (7.0 mg/mL, 1.0 mL, 48 nmol) in an aqueous PBS solution (pH = 7.2). The mixture was shaken on a temperature-controlled shaker at 37 °C for 2 h. The reaction system was then cooled to 25 °C. A prepared solution of compound L-1 (0.99 mg, 720 nmol, 15 eq) in dimethyl sulfoxide was added to the above reaction system. The mixture was shaken on a temperature-controlled shaker at 25 °C for 2 h. Finally, the reaction solution was subjected to desalting and buffer exchange (buffer: PBS pH 7.2, flow rate: 10 mL/min) using a desalting column (HiPrep 26/10, GE) to obtain ADC-7 in a PBS buffer (3.21 mg/mL, 1.83 mL) at a yield of 84.0%, which was refrigerated at 4 °C. The target DAR was 6, the drug loading was calculated by mass spectrometry, and the found DAR was 5.69.

2.3. An antibody-drug conjugate (ADC) of the following structure was prepared, wherein the Ab in the ADC was 2232-06, and the target DAR value was 6.

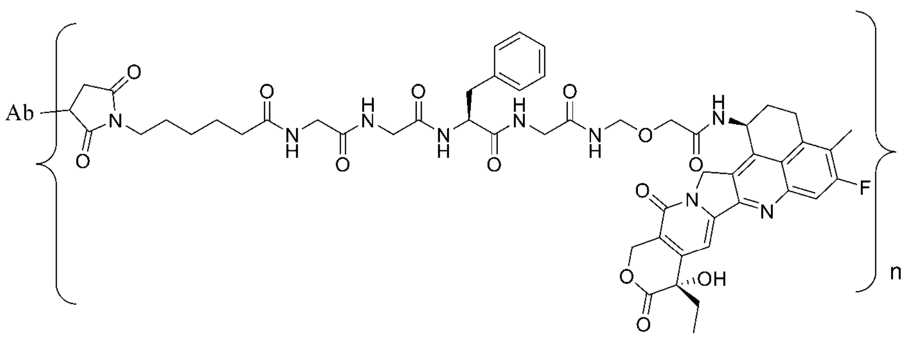

ADC-10: 2232-06 was conjugated to compound 1 with reference to the preparation method described in step 2.1 above to obtain the anti-HER3/MET antibody-drug conjugate ADC-10 with DAR6.

**Example 7. Preparation of Anti-HER3 ADC or Anti-MET ADC**

1) An antibody-drug conjugate (ADC) of the following structure was prepared, wherein the Ab in anti-MET ADC-3 was antibody S3, and the Ab in anti-HER3 ADC-4 was antibody A. The target DAR value was 4.

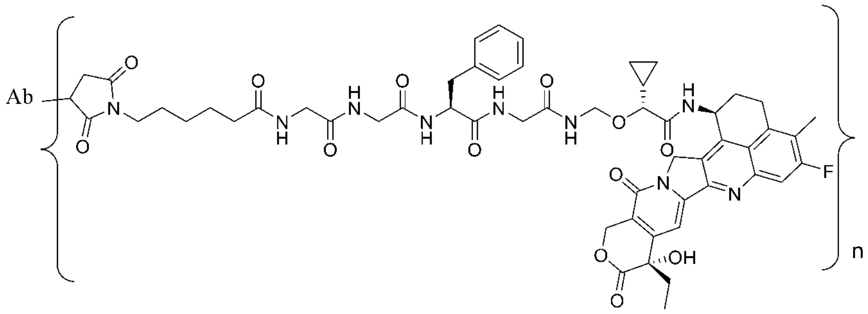

Preparation method:

Anti-MET ADC-3: An aqueous solution of tris(2-carboxyethyl)phosphine hydrochloride (TCEP·HCl) (10 mM, 17.5 μL, 175 nmol, 2.5 eq) was added to the antibody S3 (10.0 mg/mL, 1.0 mL, 70 nmol) in an aqueous PBS solution (pH = 7.2). The mixture was shaken on a temperature-controlled shaker at 37 °C for 3 h. The reaction system was then cooled to 25 °C. A solution of compound 9-A (0.60 mg, 560 nmol, 8.0 eq) in dimethyl sulfoxide was added to the above reaction system. The mixture was shaken on a temperature-controlled shaker at 25 °C for 3 h. Finally, the reaction solution was subjected to desalting and buffer exchange (buffer: PBS pH 7.2, flow rate: 10 mL/min) using a desalting column (HiPrep 26/10, GE) to obtain ADC-3 in a PBS buffer (2.0 mg/mL, 4.7 mL). The drug loading was calculated by RP-HPLC, and the found DAR was 4.7.

Anti-HER3 ADC-4: The antibody A was conjugated to the aforementioned compound 9-A with reference to the preparation method for ADC-3 to obtain ADC-4. The drug loading was calculated by RP-HPLC, and the found DAR was 4.0.

2) The ADC structure of U3-1402 is shown in the figure below:

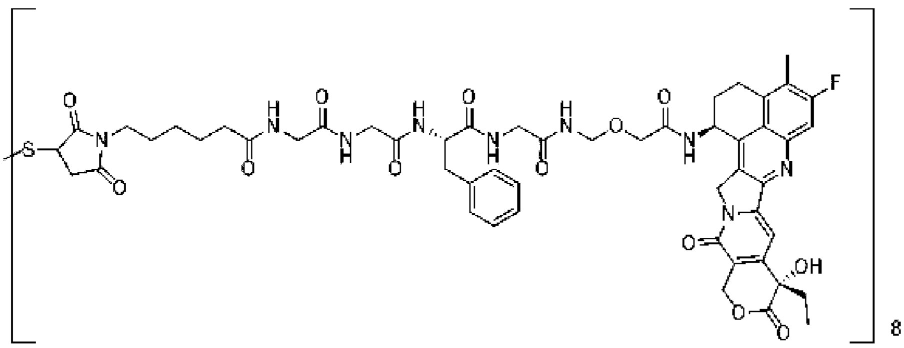
U3-1402 Ab

Preparation method for U3-1402:

An aqueous solution of tris(2-carboxyethyl)phosphine hydrochloride (TCEP·HCl) (10 mM, 828 μL, 8280 nmol, 15.0 eq) was added to the naked antibody of U3-1402 (U3-1402 Ab) (5.76 mg/mL, 552 nmol) in an aqueous PBS solution (pH = 7.2). The mixture was shaken on a temperature-controlled shaker at 37 °C for 2 h. The reaction system was then cooled to 25 °C. A solution of compound 1 (5.7 mg, 5520 nmol, 10.0 eq) in dimethyl sulfoxide was added to the above reaction system. The mixture was shaken on a temperature-controlled shaker at 25 °C for 2 h. Finally, the reaction solution was subjected to desalting and buffer exchange (buffer: PBS pH 7.2, flow rate: 10 mL/min) using a desalting column (HiPrep 26/10, GE) to obtain U3-1402 in a PBS buffer (4.18 mg/mL, 18 mL) at a yield of 94%, which was refrigerated at 4 °C. The drug loading was calculated by mass spectrometry to obtain DAR = 7.88.

**Example 8. Assay for Binding of Anti-HER3/MET Antibody-Drug Conjugates to Antigen**

1. Assay by flow cytometer (FACS)

The binding activity of anti-HER3/MET bispecific antibodies and ADCs thereof to human HER3/MET proteins on the cell surface was assayed by FACS.

Experimental method: The HCC827 Osimertinib-resistant cell strain was used and washed twice with the experimental culture medium and seeded into a 96-well U-bottom plate at $1 \times 10^5$ cells/well, and test samples at different

concentrations were added. The cells were incubated at 4 °C for 1 h and then washed twice with the experimental culture medium. Subsequently, the Alexa Fluor 647-mouse anti-human (IgG, Fcγ fragment specific) antibody (Jackson, Cat# 209-605-098) was added. After two washes, the fluorescence signal values were measured using a flow cytometer.

Experimental results: The FACS assay results in FIG. 10 show that all the anti-HER3/MET bispecific antibodies and conjugates thereof could bind to the HER3/MET antigen on the cell surface, and the binding abilities before and after conjugation were equivalent (overlapping of dashed line and solid line in FIG. 10). This indicates that toxin conjugation does not affect target binding of the antibody.

2. Assay by surface plasmon resonance (SPR)

The SPR assay was performed using a BIAcore 8K (Cityva) system. The sensor chip protein A and related reagents for assay were purchased from Cytiva.

Experimental method: ADC-1, ADC-2, and ADC-5 were diluted to 1 μg/mL with the HBS-EP+ buffer at a flow rate of 10 μL/min, and the antibodies were captured to a level of 200 RU. A His-tagged HER3/MET antigen was diluted with the HBS-EP+ buffer at a certain ratio, with concentration gradients of 6.25 nM, 12.5 nM, 25 nM, 50 nM, 100 nM, 200 nM, and 400 nM. The flow rate was set to 30 μL/min during sample analysis. The association time was 120 s, and the dissociation time was 900 s. Subsequently, regeneration was performed for 30 s. A Gly-HCl buffer at pH 1.5 was used as the regeneration buffer, and the regeneration flow rate was set to 30 μL/min. Their response signals were plotted with analysis time on the abscissa and response value on the ordinate. The data obtained were fitted by the BIAcore 8K analysis software, and their kinetic constants, such as the association rate constant ($K_a$), dissociation rate constant ($K_d$), and dissociation equilibrium constant ($K_D$), were determined using a 1:1 Langmuir binding model.

Experimental results: Table 8 shows that the anti-HER3/MET antibody-drug conjugates could bind to the human HER3 antigen and the human MET antigen.

Table 8-1. Binding kinetic parameters of anti-HER3/MET antibody-drug conjugates

| No. | Human HER3 antigen | | | Human MET antigen | | |
|---|---|---|---|---|---|---|
| | $K_a$ (1/Ms) | $K_d$ (1/s) | $K_D$ (M) | $K_a$ (1/Ms) | $K_d$ (1/s) | $K_D$ (M) |
| ADC-1 | 5.94E+05 | 1.13E-02 | 1.90E-08 | 6.54E+04 | 9.22E-04 | 1.41E-08 |
| ADC-2 | 5.46E+05 | 1.08E-02 | 1.97E-08 | 2.88E+04 | 1.10E-03 | 3.80E-08 |

Table 8-2. Binding kinetic parameters of anti-HER3/MET antibody-drug conjugate

| No. | Human HER3 antigen | | | Human MET antigen | | |
|---|---|---|---|---|---|---|
| | $K_a$ (1/Ms) | $K_d$ (1/s) | $K_D$ (M) | $K_a$ (1/Ms) | $K_d$ (1/s) | $K_D$ (M) |
| ADC-5 | 5.05E+05 | 7.21E-03 | 1.43E-08 | 6.65E+04 | 8.72E-04 | 1.31E-08 |

## Example 9. Inhibition of Tumor Cell Proliferation by Anti-HER3/MET Antibody-Drug Conjugates *in Vitro*

Experimental method: Double-positive cells NCI-H441 and NCI-H226, and PC-9 Osimertinib-resistant cell strain (constructed in-house, obtained by subjecting PC-9 cells to gradient resistance induction with Osimertinib) were selected. The cell culture medium was RPMI 1640 culture medium (ATCC modification) (Gibco, Cat# A1049101) containing 10% fetal bovine serum. The cells were grown overnight in 96-well culture plates at 600 cells/well. The next day, serially diluted ADCs at an equal volume were added. The cell viability was determined after 6 days using the CellTiter-Glo luminescent cell viability assay kit (Promega, Cat# G7570) as described in the package insert. The cell viability was evaluated as a percentage of control untreated cells. Experimental results: It can be seen from the FACS results in FIG. 11A that the HER3 expression levels were similar in the 3 cell lines, NCI-H441, NCI-H226, and PC-9 Osimertinib-resistant cell lines, the expression level of MET in NCI-H441 was the highest, and the expression level of MET in PC-9 Osimertinib-resistant cell strain was the lowest. It can be seen from the ADC killing results in FIGs. 11B and 11C that the killing ability of ADC-4 in the 3 cell lines was similar, which is consistent with the expression level of HER3. ADC-1 has the strongest ability to kill NCI-H441 and the weakest ability to kill the PC-9 Osimertinib-resistant cell strain, which was consistent with the expression level of MET, indicating that the MET arm helps to enhance the killing effect of the anti-HER3/MET antibody-drug conjugate.

## Example 10. Evaluation of Anti-HER3/MET antibody-drug conjugates in Mouse Anti-Tumor Model

The calculation formula used in the present disclosure is as follows: The tumor volume (TV) was calculated using

the formula TV = 1/2 × a × $b_2$, where a and b represent the long and short diameters of the measured tumor, respectively.

Relative tumor proliferation rate T/C% = (T - T0) / (C - C0) × 100%; tumor growth inhibition rate TGI% = 1 - T/C%.

Experimental method: A mouse xenograft model of HCC827 Osimertinib-resistant cell strain (constructed in-house, obtained by subjecting HCC827 cells to gradient resistance induction with Osimertinib) was used to evaluate the anti-tumor activity. HCC827 Osimertinib-resistant cells were inoculated subcutaneously into NCG mice (provided by GemPharmatech Co., Ltd.) at 1 × $10^7$ cells/100 μL/mouse. When the tumors grew to about 120 $mm^3$, the mice were randomly grouped based on the tumor volume and were administered an anti-HER3/MET antibody-drug conjugate. During the administration and observation periods, the tumor volume was measured twice weekly, and the measurements were recorded.

Experimental results: As shown in FIG. 12A, the body weight of the experimental animals did not decrease significantly during the high- and low-dose administration, indicating that the test drug does not cause significant toxic and side effects in the experimental animals, and has good tolerance and safety to the toxin. As shown in FIG. 12B and Table 9, the high dose of the bispecific antibody ADC exhibited good anti-tumor activity, and at the same dose, the anti-tumor activity of ADC-5 was superior to those of ADC-3 and ADC-4. The anti-tumor activity of ADC-5 was also superior to those of ADC-3 and ADC-4 under the same toxin loading condition.

Table 9. Tumor inhibitory effects of different HER3×MET bispecific antibody ADCs on mouse xenograft tumor

| Group | Administration regimen | Toxin loading | Dose (mg/kg) | TGI (%) | P value (v.s. vehicle) | P value (v.s. ADC-3) | P value (v.s. ADC-4) |
|---|---|---|---|---|---|---|---|
| G1 | Vehicle | - | - | - | - | - | - |
| G2 | ADC-5 | 6 | 10 | 108 | **** | **** | **** |
| G3 | ADC-5 | 6 | 3 | 78 | **** | ** | ** |
| G4 | ADC-5 | 6 | 2 | 66 | **** | ns | * |
| G5 | ADC-3 | 4.7 | 3 | 31 | ns | - | - |
| G6 | ADC-4 | 4 | 3 | 21 | ns | - | - |

**Example 11. Evaluation of Anti-HER3×MET Antibody-Drug Conjugates in Mouse HER3 Single-Positive Anti-Tumor Model**

The anti-tumor activity was evaluated using a mouse xenograft model of MDA-MB-453. MDA-MB-453 cells were inoculated subcutaneously at 5 × $10^6$ cells/100 μL/mouse into Nude mice (provided by Shanghai Bikai Keyi Biotechnology Co., Ltd.). When the tumors grew to about 120 $mm^3$, 42 mice were selected according to the tumor volume and randomized into 7 groups of 6, which were the vehicle, ADC-1 (10 mg/kg), ADC-1 (3 mg/kg), ADC-2 (10 mg/kg), and ADC-2 (3 mg/kg), respectively. The mice were administered once a week, and 2 administrations were performed. During the administration and observation periods, the tumor volume was measured twice weekly, and the measurements were recorded.

The tumor volume (TV) was calculated using the formula TV = 1/2 × a × $b_2$, where a and b represent the long and short diameters of the measured tumor, respectively.

Relative tumor proliferation rate T/C% = (T - T0) / (C - C0) × 100%; tumor growth inhibition rate TGI% = 1 - T/C%.

CR% (complete tumor regression proportion) = the number of mice with complete tumor regression (<150 $mm^3$) / the number of mice in the group.

As shown in FIG. 13A, the body weight of the experimental animals did not decrease significantly during the high- and low-dose administration, indicating that the test drug does not cause significant toxic and side effects in the experimental animals, and has good tolerance and safety to the toxin. As shown in FIG. 13B and Table 10, the high dose of the bispecific antibody ADC exhibited good anti-tumor activity.

Table 10. Anti-tumor effects of different HER3×MET bispecific antibody ADCs on mouse xenograft tumor

| Group | Administration regimen | Toxin loading | Dose (mg/kg) | TGI (%) | P value |
|---|---|---|---|---|---|
| G1 | Vehicle | - | - | - | - |
| G2 | ADC-1 | 6 | 10 | 90.6 | **** |
| G3 | ADC-1 | 6 | 3 | 69.7 | *** |

(continued)

| Group | Administration regimen | Toxin loading | Dose (mg/kg) | TGI (%) | P value |
|---|---|---|---|---|---|
| G4 | ADC-2 | 6 | 10 | 84.8 | **** |
| G5 | ADC-2 | 6 | 3 | 65.8 | *** |

**Example 12. *In Vitro* Inhibition of Tumor Cell Proliferation by Anti-HER3/MET Antibody-Drug Conjugate ADC-6**

Experimental method: The cell culture medium for NCI-H441 double-positive cells was RPMI 1640 culture medium (ATCC modification) (Gibco, Cat# A1049101) containing 10% fetal bovine serum. The cells were grown overnight in 96-well culture plates at 600 cells/well. The next day, serially diluted ADCs at an equal volume were added. The cell viability was determined after 6 days using the CellTiter-Glo luminescent cell viability assay kit (Promega, Cat# G7570) as described in the package insert. The cell viability was evaluated as a percentage of control untreated cells.

As shown in FIG. 14, ADC-6 and ADC-7 had killing activity on double-positive cells.

## Claims

**1.** A HER3/MET-binding molecule, comprising a first binding domain that specifically binds to MET and a second binding domain that specifically binds to HER3, wherein

the first binding domain that specifically binds to MET comprises a heavy chain variable region (VH1) and a light chain variable region (VL1), wherein the VH1 comprises a HCDR1, a HCDR2, and a HCDR3 from the amino acid sequence set forth in SEQ ID NO: 71 or 1; and the VL1 comprises a LCDR1, a LCDR2, and a LCDR3 from the amino acid sequence set forth in SEQ ID NO: 72 or 2; and/or
the second binding domain that specifically binds to HER3 comprises a heavy chain variable region (VH2) and a light chain variable region (VL2), wherein the VH2 comprises a HCDR1, a HCDR2, and a HCDR3 from the amino acid sequence set forth in SEQ ID NO: 13, and the VL2 comprises a LCDR1, a LCDR2, and a LCDR3 from the amino acid sequence set forth in SEQ ID NO: 70 or 14;
the CDRs are defined according to the Kabat, IMGT, Chothia, AbM, or Contact numbering scheme.

**2.** A HER3/MET-binding molecule, comprising a first binding domain that specifically binds to MET and a second binding domain that specifically binds to HER3, wherein

the first binding domain that specifically binds to MET comprises a heavy chain variable region (VH1) and a light chain variable region (VL1), wherein the VH1 comprises a HCDR1, a HCDR2, and a HCDR3 set forth in SEQ ID NOs: 15-17, respectively, and the VL1 comprises a LCDR1, a LCDR2, and a LCDR3 set forth in SEQ ID NOs: 76, 19, and 20, respectively; and/or
the second binding domain that specifically binds to HER3 comprises a heavy chain variable region (VH2) and a light chain variable region (VL2), wherein the VH2 comprises a HCDR1, a HCDR2, and a HCDR3 set forth in SEQ ID NOs: 51-53, respectively, and the VL2 comprises a LCDR1, a LCDR2, and a LCDR3 set forth in SEQ ID NOs: 54, 55, and 75, respectively;
preferably,
the first binding domain that specifically binds to MET comprises a heavy chain variable region (VH1) and a light chain variable region (VL1), wherein the VH1 comprises a HCDR1, a HCDR2, and a HCDR3 set forth in SEQ ID NOs: 15-17, respectively, and the VL1 comprises a LCDR1 set forth in SEQ ID NO: 18 or 74, and a LCDR2 and a LCDR3 set forth in SEQ ID NOs: 19 and 20, respectively; and/or
the second binding domain that specifically binds to HER3 comprises a heavy chain variable region (VH2) and a light chain variable region (VL2), wherein the VH2 comprises a HCDR1, a HCDR2, and a HCDR3 set forth in SEQ ID NOs: 51-53, respectively, and the VL2 comprises a LCDR1 and a LCDR2 set forth in SEQ ID NOs: 54 and 55, respectively, and a LCDR3 set forth in SEQ ID NO: 56 or 73;
more preferably,
the first binding domain that specifically binds to MET comprises a heavy chain variable region (VH1) and a light chain variable region (VL1), wherein the VH1 comprises a HCDR1, a HCDR2, and a HCDR3 set forth in SEQ ID NOs: 15-17, respectively, and the VL1 comprises a LCDR1, a LCDR2, and a LCDR3 set forth in SEQ ID NOs: 18-20, respectively; and
the second binding domain that specifically binds to HER3 comprises a heavy chain variable region (VH2) and a

light chain variable region (VL2), wherein the VH2 comprises a HCDR1, a HCDR2, and a HCDR3 set forth in SEQ ID NOs: 51-53, respectively, and the VL2 comprises a LCDR1, a LCDR2, and a LCDR3 set forth in SEQ ID NOs: 54-56, respectively; or

the first binding domain that specifically binds to MET comprises a heavy chain variable region (VH1) and a light chain variable region (VL1), wherein the VH1 comprises a HCDR1, a HCDR2, and a HCDR3 set forth in SEQ ID NOs: 15-17, respectively, and the VL1 comprises a LCDR1, a LCDR2, and a LCDR3 set forth in SEQ ID NOs: 74, 19, and 20, respectively; and

the second binding domain that specifically binds to HER3 comprises a heavy chain variable region (VH2) and a light chain variable region (VL2), wherein the VH2 comprises a HCDR1, a HCDR2, and a HCDR3 set forth in SEQ ID NOs: 51-53, respectively, and the VL2 comprises a LCDR1, a LCDR2, and a LCDR3 set forth in SEQ ID NOs: 54, 55, and 73, respectively.

**3.** The HER3/MET-binding molecule according to claim 1 or 2, wherein the VH1 of the first binding domain that specifically binds to MET comprises a K mutation at position 23 and/or a T mutation at position 78, naturally numbered relative to SEQ ID NO: 1; and/or the VL1 of the first binding domain that specifically binds to MET comprises a T mutation at position 69 naturally numbered relative to SEQ ID NO: 2;
preferably, the first binding domain that specifically binds to MET comprises 23K and 78T mutations naturally numbered relative to SEQ ID NO: 1, and a 69T mutation naturally numbered relative to SEQ ID NO: 2.

**4.** The HER3/MET-binding molecule according to any one of claims 1 to 3, wherein

the first binding domain that specifically binds to MET comprises a heavy chain variable region (VH1) and a light chain variable region (VL1), wherein the VH1 comprises the amino acid sequence set forth in SEQ ID NO: 71 or 1 or an amino acid sequence having at least 90% identity thereto, and the VL1 comprises the amino acid sequence set forth in SEQ ID NO: 72 or 2 or an amino acid sequence having at least 90% identity thereto; and/or

the second binding domain that specifically binds to HER3 comprises a heavy chain variable region (VH2) and a light chain variable region (VL2), wherein the VH2 comprises the amino acid sequence set forth in SEQ ID NO: 13 or an amino acid sequence having at least 90% identity thereto, and the VL2 comprises the amino acid sequence set forth in SEQ ID NO: 70 or 14 or an amino acid sequence having at least 90% identity thereto;

preferably,

the first binding domain that specifically binds to MET comprises a heavy chain variable region (VH1) and a light chain variable region (VL1), wherein the VH1 and the VL1 comprise the amino acid sequences set forth in SEQ ID NO: 71 and SEQ ID NO: 72, respectively; and the second binding domain that specifically binds to HER3 comprises a heavy chain variable region (VH2) and a light chain variable region (VL2), wherein the VH2 and the VL2 comprise the amino acid sequences set forth in SEQ ID NO: 13 and SEQ ID NO: 70, respectively; or

the first binding domain that specifically binds to MET comprises a heavy chain variable region (VH1) and a light chain variable region (VL1), wherein the VH1 and the VL1 comprise the amino acid sequences set forth in SEQ ID NO: 1 and SEQ ID NO: 2, respectively; and the second binding domain that specifically binds to HER3 comprises a heavy chain variable region (VH2) and a light chain variable region (VL2), wherein the VH2 and the VL2 comprise the amino acid sequences set forth in SEQ ID NO: 13 and SEQ ID NO: 14, respectively;

more preferably, the HER3/MET-binding molecule is an anti-HER3/MET antibody.

**5.** The HER3/MET-binding molecule according to any one of claims 1 to 4, further comprising a human immunoglobulin Fc region, wherein
preferably, the Fc region is an Fc region of IgG1, IgG2, IgG3, or IgG4.

**6.** The HER3/MET-binding molecule according to claim 5, wherein

the Fc region comprises a first subunit and a second subunit, and the first subunit and the second subunit have a knob-into-hole structure;

preferably, the first subunit of the Fc region is a knob chain, and the second subunit of the Fc region is a hole chain;

preferably,

the first subunit of the Fc region comprises a mutation at position 366, and the second subunit comprises a mutation at a position selected from the group consisting of 366, 368, and 407, or any combination thereof;

the first subunit of the Fc region comprises a mutation at position 354 or 356, and the second subunit comprises a mutation at position 349; or

the first subunit of the Fc region comprises a mutation at position 354 or 356, and the second subunit comprises mutations at positions 349, 366, 368, and 407;

more preferably,
the first subunit of the Fc region comprises a 366W mutation, and the second subunit comprises a mutation selected from the group consisting of 366S, 368A, and 407V, or any combination thereof;
the first subunit of the Fc region comprises a 354C or 356C mutation, and the second subunit comprises a 349C mutation; or
the first subunit of the Fc region comprises 354C/366W mutations, and the second subunit comprises 349C/366S/368A/407V mutations, wherein the mutations are defined according to the Eu numbering.

**7.** The HER3/MET-binding molecule according to any one of claims 1 to 6, further comprising a linker, wherein preferably, the linker is represented by $(G_mS_n)_h$, $(G_mQ_n)_h$, $(GGNGT)_h$, $(YGNGT)_h$, or $(EPKSS)_h$, wherein m and n are each independently selected from the group consisting of integers from 1 to 8, and h is independently selected from the group consisting of integers from 1 to 20.

**8.** The HER3/MET-binding molecule according to any one of claims 1 to 7, comprising:

a first heavy chain that is, from the N-terminus to the C-terminus, [VH1]-[linker 1]-[Obscurin-O chain]-[linker 3]-[first subunit of Fc region],
a first light chain that is, from the N-terminus to the C-terminus, [VL1]-[linker 2]-[Titin-T chain],
a second heavy chain that is, from the N-terminus to the C-terminus, [VH2]-[CH1]-[second subunit of Fc region], and
a second light chain that is, from the N-terminus to the C-terminus, [VL2]-[CL],
wherein - represents a peptide bond,
the amino acid sequence of the Obscurin-O chain is set forth in SEQ ID NO: 63,
the amino acid sequence of the Titin-T chain is set forth in SEQ ID NO: 64,
the linker 1, the linker 2, and the linker 3 may be identical or different, and may be independently present or absent; preferably, the amino acid sequences of the linker 1 and the linker 2 are GGGGS (SEQ ID NO: 91), and the linker 3 is absent.

**9.** The HER3/MET-binding molecule according to any one of claims 1 to 8, comprising the following polypeptide chain combinations:

a first heavy chain comprising the amino acid sequence set forth in SEQ ID NO: 77 or 57 or an amino acid sequence having at least 90% sequence identity thereto,
a first light chain comprising the amino acid sequence set forth in SEQ ID NO: 78 or 58 or an amino acid sequence having at least 90% sequence identity thereto,
a second heavy chain comprising the amino acid sequence set forth in SEQ ID NO: 59 or an amino acid sequence having at least 90% sequence identity thereto, and
a second light chain comprising the amino acid sequence set forth in SEQ ID NO: 79 or 60 or an amino acid sequence having at least 90% sequence identity thereto,
wherein preferably,
the HER3/MET-binding molecule comprises:

a first heavy chain set forth in SEQ ID NO: 77,
a first light chain set forth in SEQ ID NO: 78,
a second heavy chain set forth in SEQ ID NO: 59, and
a second light chain set forth in SEQ ID NO: 79; or
the HER3/MET-binding molecule comprises:

a first heavy chain set forth in SEQ ID NO: 57,
a first light chain set forth in SEQ ID NO: 58,
a second heavy chain set forth in SEQ ID NO: 59, and
a second light chain set forth in SEQ ID NO: 60.

**10.** An antibody-drug conjugate, comprising an antibody and an effector, wherein the antibody comprises a first binding domain that specifically binds to MET and a second binding domain that specifically binds to HER3;

a heavy chain variable region in the first binding domain that specifically binds to MET comprises a HCDR1, a HCDR2, and a HCDR3 of the amino acid sequences set forth in SEQ ID NOs: 15-17, and a light chain variable

region comprises a LCDR1, a LCDR2, and a LCDR3 of the amino acid sequences set forth in SEQ ID NOs: 76, 19, and 20; and a heavy chain variable region in the second binding domain that specifically binds to HER3 comprises a HCDR1, a HCDR2, and a HCDR3 of the amino acid sequences set forth in SEQ ID NOs: 51-53, and a light chain variable region comprises a LCDR1, a LCDR2, and a LCDR3 of the amino acid sequences set forth in SEQ ID NOs: 54, 55, and 75;

preferably, the heavy chain variable region in the first binding domain that specifically binds to MET comprises a HCDR1, a HCDR2, and a HCDR3 of the amino acid sequences set forth in SEQ ID NOs: 15-17, and the light chain variable region comprises a LCDR1 of the amino acid sequence set forth in SEQ ID NO: 18 or 74, and a LCDR2 and a LCDR3 of the amino acid sequences set forth in SEQ ID NOs: 19 and 20; and the heavy chain variable region in the second binding domain that specifically binds to HER3 comprises a HCDR1, a HCDR2, and a HCDR3 of the amino acid sequences set forth in SEQ ID NOs: 51-53, and the light chain variable region comprises a LCDR1 and a LCDR2 of the amino acid sequences set forth in SEQ ID NOs: 54 and 55, and a LCDR3 of the amino acid sequence set forth in SEQ ID NO: 56 or 73;

more preferably, the heavy chain variable region in the first binding domain that specifically binds to MET comprises the amino acid sequence set forth in SEQ ID NO: 71 or 1 or an amino acid sequence having at least 90% identity thereto, and the light chain variable region comprises the amino acid sequence set forth in SEQ ID NO: 72 or 2 or an amino acid sequence having at least 90% identity thereto; and the heavy chain variable region in the second binding domain that specifically binds to HER3 comprises the amino acid sequence set forth in SEQ ID NO: 13 or an amino acid sequence having at least 90% identity thereto, and the light chain variable region comprises the amino acid sequence set forth in SEQ ID NO: 70 or 14 or an amino acid sequence having at least 90% identity thereto;

more preferably, the antibody comprises:

a first heavy chain set forth in SEQ ID NO: 77,
a first light chain set forth in SEQ ID NO: 78,
a second heavy chain set forth in SEQ ID NO: 59, and
a second light chain set forth in SEQ ID NO: 79; or
the antibody comprises:

a first heavy chain set forth in SEQ ID NO: 57,
a first light chain set forth in SEQ ID NO: 58,
a second heavy chain set forth in SEQ ID NO: 59, and
a second light chain set forth in SEQ ID NO: 60.

**11.** The antibody-drug conjugate drug according to claim 10, wherein the effector is a cytotoxin;

preferably, the cytotoxin is selected from the group consisting of: a tubulin polymerization inhibitor, a Topo I inhibitor, and MMAE or a derivative thereof;

more preferably, the cytotoxin is selected from the group consisting of MMAE or a derivative thereof, exatecan or a derivative thereof, and eribulin or a derivative thereof.

**12.** The antibody-drug conjugate according to claim 10, having a structure represented by formula (I):

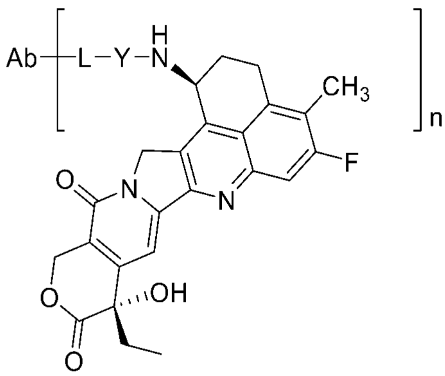

(I),

wherein:

-L- is a linker unit that is $-L^1-L^2-L^3-L^4-$;

$L^1$ is -(succinimid-3-yl-*N*)-W-C(O)-, $-CH_2-C(O)-NR^3-W-C(O)-$, or -C(O)-W-C(O)-, wherein W is selected from the group consisting of $C_{1-8}$ alkyl, $C_{1-8}$ alkyl-cycloalkyl, and linear heteroalkyl of 1 to 8 atoms, and the heteroalkyl comprises 1 to 3 heteroatoms selected from the group consisting of N, O, and S, wherein the $C_{1-8}$ alkyl, cycloalkyl, and linear heteroalkyl are each independently and optionally further substituted with one or more substituents selected from the group consisting of halogen, hydroxy, cyano, amino, alkyl, chloroalkyl, deuterated alkyl, alkoxy, and cycloalkyl;

$L^2$ is selected from the group consisting of $-NR^4(CH_2CH_2O)p^1CH_2CH_2C(O)-$, $-NR^4(CH_2CH_2O)_p{}^1CH_2C(O)-$, $-S(CH_2)_p{}^1C(O)-$, and a chemical bond, wherein $p^1$ is an integer from 1 to 20;

$L^3$ is a peptide residue consisting of 2 to 7 amino acids, wherein the amino acids are optionally further substituted with one or more substituents selected from the group consisting of halogen, hydroxy, cyano, amino, alkyl, chloroalkyl, deuterated alkyl, alkoxy, and cycloalkyl;

$L^4$ is selected from the group consisting of $-NR^5(CR^6R^7)_t-$, $-C(O)NR^5$, $-C(O)NR^5(CH_2)_t-$, and a chemical bond, wherein t is an integer from 1 to 6;

$R^3$, $R^4$, and $R^5$ are identical or different and are each independently selected from the group consisting of hydrogen, alkyl, haloalkyl, deuterated alkyl, and hydroxyalkyl;

$R^6$ and $R^7$ are identical or different and are each independently selected from the group consisting of hydrogen, halogen, alkyl, haloalkyl, deuterated alkyl, and hydroxyalkyl;

Y is selected from the group consisting of $-O-(CR^aR^b)_m-CR^1R^2-C(O)-$, $-O-CR^1R^2-(CR^aR\ )_m-$ , $-O-CR^1R^2-$, $-NH-(CR^aR^b)_m-CR^1R^2-C(O)-$, and $-S-(CR^aR^b)_m-CR^1R^2-C(O)-$;

$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl, and heterocyclyl; or $R^a$ and $R^b$, together with the carbon atom to which they are attached, form cycloalkyl or heterocyclyl;

$R^1$ is selected from the group consisting of hydrogen, halogen, haloalkyl, deuterated alkyl, cycloalkyl, cycloalkylalkyl, alkoxyalkyl, heterocyclyl, aryl, and heteroaryl;

$R^2$ is selected from the group consisting of hydrogen, halogen, haloalkyl, deuterated alkyl, cycloalkyl, cycloalkylalkyl, alkoxyalkyl, heterocyclyl, aryl, and heteroaryl;

or $R^1$ and $R^2$, together with the carbon atom to which they are attached, form cycloalkyl or heterocyclyl;

or $R^a$ and $R^2$, together with the carbon atom to which they are attached, form cycloalkyl or heterocyclyl;

m is an integer from 0 to 4;

n is 1 to 10, and n is an integer or a decimal;

Ab is the antibody as defined in claim 10.

**13.** The antibody-drug conjugate according to any one of claims 10 to 12, having a structure represented by formula II:

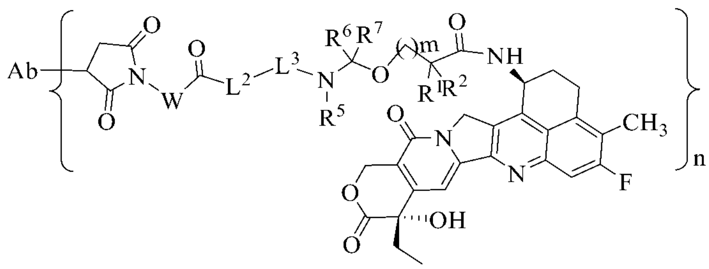

(II),

wherein:

W is selected from the group consisting of $C_{1-8}$ alkyl, $C_{1-8}$ alkyl-cycloalkyl, and linear heteroalkyl of 1 to 8 atoms, and the heteroalkyl comprises 1 to 3 heteroatoms selected from the group consisting of N, O, and S, wherein the $C_{1-8}$ alkyl, cycloalkyl, and linear heteroalkyl are each independently and optionally further substituted with one or more substituents selected from the group consisting of halogen, hydroxy, cyano, amino, alkyl, chloroalkyl, deuterated alkyl, alkoxy, and cycloalkyl;

$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$, and a chemical bond, and $p^1$ is an integer from 1 to 20;

$L^3$ is a peptide residue consisting of 2 to 7 amino acids, wherein the amino acids are optionally further substituted with one or more substituents selected from the group consisting of halogen, hydroxy, cyano, amino, alkyl, chloroalkyl, deuterated alkyl, alkoxy, and cycloalkyl;

$R^1$ is selected from the group consisting of hydrogen, halogen, cycloalkylalkyl, deuterated alkyl, cycloalkyl, heterocyclyl, aryl, and heteroaryl;
$R^2$ is selected from the group consisting of hydrogen, halogen, haloalkyl, deuterated alkyl, cycloalkyl, heterocyclyl, aryl, and heteroaryl;
or $R^1$ and $R^2$, together with the carbon atom to which they are attached, form cycloalkyl or heterocyclyl;
$R^4$ and $R^5$ are identical or different and are each independently selected from the group consisting of hydrogen, alkyl, haloalkyl, deuterated alkyl, and hydroxyalkyl;
$R^6$ and $R^7$ are identical or different and are each independently selected from the group consisting of hydrogen, halogen, alkyl, haloalkyl, deuterated alkyl, and hydroxyalkyl;
m is an integer from 0 to 4;
n is as defined in claim 12;
Ab is the antibody as defined in claim 10.

**14.** The antibody-drug conjugate according to any one of claims 10 to 13, having a structure represented by formula (III):

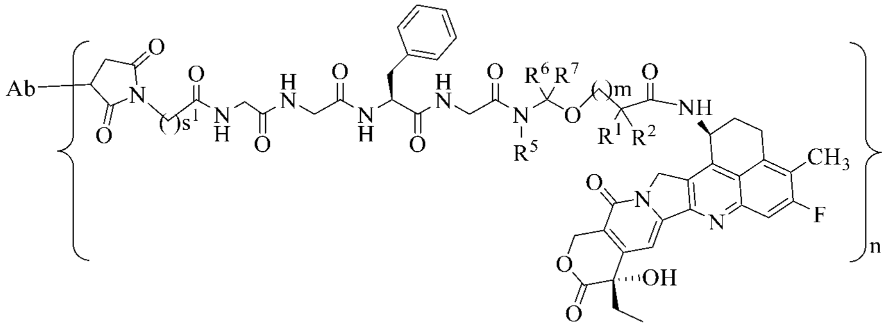

(III)

wherein:

$s^1$ is an integer from 2 to 8, preferably 5;
$R^1$, $R^2$, $R^5$-$R^7$, and m are as defined in claim 13;
n is as defined in claim 12;
Ab is the antibody as defined in claim 10.

**15.** The antibody-drug conjugate according to claim 12, wherein -L-Y- is optionally selected from the group consisting of:

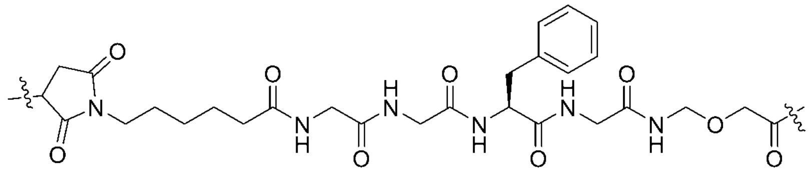

and

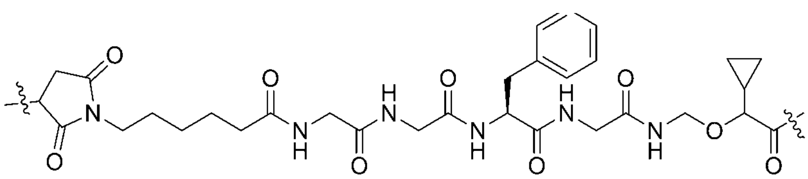

,

and is preferably

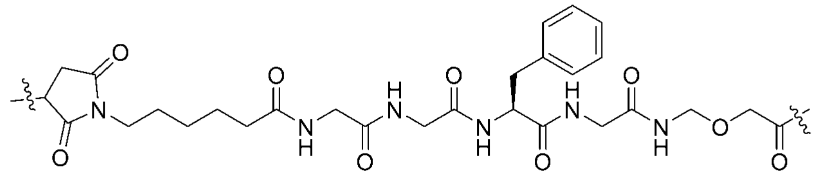

,

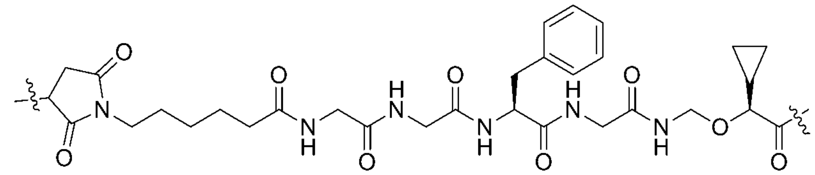

,

and

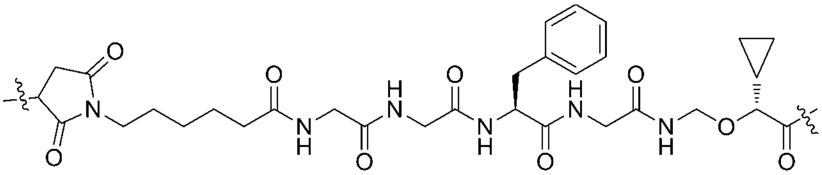

.

**16.** The antibody-drug conjugate according to any one of claims 10 to 15, wherein the antibody-drug conjugate is selected from the group consisting of the following structural formulas:

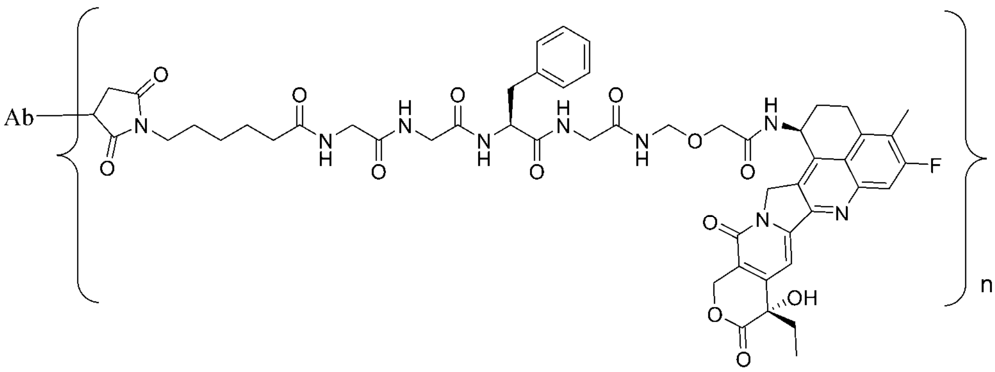

,

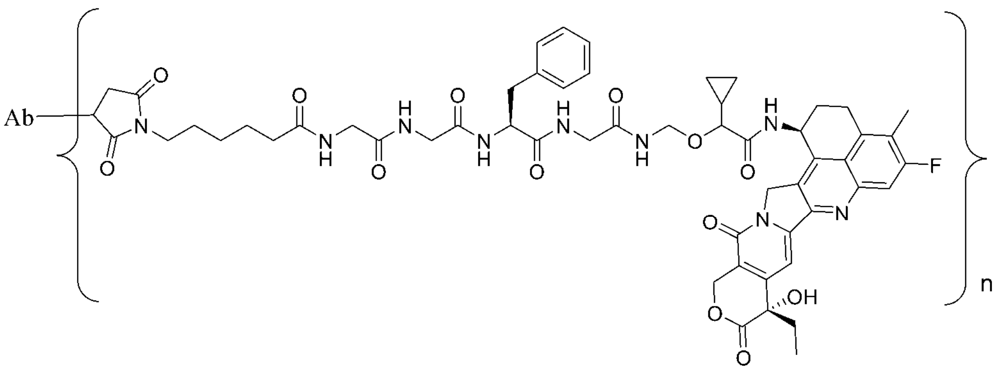

,

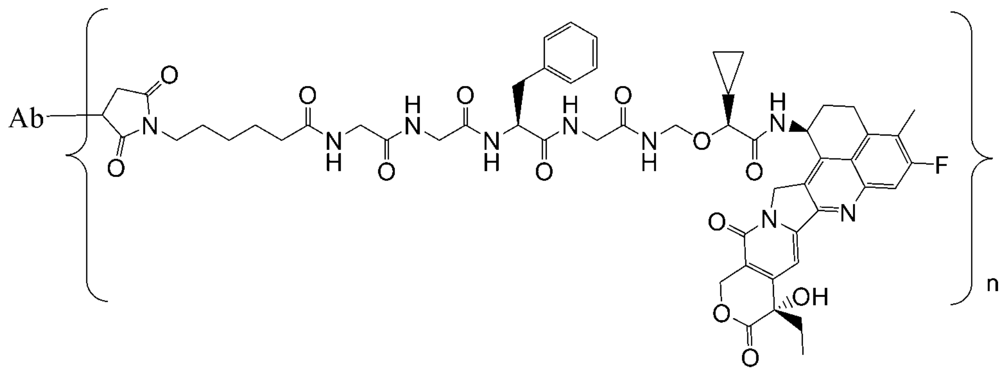

,

and

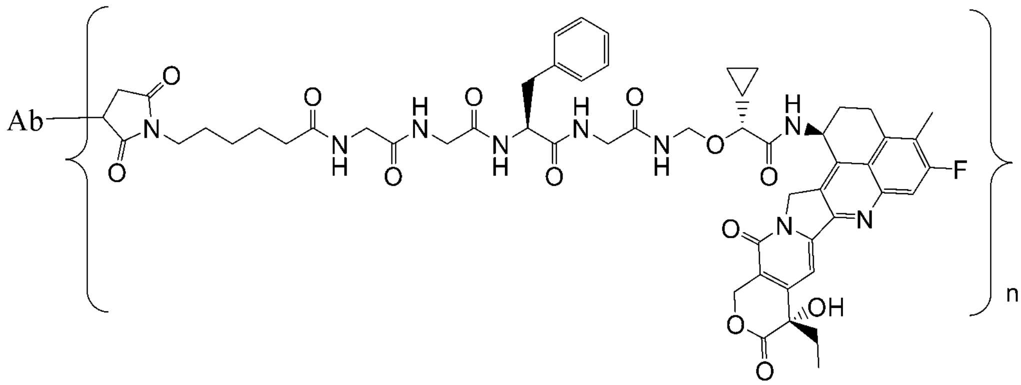

,

wherein:

n is 1 to 10 and may be an integer or a decimal; preferably, n is an integer or a decimal from 1 to 6;
Ab
Ab is the antibody as defined in claim 10.

**17.** A method for preparing the antibody-drug conjugate according to any one of claims 13 to 16, comprising subjecting Ab to a coupling reaction with a drug,
preferably comprising the following steps:

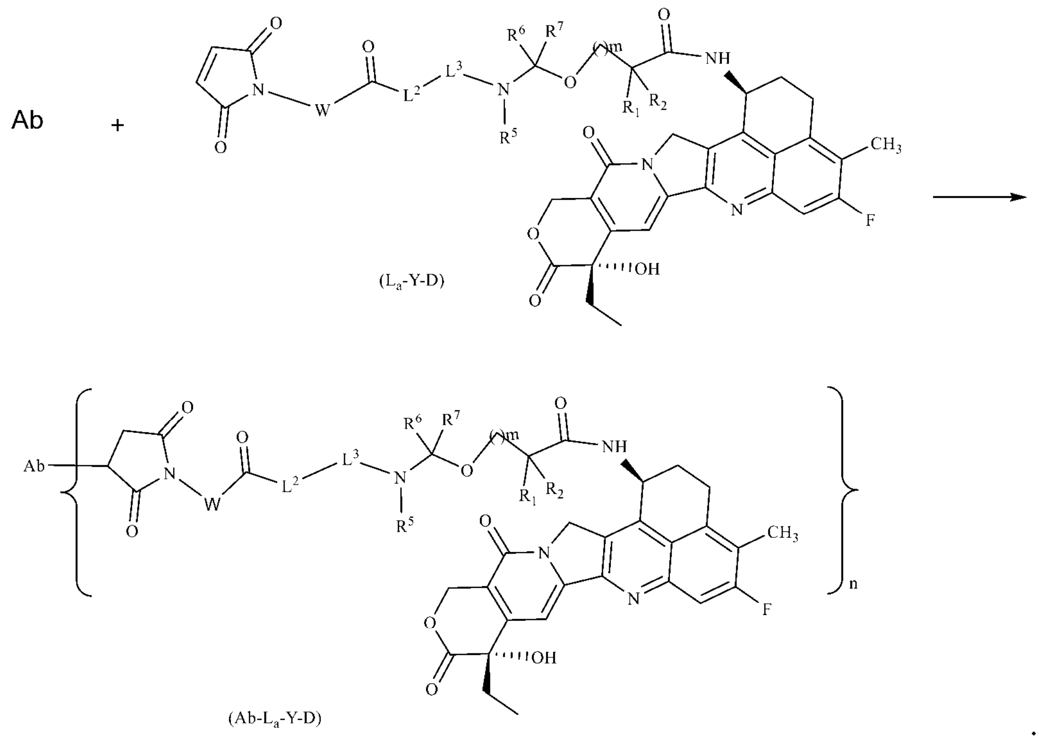

($L_a$-Y-D)

(Ab-$L_a$-Y-D)

**18.** The antibody-drug conjugate according to claim 10, having a structure represented by formula (IV):

Ab-(L-De)k (IV)

wherein -L- is a linker unit covalently attaching Ab to De,
k is an integer or a decimal from 1 to 20; preferably, n is an integer or a decimal from 1 to 6;
De is represented by the following formula:

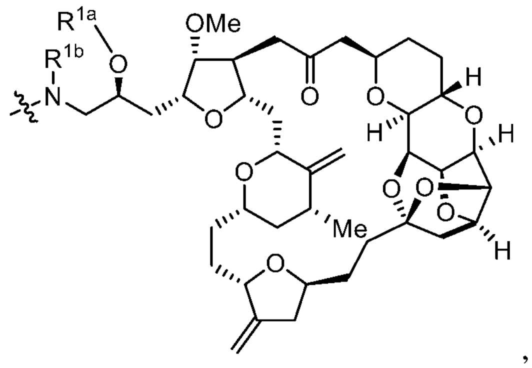

,

wherein:

$R^{1a}$ is selected from the group consisting of hydrogen, alkyl, cycloalkyl, aryl, and heteroaryl, wherein the alkyl, cycloalkyl, aryl, and heteroaryl are each independently and optionally substituted with one or more substituents selected from the group consisting of alkyl, alkoxy, halogen, deuterium, amino, cyano, nitro, hydroxy, hydroxyalkyl, cycloalkyl, heterocyclyl, aryl, and heteroaryl; preferably, $R^{1a}$ is methyl;
$R^{1b}$ is selected from the group consisting of hydrogen, alkyl, alkoxy, cycloalkyl, aryl, and heteroaryl, wherein the alkyl, cycloalkyl, aryl, and heteroaryl are each independently and optionally substituted with one or more substituents selected from the group consisting of alkyl, alkoxy, halogen, deuterium, amino, cyano, nitro, hydroxy, hydroxyalkyl, cycloalkyl, heterocyclyl, aryl, and heteroaryl; preferably, $R^{1b}$ is hydrogen; or

$R^{1a}$ and $R^{1b}$, together with the carbon atom to which they are attached, form $C_{5-8}$ heterocycloalkyl, wherein the heteroalkyl is optionally substituted with one or more substituents of alkyl, alkoxy, halogen, deuterium, amino, cyano, nitro, hydroxy, hydroxyalkyl, cycloalkyl, heterocyclyl, aryl, and heteroaryl, and $R^{1a}$ and $R^{1b}$ are not both hydrogen;
Ab is the antibody as defined in claim 10.

**19.** The antibody-drug conjugate according to claim 18, wherein the linker unit comprises a cleavable peptide moiety;

preferably, the cleavable peptide moiety is cleavable by an enzyme;
more preferably, the enzyme is a cathepsin.

**20.** The antibody-drug conjugate according to claim 18 or 19, wherein:

the linker unit comprises a peptide residue consisting of 2 to 7 amino acids,
wherein the amino acids are selected from the group consisting of: phenylalanine, glycine, valine, lysine, citrulline, serine, glutamic acid, and aspartic acid;
more preferably, the peptide residue is selected from the group consisting of: valine-citrulline, alanine-alanine-asparagine, glycine-glycine-lysine, valine-lysine, valine-alanine, valine-phenylalanine, and glycine-glycine-phenylalanine-glycine (GGFG, SEQ ID NO: 69).

**21.** The antibody-drug conjugate according to claim 18, wherein the linker unit comprises a cleavable sulfonamide moiety or a cleavable disulfide moiety;
preferably, the linker unit is cleavable under reducing conditions.

**22.** The antibody-drug conjugate according to any one of claims 18 to 21, wherein the linker unit comprises a spacer unit attached to De;
preferably, the spacer unit comprises p-aminobenzyloxycarbonyl (PAB).

**23.** The antibody-drug conjugate according to any one of claims 18 to 22, wherein the antibody-drug conjugate is represented by any one of structures selected from the group consisting of:

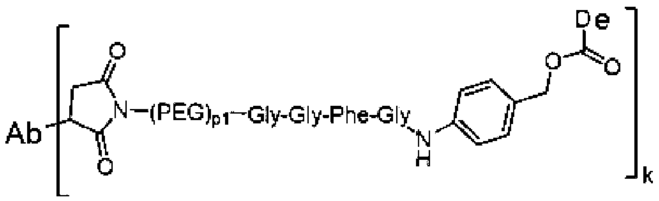

,

wherein k is selected from the group consisting of 1 to 10, and may be an integer or a decimal; and p1 is selected from the group consisting of 2, 4, 6, and 8;

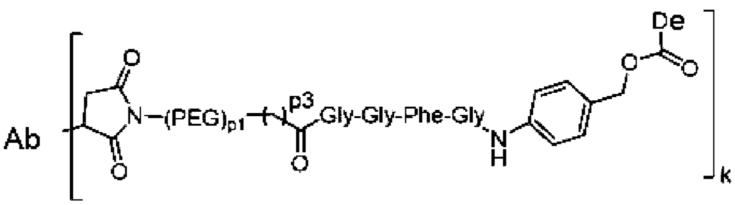

,

wherein k is selected from the group consisting of 1 to 10, and may be an integer or a decimal; p1 is selected from the group consisting of 2, 4, 6, and 8; and P3 is selected from the group consisting of 0, 1, and 2;

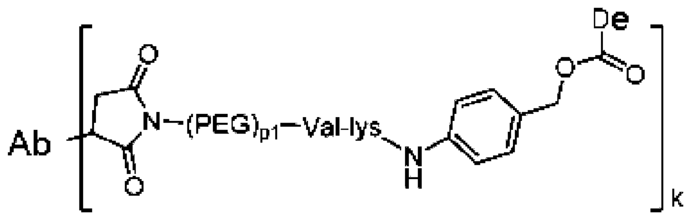

,

wherein k is selected from the group consisting of 1 to 10, and may be an integer or a decimal; and p1 is selected from the group consisting of 2, 4, 6, and 8;

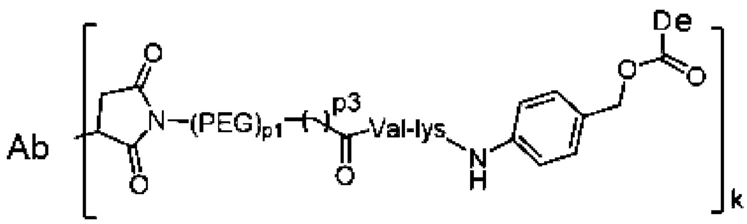

,

wherein k is selected from the group consisting of 1 to 10, and may be an integer or a decimal; p1 is selected from the group consisting of 2, 4, 6, and 8; and P3 is selected from the group consisting of 0, 1, and 2;

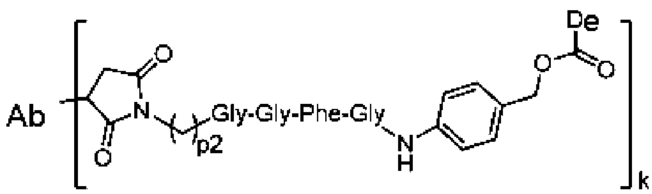

,

wherein k is selected from the group consisting of 1 to 10, and may be an integer or a decimal; and p2 is selected from the group consisting of 2, 4, 6, and 8;

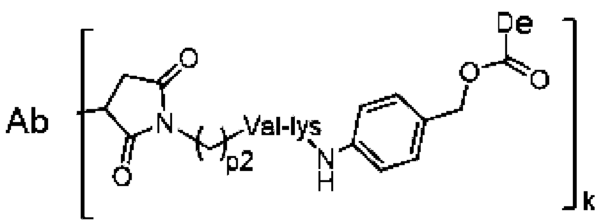

,

wherein k is selected from the group consisting of 1 to 10, and may be an integer or a decimal; and p2 is selected from the group consisting of 2, 4, 6, and 8;

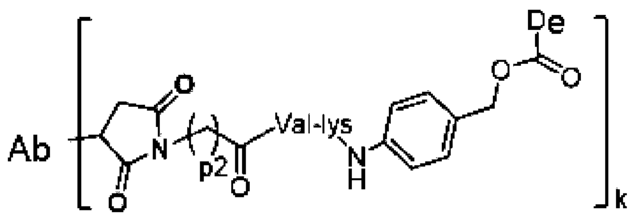

,

wherein k is selected from the group consisting of 1 to 10, and may be an integer or a decimal; and p2 is selected from the group consisting of 2, 4, 6, and 8;

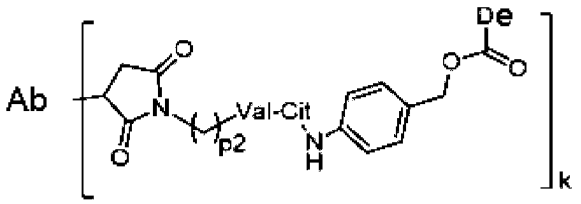

,

wherein k is selected from the group consisting of 1 to 10, and may be an integer or a decimal; and p2 is selected from the group consisting of 2, 4, 6, and 8;

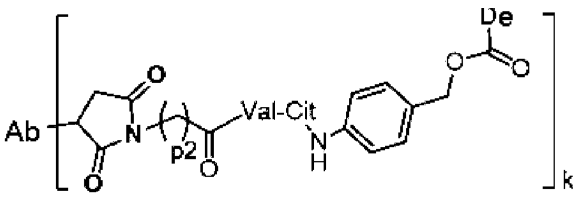

wherein k is selected from the group consisting of 1 to 10, and may be an integer or a decimal; and p2 is selected from the group consisting of 2, 4, 6, and 8;

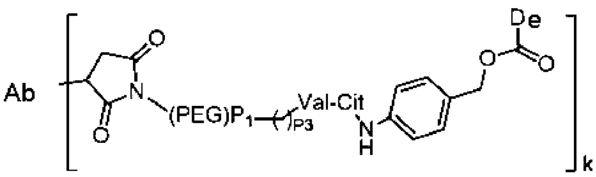

,

wherein k is selected from the group consisting of 1 to 10, and may be an integer or a decimal; p1 is selected from the group consisting of 2, 4, 6, and 8; and P3 is selected from the group consisting of 0, 1, and 2;

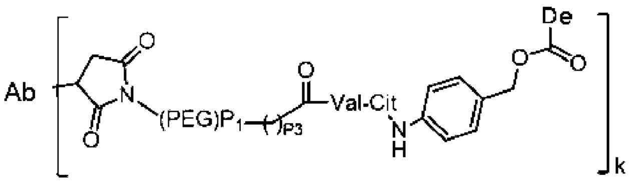

,

wherein k is selected from the group consisting of 1 to 10, and may be an integer or a decimal; p1 is selected from the group consisting of 2, 4, 6, and 8; and P3 is selected from the group consisting of 0, 1, and 2; preferably,

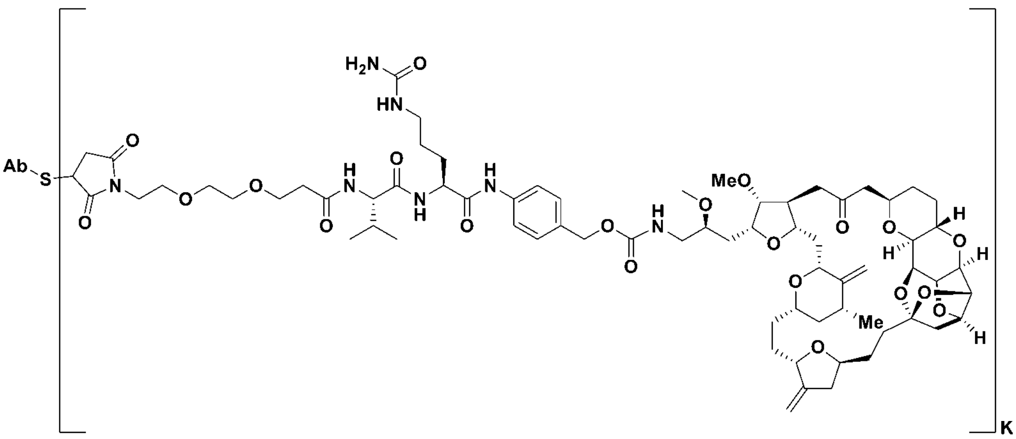

k is selected from the group consisting of 1 to 10, and is an integer or a decimal.

**24.** An antibody-drug conjugate, having the following structure:

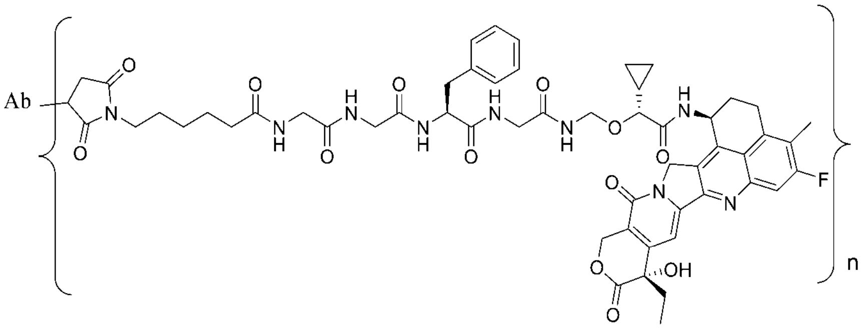

,

wherein n is 1 to 10, and n is an integer or a decimal;
or

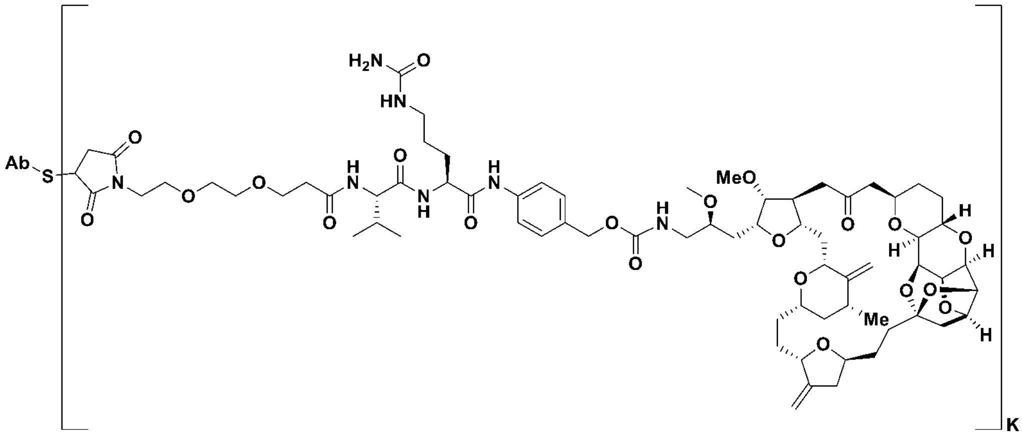

,

wherein k is selected from the group consisting of 1 to 10, and is an integer or a decimal;
Ab comprises the first binding domain that specifically binds to MET and the second binding domain that specifically binds to HER3 according to claims 1-9.

**25.** A MET-binding molecule, comprising a heavy chain variable region (VH1) and a light chain variable region (VL1), wherein the VH1 comprises a HCDR1, a HCDR2, and a HCDR3 from the amino acid sequence set forth in SEQ ID NO: 71; and the VL1 comprises a LCDR1, a LCDR2, and a LCDR3 from the amino acid sequence set forth in SEQ ID NO: 72;

the CDRs are defined according to the Kabat, IMGT, Chothia, AbM, or Contact numbering scheme;
preferably, the heavy chain variable region comprises a HCDR1, a HCDR2, and a HCDR3 set forth in SEQ ID NOs: 15-17, respectively, and the light chain variable region comprises a LCDR1, a LCDR2, and a LCDR3 set forth in SEQ ID NOs: 74, 19, and 20, respectively.

**26.** The MET-binding molecule according to claim 25, wherein the VH1 comprises a K mutation at position 23 and/or a T mutation at position 78, naturally numbered relative to SEQ ID NO: 1; and/or the VL1 comprises a T mutation at position 69 naturally numbered relative to SEQ ID NO: 2;
preferably, the MET-binding molecule comprises E23K and S78T mutations naturally numbered relative to SEQ ID NO: 1, and an A69T mutation naturally numbered relative to SEQ ID NO: 2.

**27.** The MET-binding molecule according to claim 25 or 26, wherein the heavy chain variable region comprises the amino acid sequence set forth in SEQ ID NO: 71 or an amino acid sequence having at least 90% identity thereto, and the light chain variable region comprises the amino acid sequence set forth in SEQ ID NO: 72 or an amino acid sequence

having at least 90% identity thereto.

**28.** A HER3-binding molecule, comprising a heavy chain variable region (VH2) and a light chain variable region (VL2), wherein the VH2 comprises a HCDR1, a HCDR2, and a HCDR3 from the amino acid sequence set forth in SEQ ID NO: 13, and the VL2 comprises a LCDR1, a LCDR2, and a LCDR3 from the amino acid sequence set forth in SEQ ID NO: 70;

the CDRs are defined according to the Kabat, IMGT, Chothia, AbM, or Contact numbering scheme;
preferably, the heavy chain variable region comprises a HCDR1, a HCDR2, and a HCDR3 set forth in SEQ ID NOs: 51-53, respectively, and the light chain variable region comprises a LCDR1, a LCDR2, and a LCDR3 of the amino acid sequences set forth in SEQ ID NOs: 54, 55, and 73, respectively.

**29.** The HER3-binding molecule according to claim 28, wherein the heavy chain variable region comprises the amino acid sequence set forth in SEQ ID NO: 13 or an amino acid sequence having at least 90% identity thereto, and the light chain variable region comprises the amino acid sequence set forth in SEQ ID NO: 70 or an amino acid sequence having at least 90% identity thereto.

**30.** A polynucleotide encoding the HER3/MET-binding molecule according to any one of claims 1 to 9, the MET-binding molecule according to any one of claims 25 to 27, or the HER3-binding molecule according to claim 28 or 29.

**31.** A vector, comprising the polynucleotide according to claim 30.

**32.** A host cell, comprising the polynucleotide according to claim 30 or the vector according to claim 31.

**33.** A pharmaceutical composition, comprising the HER3/MET-binding molecule according to any one of claims 1 to 9, the antibody-drug conjugate according to any one of claims 10 to 16 and 18 to 24, an antibody-drug conjugate prepared by the method according to claim 17, the MET-binding molecule according to any one of claims 25 to 27, the HER3-binding molecule according to claim 28 or 29, the polynucleotide according to claim 30, or the vector according to claim 31, wherein
preferably, the pharmaceutical composition further comprises one or more pharmaceutically acceptable excipients, diluents, or auxiliary materials.

**34.** A method for preparing the HER3/MET-binding molecule according to any one of claims 1 to 9, the MET-binding molecule according to any one of claims 25 to 27, or the HER3-binding molecule according to claim 28 or 29, comprising culturing the host cell according to claim 32 and expressing the HER3/MET-binding molecule according to any one of claims 1 to 9, the MET-binding molecule according to any one of claims 25 to 27, or the HER3-binding molecule according to claim 28 or 29,
optionally, comprising isolating and/or purifying the HER3/MET-binding molecule, the MET-binding molecule, or the HER3-binding molecule.

**35.** Use of the HER3/MET-binding molecule according to any one of claims 1 to 9, the antibody-drug conjugate according to any one of claims 10 to 16 and 18 to 24, an antibody-drug conjugate prepared by the method according to claim 17, the MET-binding molecule according to any one of claims 25 to 27, the HER3-binding molecule according to claim 28 or 29, the polynucleotide according to claim 30, the vector according to claim 31, or the pharmaceutical composition according to claim 33 in the treatment or alleviation of cancer, or in the manufacture of a medicament for treating or alleviating cancer, wherein

preferably, the cancer is breast cancer or lung cancer; preferably, the cancer is HER3-positive, preferably HER3-positive breast cancer or lung cancer;
preferably, the cancer is HER3/MET double-positive, preferably HER3/MET double-positive breast cancer or lung cancer.

**36.** A method for treating or alleviating cancer, comprising the step of:

administering to a subject a therapeutically effective amount of the HER3/MET-binding molecule according to any one of claims 1 to 9, the antibody-drug conjugate according to any one of claims 10 to 16 and 18 to 24, an antibody-drug conjugate prepared by the method according to claim 17, the MET-binding molecule according to any one of claims 25 to 27, the HER3-binding molecule according to claim 28 or 29, the polynucleotide according to claim 30,

the vector according to claim 31, or the pharmaceutical composition according to claim 33, wherein preferably, the cancer is breast cancer or lung cancer; preferably, the cancer is HER3-positive; preferably, the cancer is HER3/MET double-positive.

**37.** An antibody-drug conjugate, wherein

Ab is an antibody comprising a first binding domain that specifically binds to MET and a second binding domain that specifically binds to HER3, wherein the first binding domain that specifically binds to MET comprises a heavy chain variable region (VH1) and a light chain variable region (VL1), and the second binding domain that specifically binds to HER3 comprises a heavy chain variable region (VH2) and a light chain variable region (VL2); Ab comprises:

a first heavy chain that is, from the N-terminus to the C-terminus, [VH1]-[linker 1]-[Obscurin-O chain]-[linker 3]-[first subunit of Fc region],
a first light chain that is, from the N-terminus to the C-terminus, [VL1]-[linker 2]-[Titin-T chain],
a second heavy chain that is, from the N-terminus to the C-terminus, [VH2]-[CH1]-[second subunit of Fc region], and
a second light chain that is, from the N-terminus to the C-terminus, [VL2]-[CL],
wherein - represents a peptide bond, and the linker 1, the linker 2, and the linker 3 may be identical or different, and may be independently present or absent;
preferably, i) the antibody-drug conjugate is represented by general formula (I):

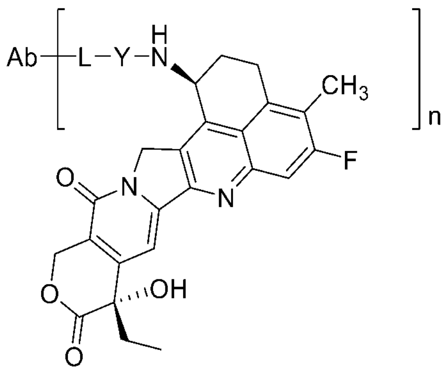

wherein -L- is a linker unit that is $-L^1-L^2-L^3-L^4-$;
$L^1$ is -(succinimid-3-yl-*N*)-W-C(O)-, $-CH_2-C(O)-NR^3-W-C(O)-$, or -C(O)-W-C(O)-, wherein W is selected from the group consisting of $C_{1-8}$ alkyl, $C_{1-8}$ alkyl-cycloalkyl, and linear heteroalkyl of 1 to 8 atoms, and the heteroalkyl comprises 1 to 3 heteroatoms selected from the group consisting of N, O, and S, wherein the $C_{1-8}$ alkyl, cycloalkyl, and linear heteroalkyl are each independently and optionally further substituted with one or more substituents selected from the group consisting of halogen, hydroxy, cyano, amino, alkyl, chloroalkyl, deuterated alkyl, alkoxy, and cycloalkyl;
$L^2$ is selected from the group consisting of $-NR^4(CH_2CH_2O)p^1CH_2CH_2C(O)-$, $-NR^4(CH_2CH_2O)p^1CH_2C(O)-$, $-S(CH_2)p^1C(O)-$, and a chemical bond, wherein $p^1$ is an integer from 1 to 20;
$L^3$ is a peptide residue consisting of 2 to 7 amino acids, wherein the amino acids are optionally further substituted with one or more substituents selected from the group consisting of halogen, hydroxy, cyano, amino, alkyl, chloroalkyl, deuterated alkyl, alkoxy, and cycloalkyl;
$L^4$ is selected from the group consisting of $-NR^5(CR^6R^7)_t-$, $-C(O)NR^5$, $-C(O)NR^5(CH_2)_t-$, and a chemical bond, wherein t is an integer from 1 to 6;
$R^3$, $R^4$, and $R^5$ are identical or different and are each independently selected from the group consisting of hydrogen, alkyl, haloalkyl, deuterated alkyl, and hydroxyalkyl;
$R^6$ and $R^7$ are identical or different and are each independently selected from the group consisting of hydrogen, halogen, alkyl, haloalkyl, deuterated alkyl, and hydroxyalkyl;
Y is selected from the group consisting of $-O-(CR^aR^b)_m-CR^1R^2-C(O)-$, $-O-CR^1R^2-(CR^aR^b)_m-$, $-O-CR^1R^2-$, $-NH-(CR^aR^b)_m-CR^1R^2-C(O)-$, and $-S-(CR^aR^b)_m-CR^1R^2-C(O)-$;
$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl, and heterocyclyl; or $R^a$ and $R^b$, together with the carbon atom to which they are

attached, form cycloalkyl or heterocyclyl;
$R^1$ is selected from the group consisting of hydrogen, halogen, haloalkyl, deuterated alkyl, cycloalkyl, cycloalkylalkyl, alkoxyalkyl, heterocyclyl, aryl, and heteroaryl;
$R^2$ is selected from the group consisting of hydrogen, halogen, haloalkyl, deuterated alkyl, cycloalkyl, cycloalkylalkyl, alkoxyalkyl, heterocyclyl, aryl, and heteroaryl;
or $R^1$ and $R^2$, together with the carbon atom to which they are attached, form cycloalkyl or heterocyclyl;
or $R^a$ and $R^2$, together with the carbon atom to which they are attached, form cycloalkyl or heterocyclyl;
m is an integer from 0 to 4;
or
ii) the antibody-drug conjugate has a structure represented by general formula Ab-(L-De)k:
L is a linker covalently attaching Ab to De,
De is represented by (III):

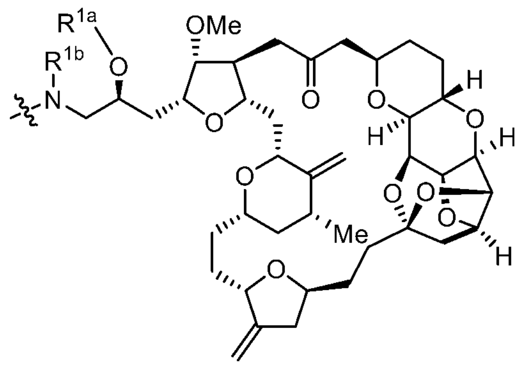

formula (IV),

wherein:

$R^{1a}$ is selected from the group consisting of hydrogen, alkyl, cycloalkyl, aryl, and heteroaryl, wherein the alkyl, cycloalkyl, aryl, and heteroaryl are each independently and optionally substituted with one or more substituents selected from the group consisting of alkyl, alkoxy, halogen, deuterium, amino, cyano, nitro, hydroxy, hydroxyalkyl, cycloalkyl, heterocyclyl, aryl, and heteroaryl; preferably, $R^{1a}$ is methyl;
$R^{1b}$ is selected from the group consisting of hydrogen, alkyl, alkoxy, cycloalkyl, aryl, and heteroaryl, wherein the alkyl, cycloalkyl, aryl, and heteroaryl are each independently and optionally substituted with one or more substituents selected from the group consisting of alkyl, alkoxy, halogen, deuterium, amino, cyano, nitro, hydroxy, hydroxyalkyl, cycloalkyl, heterocyclyl, aryl, and heteroaryl; preferably, $R^{1b}$ is hydrogen; or
$R^{1a}$ and $R^{1b}$, together with the carbon atom to which they are attached, form $C_{5-8}$ heterocycloalkyl, wherein the heteroalkyl is optionally substituted with one or more substituents of alkyl, alkoxy, halogen, deuterium, amino, cyano, nitro, hydroxy, hydroxyalkyl, cycloalkyl, heterocyclyl, aryl, and heteroaryl, and $R^{1a}$ and $R^{1b}$ are not both hydrogen.

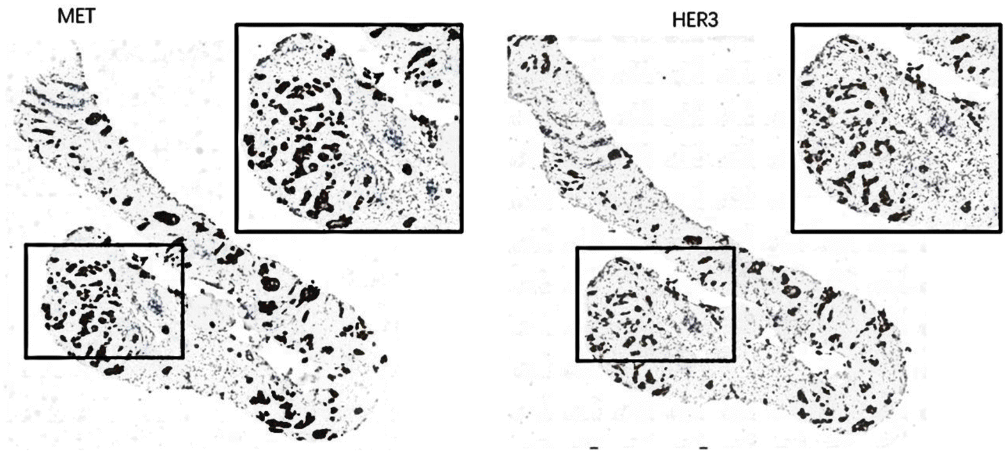


FIG. 1A

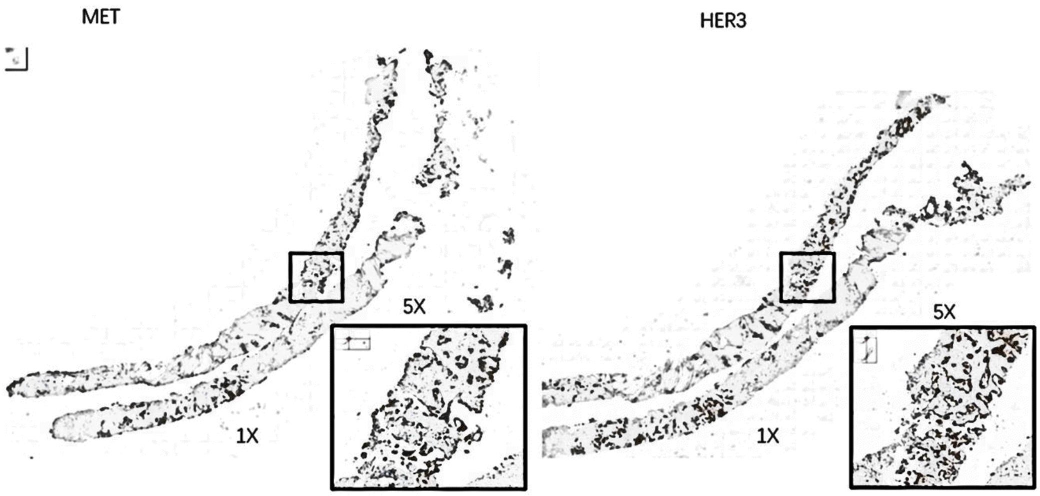


FIG. 1B

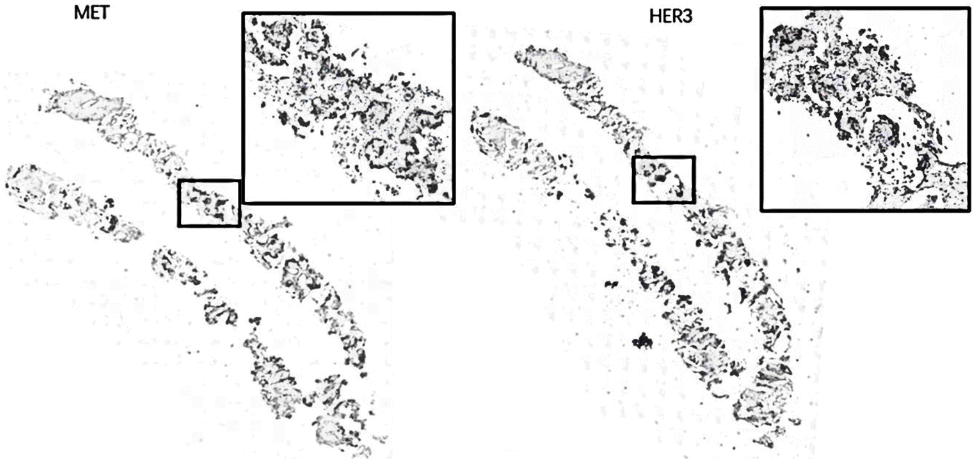


FIG. 1C

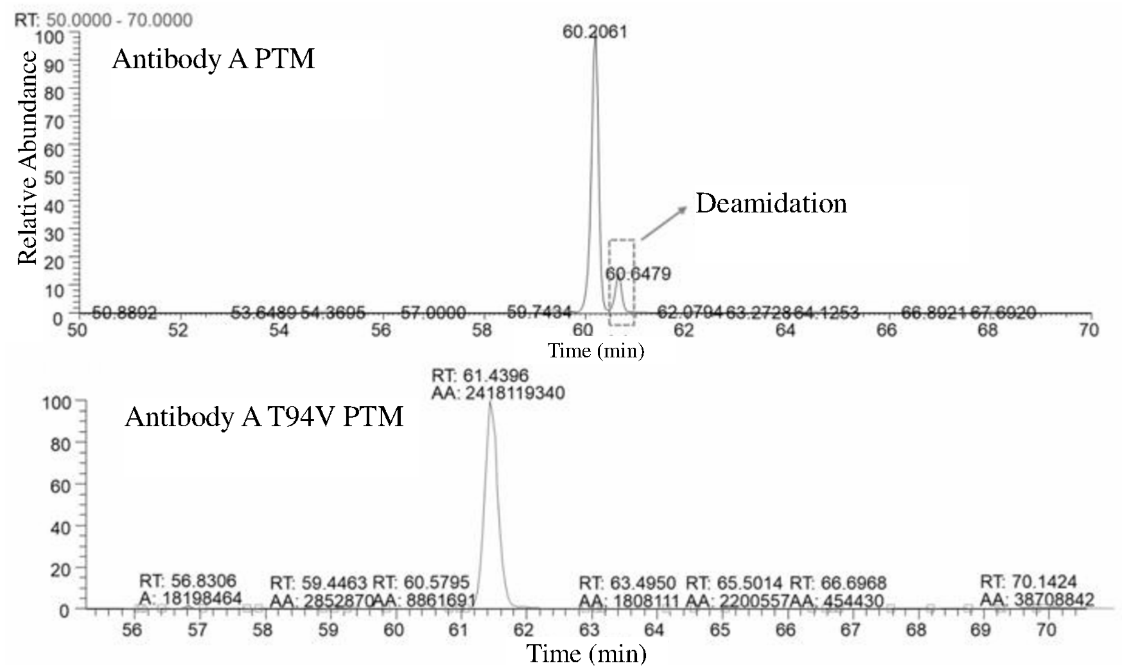
RT: 50.0000 - 70.0000
Antibody A PTM
60.2061
Deamidation
60.6479
Relative Abundance
Time (min)
Antibody A T94V PTM
RT: 61.4396
AA: 2418119340
RT: 56.8306
A: 18198464
RT: 59.4463 RT: 60.5795
AA: 2852870AA: 8861691
RT: 63.4950 RT: 65.5014 RT: 66.6968
AA: 1808111 AA: 2200557AA: 454430
RT: 70.1424
AA: 38708842
Time (min)

FIG. 2

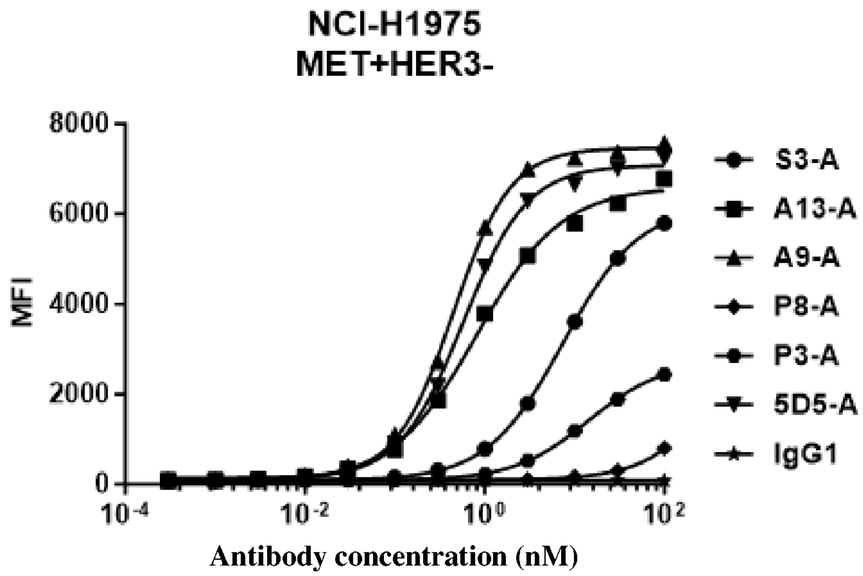
NCI-H1975
MET+HER3-
MFI
S3-A
A13-A
A9-A
P8-A
P3-A
5D5-A
IgG1
Antibody concentration (nM)

FIG. 3

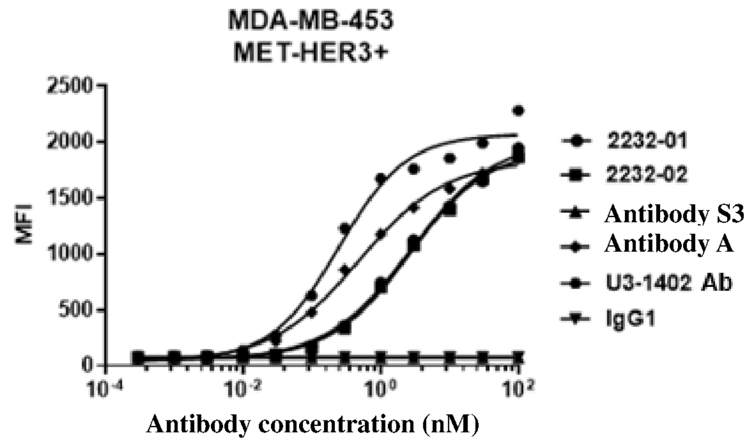


FIG. 4A

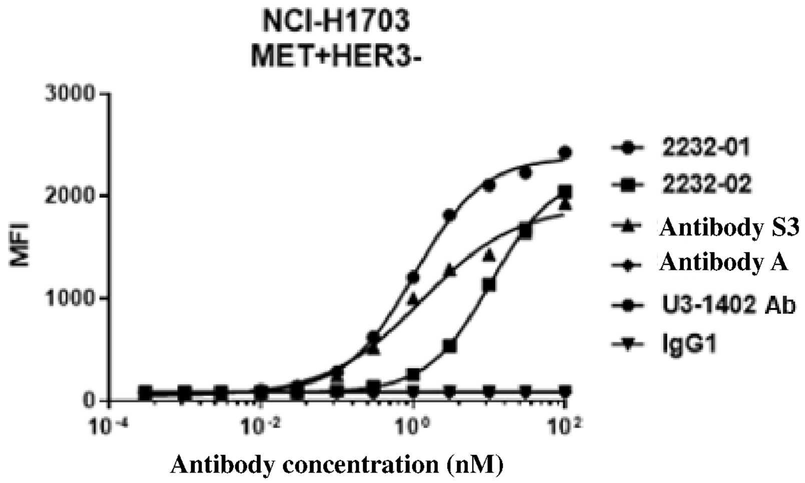


FIG. 4B

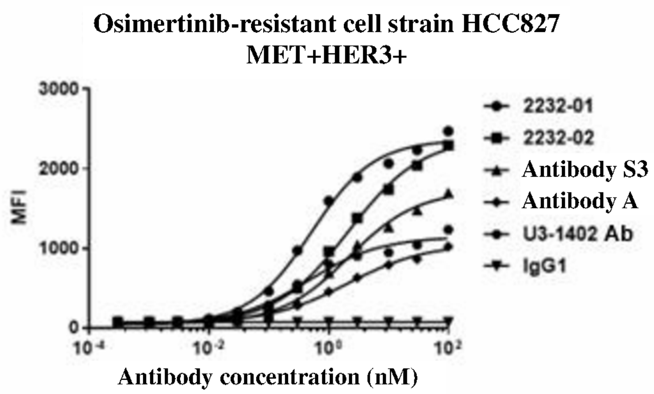


FIG. 4C

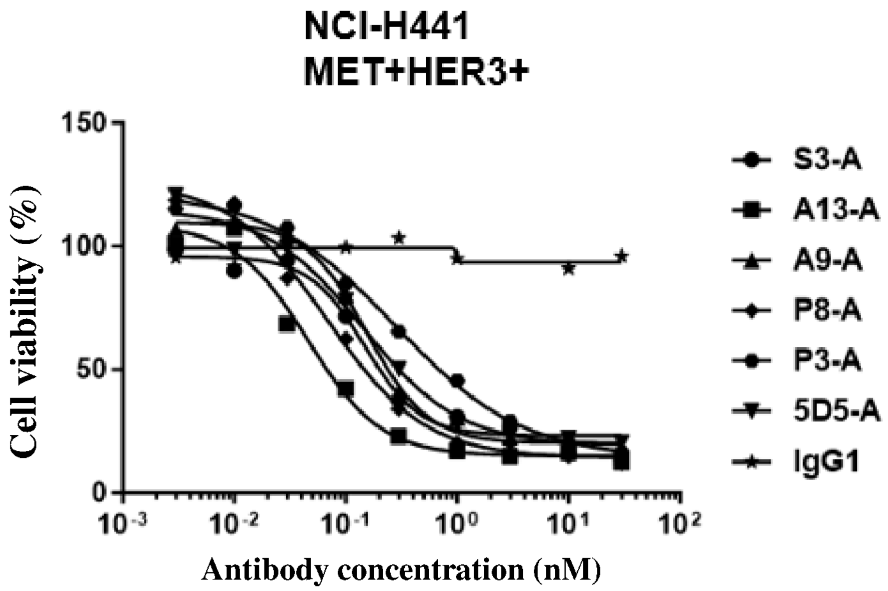


FIG. 5A

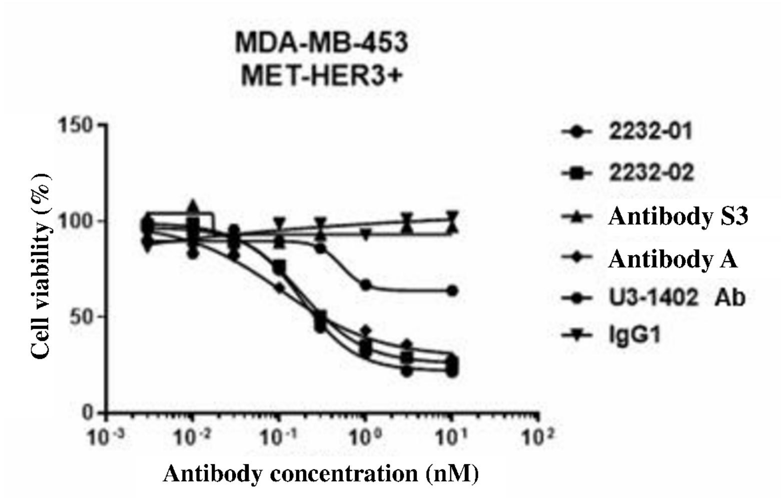


FIG. 5B

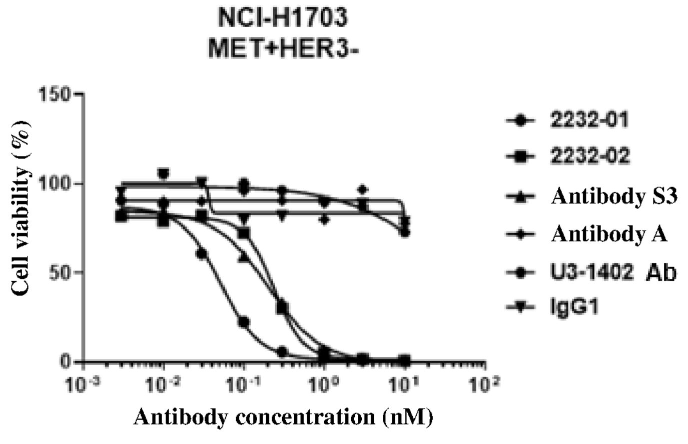


FIG. 5C

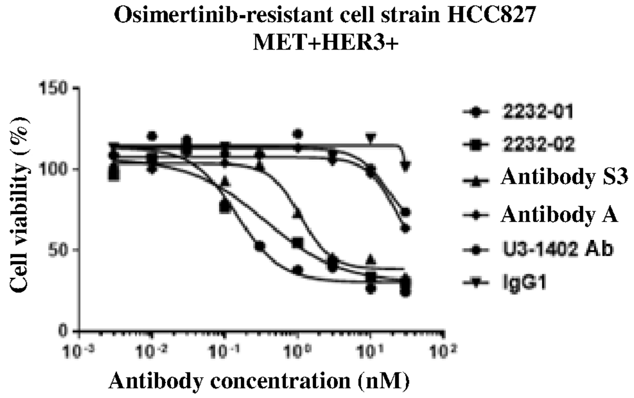


FIG. 5D

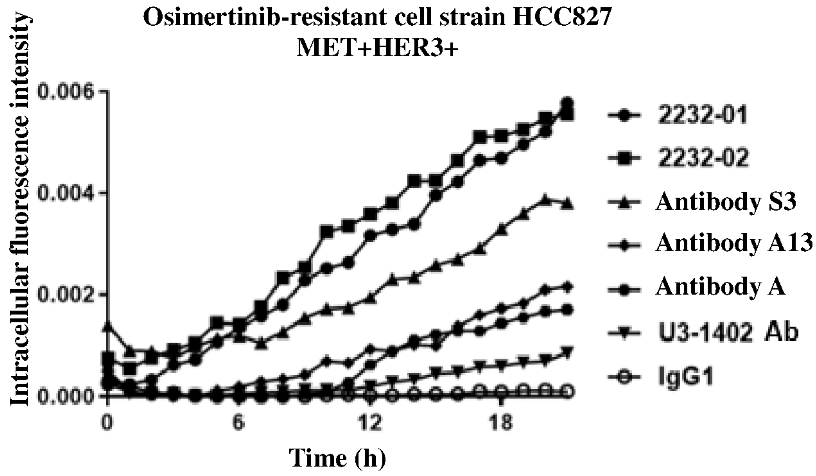


FIG. 6

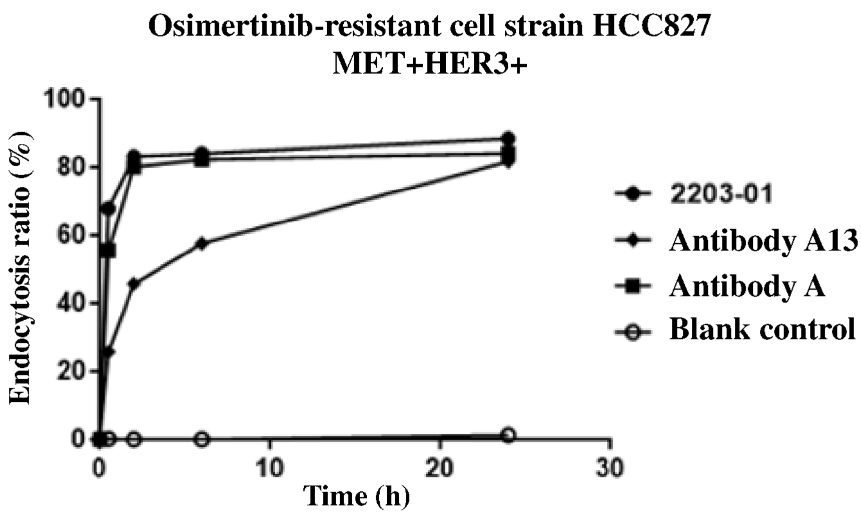


FIG. 7

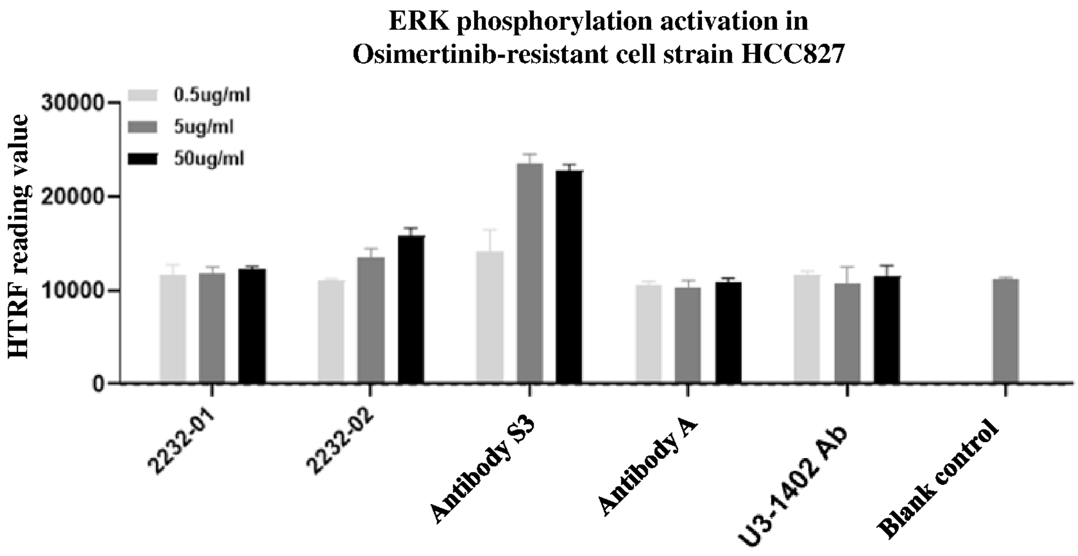


FIG. 8

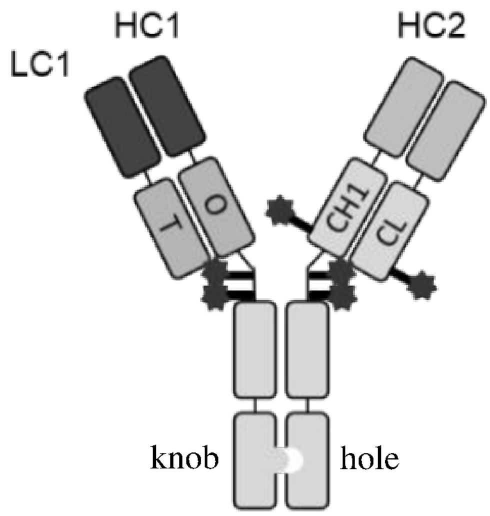


FIG. 9A

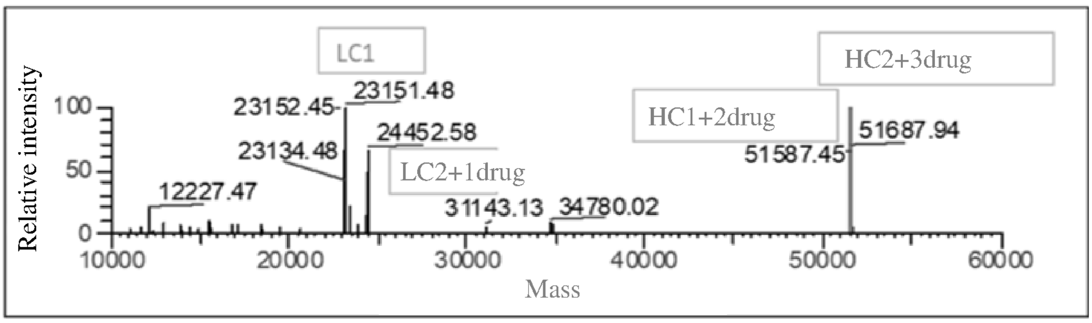


FIG. 9B

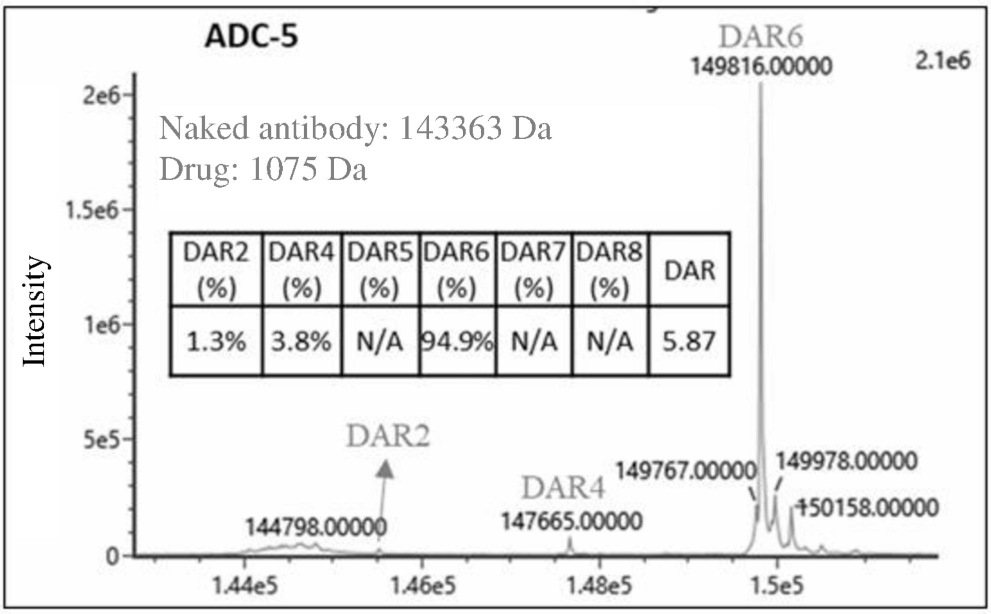
ADC-5
DAR6
149816.00000
2.1e6
2e6
1.5e6
1e6
5e5
0
Intensity
Naked antibody: 143363 Da
Drug: 1075 Da
DAR2 (%) | DAR4 (%) | DAR5 (%) | DAR6 (%) | DAR7 (%) | DAR8 (%) | DAR
1.3% | 3.8% | N/A | 94.9% | N/A | N/A | 5.87
DAR2
144798.00000
DAR4
147665.00000
149767.00000
149978.00000
150158.00000
1.44e5
1.46e5
1.48e5
1.5e5

FIG. 9C

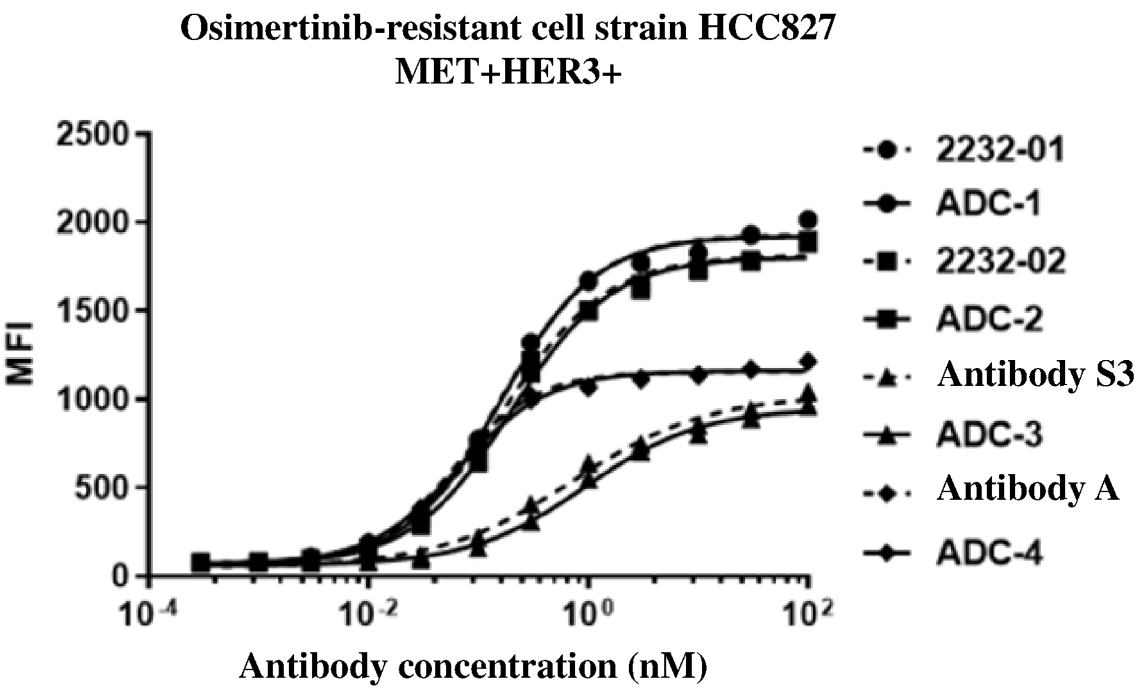
Osimertinib-resistant cell strain HCC827
MET+HER3+
MFI
2500
2000
1500
1000
500
0
$10^{-4}$
$10^{-2}$
$10^{0}$
$10^{2}$
Antibody concentration (nM)
2232-01
ADC-1
2232-02
ADC-2
Antibody S3
ADC-3
Antibody A
ADC-4

FIG. 10

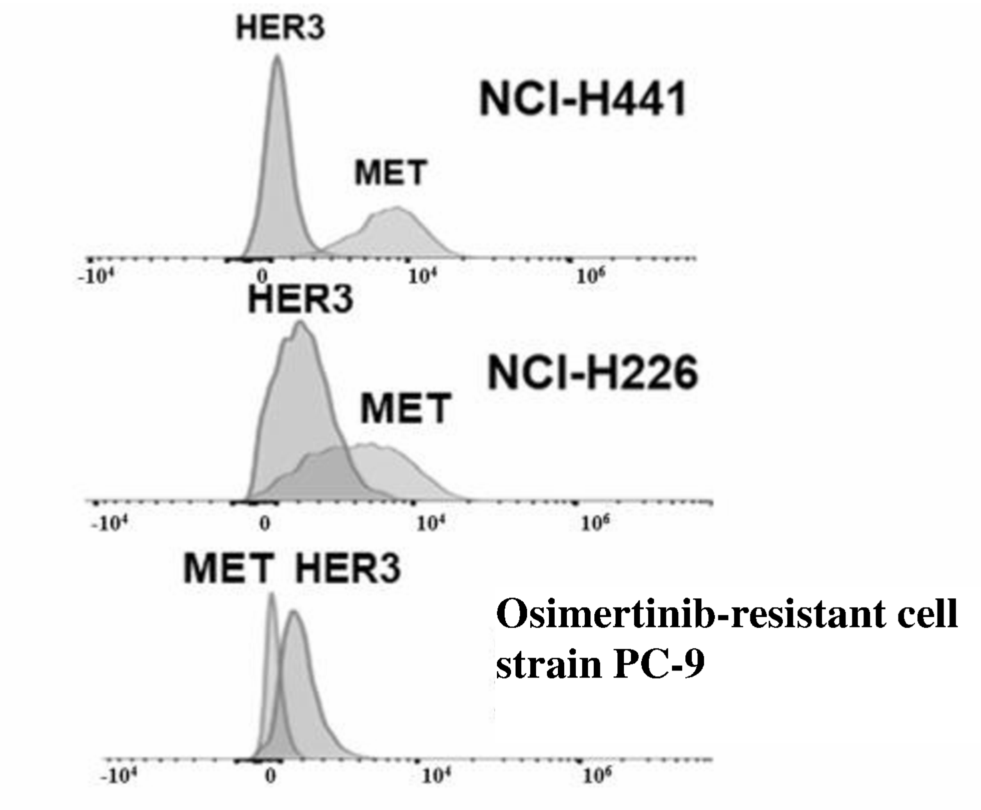


FIG. 11A

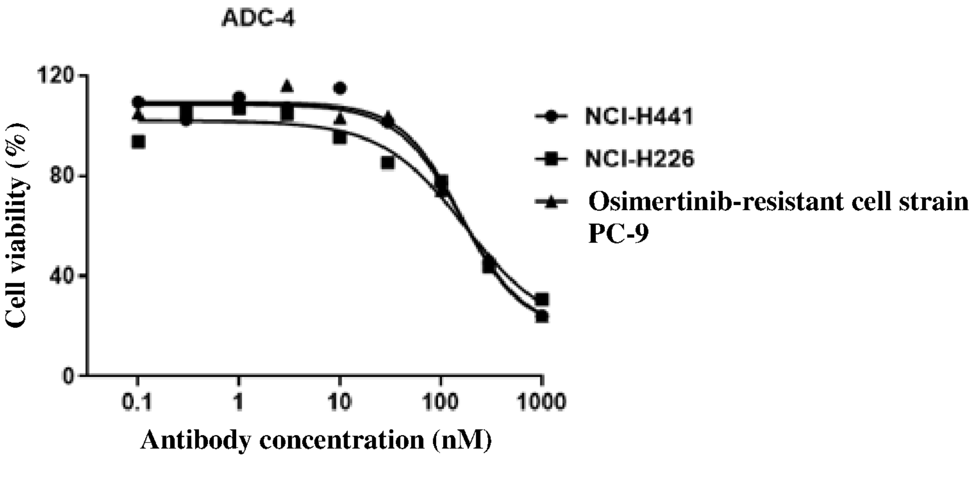


FIG. 11B

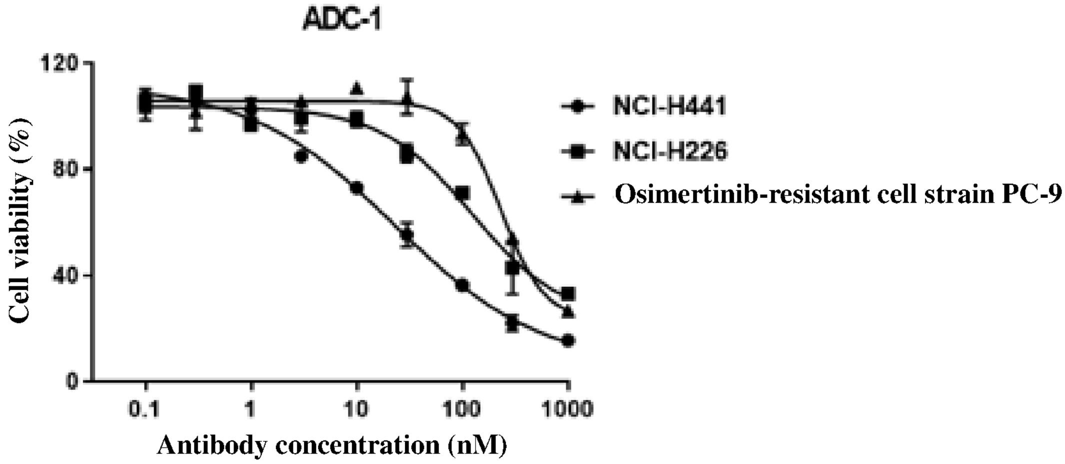


FIG. 11C

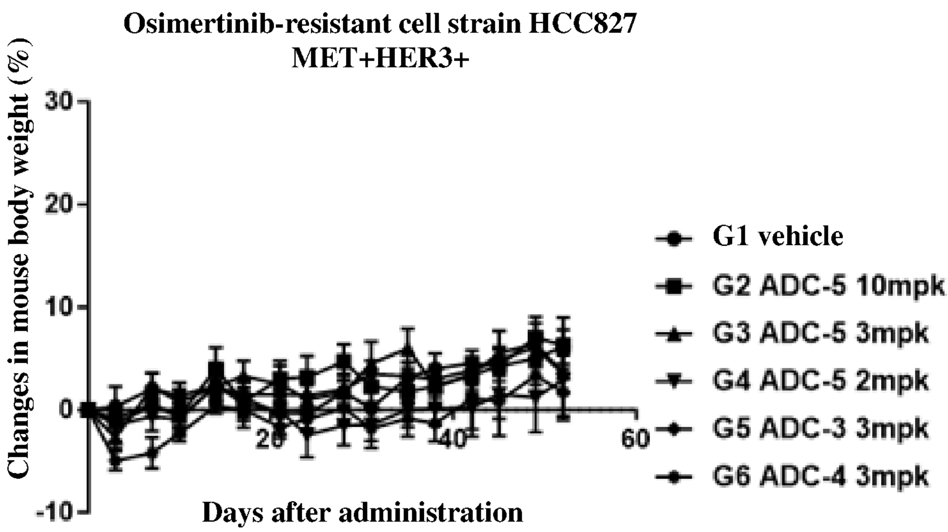


FIG. 12A

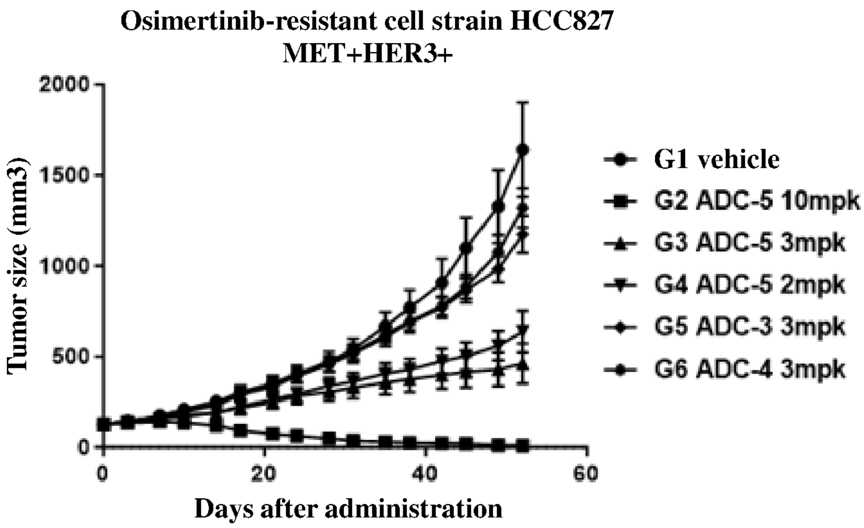


FIG. 12B

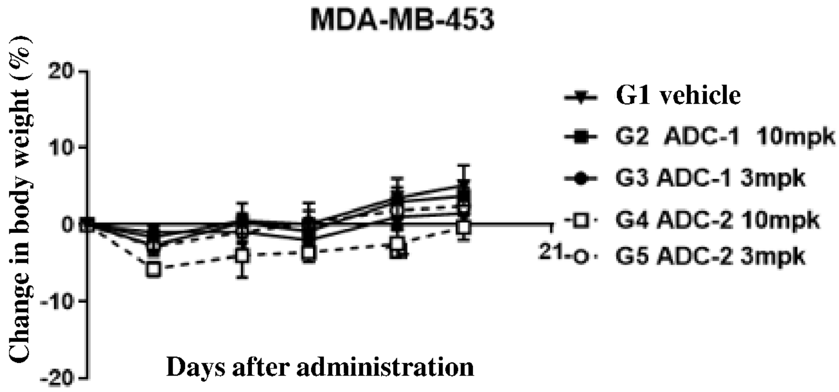


FIG. 13A

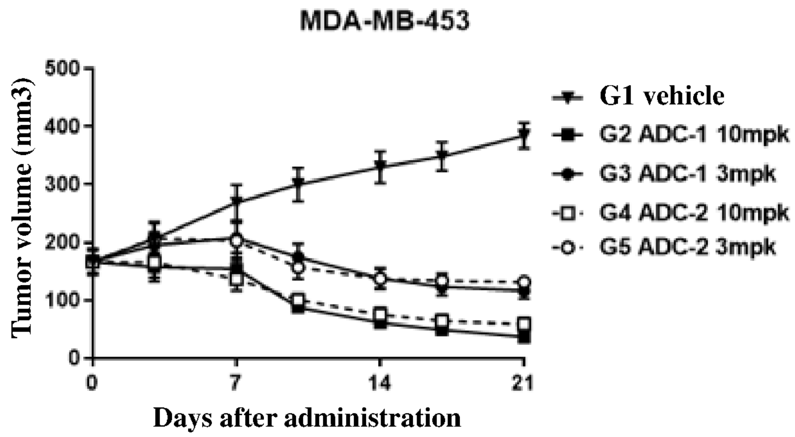


FIG. 13B

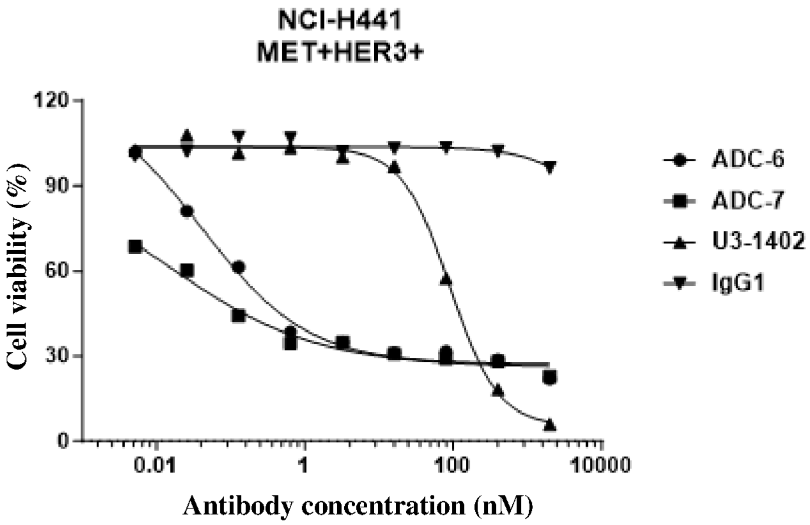
NCI-H441
MET+HER3+
120
90
60
30
0
Cell viability (%)
0.01
1
100
10000
Antibody concentration (nM)
ADC-6
ADC-7
U3-1402
IgG1

FIG. 14

## INTERNATIONAL SEARCH REPORT

International application No.
**PCT/CN2024/118787**

**A. CLASSIFICATION OF SUBJECT MATTER**

C07K16/46(2006.01)i; C07K16/28(2006.01)i; C12N15/63(2006.01)i; A61K47/65(2017.01)i; A61K47/68(2017.01)i; A61K39/395(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07K C12N A61K A61P

Documentation searched other than minimum documentation to the extent that such documents are included in the fields searched

Electronic data base consulted during the international search (name of data base and, where practicable, search terms used)

CNTXT; ENTXTC; VCN; CJFD; DWPI; ENTXT; CNKI; WFNPL; NPTXT; NPABS; Web of Science; STNext; NCBI; EMBL; IMGT; 中国专利生物序列检索系统, China Patent Biological Sequence Search System; 读秀, DUXIU: met, c-met, her3, epidermal growth factor receptor3, ErbB-3, ADC, Titin, Obscurin, Antibody, Drug, Conjugates, Mesenchymal-Epithelial Transition Factors, 抗体, 药物, 偶联物, 间充质-上皮转变因子, 权利要求中涉及的序列检索, search for sequences involved in claims

**C. DOCUMENTS CONSIDERED TO BE RELEVANT**

| Category* | Citation of document, with indication, where appropriate, of the relevant passages | Relevant to claim No. |
|---|---|---|
| Y | LI, Zhuolin et al. "BCG022: A Novel Bispecific Antibody-Drug Conjugate Targeting HER3 and MET" *Cancer Research,* Vol. 83, No. (8_Supplement), 14 April 2023 (2023-04-14), LB212<br>abstract | 37 |
| Y | WO 2022237882 A1 (JIANGSU HENGRUI PHARMACEUTICALS CO., LTD. et al.) 17 November 2022 (2022-11-17)<br>claim 12, and description, page 35, paragraph 4 | 37 |
| A | LI, Zhuolin et al. "BCG022: A Novel Bispecific Antibody-Drug Conjugate Targeting HER3 and MET" *Cancer Research,* Vol. 83, No. (8_Supplement), 14 April 2023 (2023-04-14), LB212<br>abstract | 1-34, 35 (in part) |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 December 2024** | **11 December 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
PCT/CN2024/118787

C. DOCUMENTS CONSIDERED TO BE RELEVANT

| Category* | Citation of document, with indication, where appropriate, of the relevant passages | Relevant to claim No. |
|---|---|---|
| A | CN 109706120 A (SHENZHEN BINDE BIOTECHNOLOGY CO., LTD.) 03 May 2019 (2019-05-03)<br>claims 1-10 | 1-27, 30-34, 35 (in part), 37 |
| A | WO 2022078425 A1 (JIANGSU HENGRUI MEDICINE CO., LTD. et al.) 21 April 2022 (2022-04-21)<br>description, embodiment 2 | 1-24, 28-34, 35 (in part), 37 |

Form PCT/ISA/210 (second sheet) (July 2022)

**INTERNATIONAL SEARCH REPORT**

International application No.
**PCT/CN2024/118787**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/118787**

**Box No. II Observations where certain claims were found unsearchable (Continuation of item 2 of first sheet)**

This international search report has not been established in respect of certain claims under Article 17(2)(a) for the following reasons:

1. [✓] Claims Nos.: **35 (in part), 36**
because they relate to subject matter not required to be searched by this Authority, namely:

The solution of claim 35 involving "the use for treating or alleviating cancer" and the method involved in claim 36 are disease treatment methods that are practiced on a living human or animal body for the direct purpose of disease treatment, fall within methods for treatment of the human or animal body by therapy, and fall within the cases set out in PCT Rule 39.1(iv) for which an international search is not required.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/118787**

| Patent document cited in search report | Publication date (day/month/year) | Patent family member(s) | Publication date (day/month/year) |
|---|---|---|---|
| WO 2022237882 A1 | 17 November 2022 | EP 4339213 A1 | 20 March 2024 |
| | | MX 2023013307 A | 04 December 2023 |
| | | KR 20240007196 A | 16 January 2024 |
| | | AU 2022273737 A1 | 16 November 2023 |
| | | CA 3219388 A1 | 17 November 2022 |
| | | TW 202311295 A | 16 March 2023 |
| | | JP 2024517907 A | 23 April 2024 |
| CN 109706120 A | 03 May 2019 | None | |
| WO 2022078425 A1 | 21 April 2022 | CA 3196940 A1 | 21 April 2022 |
| | | MX 2023004189 A | 03 May 2023 |
| | | JP 2023545925 A | 01 November 2023 |
| | | KR 20230086702 A | 15 June 2023 |
| | | AU 2021360625 A1 | 01 June 2023 |
| | | US 2024026028 A1 | 25 January 2024 |
| | | TW 202304983 A | 01 February 2023 |
| | | EP 4230653 A1 | 23 August 2023 |
| | | EP 4230653 A4 | 19 June 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 109535257 A
- WO 2018142322 A
- WO 2013106717 A
- CN 105829346 A
- EP 2907824 A
- CN 117460540 A
- CN 104755494 A


### Non-patent literature cited in the description


- **SCHAEFER et al.** *Proceedings of the National Academy of Sciences of the United States of America*, 2011, vol. 108 (27), 11187-11192
- **MAZOR et al.** *mAbs*, 2015, vol. 7 (2), 377-389
- **LIU et al.** *Journal of Biological Chemistry*, 2015, vol. 290 (12), 7535-7562
- **LEWIS et al.** *Nature Biotechnology*, 2014, vol. 32 (2), 191-198
- **DILLON et al.** *mAbs*, 2017, vol. 9 (2), 213-230
- Antibodies: A Laboratory Manual. Cold Spring Harbor Press
- *J. biol. chem.*, 1968, vol. 243, 3558
- **ARAN F. LABRIJN et al.** *Nature Reviews Drug Discovery*, 2019, vol. 18, 585-608
- **CHEN S1 et al.** *J Immunol Res.*, 11 February 2019, vol. 2019, 4516041
- **HOLLIGER** ; **HUDSON**. *Nature Biotech.*, 2005, vol. 23 (9), 1126-1136
- **ADAIR** ; **LAWSON**. *Drug Design Reviews-Online*, 2005, vol. 2 (3), 209-217
- **VERMA et al.** *Journal of Immunological Methods*, 1998, vol. 216, 165-181
- **JOHNSON** ; **WU**. *Nucleic Acids Res.*, 2000, vol. 28, 214-8
- **CHOTHIA et al.** *J. Mol. Biol.*, 1986, vol. 196, 901-17
- **CHOTHIA et al.** *Nature*, 1989, vol. 342, 877-83
- **MARTIN et al.** *Proc Natl Acad Sci (USA)*, 1989, vol. 86, 9268-9272
- AbMTM, A Computer Program for Modeling Variable Regions of Antibodies. Oxford Molecular, Ltd.
- **SAMUDRALA et al.** Ab Initio Protein Structure Prediction Using a Combined Hierarchical Approach. *PROTEINS, Structure, Function and Genetics*, 1999, vol. 3, 194-198
- **MACCALLUM et al.** *J. Mol. Biol.*, 1996, vol. 5, 732-45
- **MAKABE et al.** *Journal of Biological Chemistry*, 2008, vol. 283, 1156-1166
- **CACECI et al.** *Byte*, 1984, vol. 9, 340-362
- **WONG** ; **LOHMAN**. *Proc. Natl. Acad. Sci. USA*, 1993, vol. 90, 5428-5432
- **LI N. et al.** *Methods Mol. Biol*, 2008, vol. 457, 305-17
- **PAHARA J et al.** *Exp Cell Res.*, 2010, vol. 316, 2237-50
- **MAZOR et al.** *J. Immunol. Methods*, 2007, vol. 321, 41-59
- **SAMBROOK et al.** Molecular Cloning: A Laboratory Manual. Cold Spring Harbor Laboratory Press
- **AUSUBEL et al.** Current Protocols in Molecular Biology. Greene Publishing Association
- **TANIZAKI et al.** *British Journal of Cancer*, 2011, vol. 105 (6), 807-813